(19) Europäisches Patentamt
European Patent Office
Office européen des brevets

(11) Veröffentlichungsnummer: **0 045 519**
**B1**

# (12) EUROPÄISCHE PATENTSCHRIFT

(45) Veröffentlichungstag der Patentschrift: **19.06.85**

(51) Int. Cl.[4]: **C 07 D 487/04**, A 61 K 31/55, C 07 D 207/32, C 07 D 207/34 // (C07D487/04, 223:00, 209:00)

(21) Anmeldenummer: **81106137.3**

(22) Anmeldetag: **05.08.81**

(54) **Pyrrolo(3,4-d)(2)benzazepine, Zwischenprodukte und Verfahren zu deren Herstellung sowie diese enthaltende Arzneimittel.**

(30) Priorität: **05.08.80 US 175555**
**23.07.81 US 286124**

(43) Veröffentlichungstag der Anmeldung: **10.02.82 Patentblatt 82/6**

(45) Bekanntmachung des Hinweises auf die Patenterteilung: **19.06.85 Patentblatt 85/25**

(84) Benannte Vertragsstaaten: **AT BE CH DE FR GB IT LI LU NL SE**

(56) Entgegenhaltungen:
**DE - A - 1 695 211**
**DE - A - 2 524 048**
**US - A - 4 169 150**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Fryer, Rodney Ian, 5 Eton Drive, North Caldwell, N.J. (US)**
Erfinder: **Trybulski, Eugene J., 13 Homer Street, Parsippany, N.J. (US)**
Erfinder: **Walser, Armin, 19 Crane Avenue, West Caldwell, N.J. (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 045 519 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In DE-AL-2 524 048 sind 6-Aryl-1,4-dihydro- oder -2,4-dihydro-pyrazolo[4,3-d] [2]benzazepine beschrieben, welche antidepressive Effektive vom Imipramin-Typus und insbesondere anxiolytische Wirkungen von langer Dauer zeigen. In DE-AI-1 695 211 sind 1,2,3,5-Tetrahydropyririmido[1,2-a] [1,4]benzodiazepine und 1,2-Dihydro-4 H-imidazo[1,2-a] [1,4]benzodiazepine beschrieben, welche antikonvulsive, muskelrelaxierende, psychostimulierende und/oder sedierende Eigenschaften haben. US-A-4 169 150 sind 4 H-Pyrrolo[1,2-a] [1,4]benzodiazepine beschrieben, welche anxiolytische, sedative, antikonvulsive, muskelrelaxierende und hypnotische Eigenschaften aufweisen.

Die vorliegende Erfindung betrifft Pyrrolo[3,4-d] [2]benzazepine der allgemeinen Formel

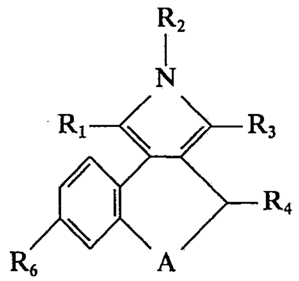

(I)

worin A eine der Gruppen

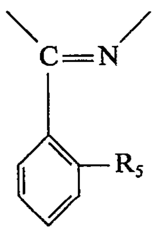

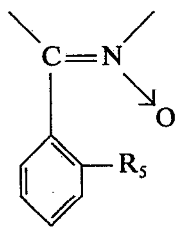

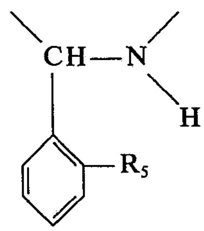

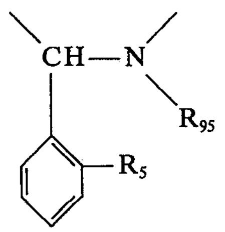

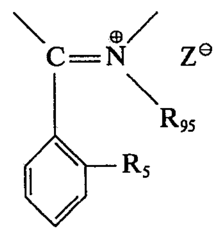

(a) (b) (c) (d) und (e)

$R_1$ Wasserstoff, niederes Alkyl, $C_3$- bis $C_7$-Alkenyl, $C_3$— bis $C_7$-Alkinyl, Hydroxymethyl oder die Gruppe —$COR_{11}$, $R_{11}$ Wasserstoff, Hydroxy, niederes Alkoxy oder Amino oder mono- oder di-niederes Alkylamino, $R_3$ Wasserstoff, niederes Alkyl, $C_3$- bis $C_7$-Alkenyl, $C_3$- bis $C_7$-Alkinyl, Hydroxymethyl oder die Gruppe —$COR_{111}$, $R_{111}$ Wasserstoff, Hydroxy, Trihalomethyl, niederes Alkoxy oder Amino oder mono- oder di-niederes Alkylamino, $R_2$ Wasserstoff, niederes Alkyl, $C_3$- bis $C_7$-Alkenyl, $C_3$- bis $C_7$-Alkinyl, die Gruppe —COO—niederes Alkyl, Hydroxy—$C_2$-bis $C_7$-Alkyl, Amino- oder mono- oder di-niederes Alkylamino—$C_2$- bis $C_7$-Alkyl oder die Gruppe —$C_1$- bis $C_6$-Alkyl—$COR_{21}$, $R_{21}$ Hydroxy, niederes Alkoxy, Amino oder mono- oder di-niederes Alkylamino, $R_4$ Wasserstoff, geradkettiges niederes Alkoxy, Hydroxy oder die Gruppe —OCOR, R Wasserstoff, $C_1$- bis $C_6$-Alkyl, niederes Haloalkyl oder Phenyl, $R_5$ Halogen mit einer Ordnungszahl, welche nicht größer als 35 ist, oder Wasserstoff, $R_6$ Halogen mit einer Ordnungszahl, welche nicht größer als 35 ist, $R_{95}$ niederes Alkyl und $Z^{\ominus}$ das Anion einer Säure bedeuten, mit der Maßgabe, daß die als »nieder« bezeichneten Reste höchstens 7 C-Atome aufweisen und den folgenden Maßgaben:

(A) wenn einer der Reste $R_1$ und $R_3$ Hydroxymethyl oder die Gruppe —$COR_{11}$ oder —$COR_{111}$ bedeutet, wobei $R_{11}$ und $R_{111}$ obige Bedeutung besitzen, dann bedeuten der verbleibende Rest Wasserstoff, niederes Alkyl, $C_3$- bis $C_7$-Alkenyl oder $C_3$- bis $C_7$-Alkinyl und $R_2$ Wasserstoff;
(B) wenn $R_4$ —OCOR bedeuten, dann bedeuten $R_1$ und $R_3$ Wasserstoff, niederes Alkyl, $C_3$- bis $C_7$-Alkenyl oder $C_3$- bis $C_7$-Alkinyl und $R_2$ die Gruppe —CO—niederes Alkoxy;
(C) wenn $R_4$ Hydroxy bedeutet, dann bedeuten $R_1$ und $R_3$ Wasserstoff, niederes Alkyl, $C_3$- bis $C_7$-Alkenyl oder $C_3$- bis $C_7$- Alkenyl und $R_2$ Wasserstoff;
(D) wenn $R_4$ geradkettiges niederes Alkoxy bedeutet, dann bedeuten $R_1$ und $R_3$ Wasserstoff, niederes Alkyl, $C_3$- bis $C_7$-Alkenyl oder $C_3$- bis $C_7$-Alkinyl;
(E) wenn A die obige Gruppe (b), (c), (d) oder (e) bedeutet, dann bedeutet $R_4$ Wasserstoff; und
(F) wenn A die obige Gruppe (b) oder (e) bedeutet, dann besitzt $R_2$ eine von Amino- oder mono- oder di-niederes Alkylamino—$C_2$- bis $C_7$-Alkyl verschiedene Bedeutung.

In einem Aspekt betrifft die vorliegende Erfindung Verbindungen der Formel

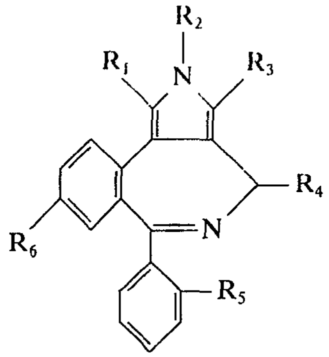

(I')

worin $R_1$ Wasserstoff, niederes Alkyl, $C_3$- bis $C_7$-Alkenyl, $C_3$- bis $C_7$-Alkinyl, Hydroxymethyl oder die Gruppe $-COR_{11}$, $R_{11}$ Wasserstoff, Hydroxy, niederes Alkoxy oder Amino oder mono- oder di-niederes Alkylamino, $R_3$ Wasserstoff, niederes Alkyl, $C_3$- bis $C_7$-Alkenyl, $C_3$- bis $C_7$-Alkinyl, Hydroxymethyl oder die Gruppe $-COR_{111}$, $R_{111}$ Wasserstoff, Hydroxy, Trihalomethyl, niederes Alkoxy oder Amino oder mono- oder di-niederes Alkylamino, $R_2$ Wasserstoff, niederes Alkyl, $C_3$- bis $C_7$-Alkenyl, $C_3$- bis $C_7$-Alkinyl, die Gruppe $-COO-$niederes Alkyl, Hydroxy$-C_2$-bis $C_7$-Alkyl, Amino- oder mono- oder di-niederes Alkylamino$-C_2$- bis $C_7$-Alkyl oder die Gruppe $-C_1$- bis $C_6$-Alkyl$-COR_{21}$; $R_{21}$ Hydroxy, niederes Alkoxy, Amino oder mono- oder di-niederes Alkylamino, $R_4$ Wasserstoff, geradkettiges niederes Alkoxy, Hydroxy oder die Gruppe $-OCOR$, R Wasserstoff, $C_1$- bis $C_6$-Alkyl, niederes Haloalkyl oder Phenyl, $R_5$ Halogen mit einer Ordnungszahl, welche nicht größer als 35 ist, oder Wasserstoff, $R_6$ Halogen mit einer Ordnungszahl, welche nicht größer als 35 ist, $R_{95}$ niederes Alkyl und $Z^{\ominus}$ das Anion einer Säure bedeuten, mit der Maßgabe, daß die als »nieder« bezeichneten Reste höchstens 7 C-Atome aufweisen und den folgenden Maßgaben:

(A) wenn einer der Reste $R_1$ und $R_3$ Hydroxymethyl oder die Gruppe $-COR_{11}$ oder $-COR_{111}$ bedeutet, wobei $R_{11}$ und $R_{111}$ obige Bedeutung besitzen, dann bedeuten der verbleibende Rest Wasserstoff, niederes Alkyl, $C_3$- bis $C_7$-Alkenyl oder $C_3$- bis $C_7$-Alkinyl und $R_2$ Wasserstoff;
(B) wenn $R_4$ $-OCOR$ bedeutet, dann bedeuten $R_1$ und $R_3$ Wasserstoff, niederes Alkyl, $C_3$- bis $C_7$-Alkenyl oder $C_3$- bis $C_7$-Alkinyl und $R_2$ die Gruppe $-CO-$niederes Alkoxy;
(C) wenn $R_4$ Hydroxy bedeutet, dann bedeuten $R_1$ und $R_3$ Wasserstoff, niederes Alkyl, $C_3$- bis $C_7$-Alkenyl oder $C_3$- bis $C_7$-Alkinyl und $R_2$ Wasserstoff;
(D) wenn $R_4$ geradkettiges niederes Alkoxy bedeutet, dann bedeuten $R_1$ und $R_3$ Wasserstoff, niederes Alkyl, $C_3$- bis $C_7$-Alkenyl oder $C_3$- bis $C_7$-Alkinyl;

und pharmazeutisch annehmbare Säureadditionssalze davon.

Diese Verbindungen besitzen sedative und antiolytische Eigenschaften.

Der Ausdruck »Halogen« oder »Halo« bedeutet, sofern nichts anderes angegeben ist, Brom, Chlor oder Fluor.

Der Ausdruck »niederes Alkyl« oder »Alkyl« bezeichnet, sofern nichts anderes angegeben ist, geradkettige oder verzweigte $C_1$- bis $C_7$-Kohlenwasserstoffreste, vorzugsweise $C_1$- bis $C_4$-Kohlenwasserstoffreste, wie Methyl, Äthyl, Propyl, Isopropyl und dergleichen.

Der Ausdruck »$C_3$- bis $C_7$-Alkenyl oder -Alkinyl« bezeichnet geradkettige oder verzweigte $C_3$- bis $C_7$-Kohlenwasserstoffreste, worin mindestens eine Kohlenstoff—Kohlenstoff-Bindung ungesättigt ist und eine Doppel- oder Dreifachbindung ist. Die ungesättigte Bindung kann allerdings nicht zwischen dem Kohlenstoffatom, das direkt am Pyrrolring gebunden ist, und dem benachbarten Kohlenstoffatom sein, d. h. das endständig gebundene Kohlenstoffatom ist nicht ungesättigt.

Verbindungen der Formel I', worin $R_1$ und $R_3$ Wasserstoff, niederes Alkyl, $C_3$- bis $C_7$.Alkenyl oder $C_3$- bis $C_7$-Alkinyl bedeuten, $R_2$ obige Bedeutung besitzt, jedoch nicht Amino- oder mono- oder di-niederes Alkylamino$-C_2$- bis $C_7$-Alkyl bedeutet, und $R_4$ Wasserstoff bedeutet, können mit einem niederen Alkylhalogenid oder -sulfonat an der Iminogruppe quaternisiert werden. Die so erhaltenen quaternären Iminiumsalze und Verbindungen der Formel I', worin $R_1$ und $R_3$ Wasserstoff, niederes Alkyl, $C_3$- bis $C_7$-Alkenyl oder $C_3$- bis $C_7$-Alkinyl und $R_4$ Wasserstoff bedeuten, können erwünschtenfalls mit geeigneten Reduktionsmitteln, wie Lithiumaluminiumhydrid, Natriumborhydrid oder Natriumcyanoborhydrid, zu den entsprechenden 5-Alkyl-5,6-dihydro- oder 5,6-Dihydro-Verbindungen reduziert werden.

Die 5,6-Dihydro-Derivate und die quaternären Iminiumsalze sind ebenfalls anxiolytische Wirkstoffe.

Verbindungen der Formel I', worin $R_1$ und $R_3$ Wasserstoff, niederes Alkyl, $C_3$- bis $C_7$-Alkenyl und $C_3$- bis $C_7$-Alkinyl bedeuten, $R_2$ obige Bedeutung besitzt, jedoch nicht Amino- oder mono- oder di-niederes Alkylamino$-C_2$- bis $C_7$-Alkyl bedeutet, $R_4$ Wasserstoff bedeutet und $R_5$ und $R_6$ obige Bedeutung besitzen, können zu den entsprechenden Iminoxiden und N-Oxiden umgesetzt werden, welche ebenfalls anxiolytische Wirkstoffe sind.

Die erfindungsgemäßen Verbindungen können hergestellt werden, indem man

a) eine Verbindung der allgemeinen Formel

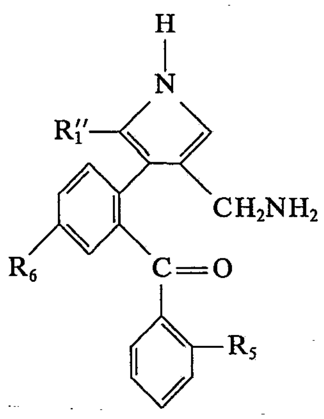

(B)

worin $R_5$ und $R_6$ obige Bedeutung besitzen, und $R_1''$ Wasserstoff, niederes Alkyl, $C_3$- bis $C_7$-Alkenyl oder $C_3$- bis $C_7$-Alkinyl bedeutet, cyclisiert, oder

b) eine Verbindung der Formel I, worin A die obige Gruppe (a), $R_2$ und $R_4$ je Wasserstoff, und einer der Reste $R_1$ und $R_3$ Wasserstoff und der andere Wasserstoff, niederes Alkyl, $C_3$- bis $C_7$-Alkenyl oder $C_3$- bis $C_7$-Alkinyl bedeuten, in der 1- und/oder 3-Stellung niederalkyliert, $C_3$- bis $C_7$-alkenyliert oder $C_3$- bis $C_7$-alkinyliert, oder

c) eine Verbindung der Formel I, worin A die obige Gruppe (a), $R_1$ und $R_3$ je Wasserstoff, niederes Alkyl, $C_3$- bis $C_7$-Alkenyl oder $C_3$- bis $C_7$-Alkinyl und $R_2$ Wasserstoff bedeuten, in der 2-Stellung mit einer niederen Alkyl-, $C_3$- bis $C_7$-Alkenyl-, $C_3$- bis $C_7$-Alkinyl- oder mit einer Hydroxy—$C_2$- bis $C_7$-Alkylgruppe, oder mit der Gruppe —COO—niederes Alkyl, $-(CH_2)_n-$COO—niederes Alkyl, $-(CH_2)_n-CO-NR_{91}R_{92}$ oder $-(CH_2)_{n+1}-NR_{91}R_{92}$, worin n die Zahl 1 bis 6 und $R_{91}$ und $R_{92}$ je Wasserstoff oder niederes Alkyl bedeuten, substituiert, oder

d) eine Verbindung der Formel I, worin A die obige Gruppe (a), $R_1$ und $R_3$ je Wasserstoff, niederes Alkyl, $C_3$- bis $C_7$-Alkenyl oder $C_3$- bis $C_7$-Alkinyl und $R_2$ die Gruppe $-(CH_2)_nCOO-$niederes Alkyl oder $-(CH_2)_n-CO-NR_{91}R_{92}$ bedeuten und n, $R_{91}$ und $R_{92}$ obige Bedeutung besitzen, reduziert, oder

e) eine Verbindung der Formel I, worin A die obige Gruppe (a), $R_1$ und $R_3$ je Wasserstoff, niederes Alkyl, $C_3$- bis $C_7$-Alkenyl oder $C_3$- bis $C_7$-Alkinyl und $R_2$ die Gruppe $-(CH)_n-$COO—niederes Alkyl bedeuten, und n obige Bedeutung besitzt, in die entsprechende Carbonsäure oder in ein entsprechendes Amid, niederes Alkylamid oder di-niederes Alkylamid umwandelt, oder

f) eine Verbindung der Formel I, worin A die obige Gruppe (a), $R_2$ und $R_4$ je Wasserstoff und einer der Reste $R_1$ und $R_3$ Wasserstoff und der andere Wasserstoff, niederes Alkyl, $C_3$- bis $C_7$-Alkenyl oder $C_3$ bis $C_7$-Alkinyl bedeuten, in der 1- oder 3-Stellung formyliert oder hydroxymethyliert, oder

g) eine Verbindung der Formel I, worin A die obige Gruppe (a), $R_2$ und $R_4$ je Wasserstoff und einer der Reste $R_1$ und $R_3$ Formyl und der andere Wasserstoff, niederes Alkyl, $C_3$- bis $C_7$-Alkenyl oder $C_3$- bis $C_7$-Alkinyl bedeuten, zur entsprechenden Hydroxymethylverbindung reduziert, oder

h) eine Verbindung der Formel I, worin A die obige Gruppe (a), $R_2$, $R_3$ und $R_4$ je Wasserstoff und $R_1$ Wasserstoff, niederes Alkyl, $C_3$- bis $C_7$-Alkenyl oder $C_3$- bis $C_7$-Alkinyl bedeuten, mit einem Trihaloacetylchlorid behandelt, oder

i) eine Verbindung der Formel I, worin A die obige Gruppe (a), $R_2$ und $R_4$ je Wasserstoff, $R_1$ Wasserstoff, niederes Alkyl, $C_3$- bis $C_7$-Alkenyl oder $C_3$- bis $C_7$-Alkinyl und $R_3$ Trihaloacetyl bedeuten, mit einem Alkalimetall-niederen Alkoxid oder mit einer Verbindung der Formel $HNR_{35}R_{36}$, worin $R_{36}$ je Wasserstoff oder niederes Alkyl bedeuten, behandelt, oder

j) eine Verbindung der Formel I, worin A die obige Gruppe (a), $R_2$ und $R_4$ je Wasserstoff und einer der Reste $R_1$ und $R_3$ Formyl und der andere Wasserstoff, niederes Alkyl, $C_3$- bis $C_7$-Alkenyl oder $C_3$- bis $C_7$-Alkinyl bedeuten, zur entsprechenden Carbonsäure oxydiert, oder

k) eine Verbindung der Formel I, worin A die obige Gruppe (a), $R_2$ und $R_4$ je Wasserstoff und einer der Reste $R_1$ und $R_3$ die Gruppe —COOH und der andere Wasserstoff, niederes Alkyl, $C_3$- bis $C_7$-Alkenyl oder $C_3$- bis $C_7$-Alkinyl bedeuten, verestert, oder

l) eine Verbindung der Formel I, worin A die obige Gruppe (a), $R_2$ und $R_4$ je Wasserstoff und einer der Reste $R_1$ und $R_3$ niederes Alkoxycarbonyl und der andere Wasserstoff, niederes Alkyl, $C_3$- bis $C_7$-Alkenyl oder $C_3$- bis $C_7$-Alkinyl bedeuten, mit einer Verbindung der Formel $HNR_{35}R_{36}$, worin $R_{35}$ und $R_{36}$ obige Bedeutung besitzen, behandelt, oder

m) eine Verbindung der Formel I, worin A die obige Gruppe (a), $R_4$ Wasserstoff, $R_1$ und $R_3$ je Wasserstoff, niederes Alkyl, $C_3$ bis $C_7$-Alkenyl oder $C_3$- bis $C_7$-Alkinyl und $R_2$ Wasserstoff,

niederes Alkyl, $C_3$- bis $C_7$-Alkenyl, $C_3$- bis $C_7$-Alkinyl, die Gruppe —COO—niederes Alkyl, Hydroxy—$C_2$- bis $C_7$-Alkyl oder die Gruppe $-(CH_2)_n-$COO—niederes Alkyl oder $-(CH_2)_n-$CO—$NR_{91}R_{92}$ bedeuten und n, $R_{91}$ und $R_{92}$ obige Bedeutung besitzen, $N_5$-oxydiert, oder

n) eine Verbindung der Formel I, worin A die obige Gruppe (b), $R_4$ Wasserstoff, $R_2$ die Gruppe —COO—niederes Alkyl und $R_1$ und $R_3$ je Wasserstoff, niederes Alkyl, $C_3$ bis $C_7$ Alkenyl oder $C_3$ bis $C_7$-Alkinyl bedeuten, mit einem den Rest —COR liefernden Acylierungsmittel behandelt, wobei R obige Bedeutung besitzt, oder

o) eine Verbindung der Formel I, worin A die obige Gruppe (a), $R_4$ die Gruppe —OCOR, $R_2$ die Gruppe —COO—niederes Alkyl und $R_1$ und $R_3$ je Wasserstoff, niederes Alkyl, $C_3$- bis $C_7$-Alkenyl oder $C_3$- bis $C_7$-Alkinyl bedeuten und R obige Bedeutung besitzt, oder eine Verbindung der Formel I, worin in A die obige Gruppe (b), $R_2$ die Gruppe —COO—niederes Alkyl, $R_4$ Wasserstoff und $R_1$ und $R_3$ je Wasserstoff, niederes Alkyl, $C_3$- bis $C_7$-Alkenyl oder $C_3$- bis$C_7$-Alkinyl bedeuten, einer alkalischen Hydrolyse unterwirft, oder

p) eine Verbindung der Formel I, worin A die obige Gruppe (a), $R_4$ die Gruppe —OCOR, $R_2$ die Gruppe —COO—niederes Alkyl und $R_1$ und $R_3$- bis $C_7$-Alkinyl bedeuten und R obige Bedeutung besitzt, in Gegenwart eines $C_1$- bis $C_7$-geradkettigen Alkohols mit einem Alkalimetallhydroxid oder -alkoxid behandelt, oder

q) eine Verbindung der Formel I, worin A die obige Gruppe (a), $R_4$ Wasserstoff und $R_1$ und $R_3$ je Wasserstoff, niederes Alkyl, $C_3$- bis $C_7$-Alkenyl oder $C_3$- bis $C_7$-Alkinyl bedeuten, reduziert, oder

r) in einer Verbindung der Formel I worin A die obige Gruppe (a), $R_4$ Wasserstoff, $R_1$ und $R_3$ je Wasserstoff, niederes Alkyl, $C_3$- bis $C_7$-Alkenyl oder $C_3$- bis $C_7$-Alkinyl und $R_2$ Wasserstoff, niederes Alkyl, $C_3$- bis $C_7$-Alkenyl, $C_3$- bis $C_7$-Alkinyl, die Gruppe —COO—niederes Alkyl, Hydroxy—$C_2$- bis $C_7$-Alkyl oder die Gruppe $-(CH_2)_n-$COO—niederes Alkyl oder $-(CH_2)_n-$CO—$NR_{91}R_{92}$ bedeuten, und n, $R_{91}$ und $R_{92}$ obige Bedeutung besitzen, die Imingruppe quaternisiert, oder

s) eine Verbindung der Formel I, worin A die obige Gruppe (e), $R_4$ Wasserstoff, $R_1$ und $R_3$ je Wasserstoff, niederes Alkyl, $C_3$- bis $C_7$-Alkenyl oder $C_3$- bis $C_7$-Alkinyl und $R_2$ Wasserstoff,niederes Alkyl, $C_3$- bis $C_7$-Alkenyl, $C_3$-bis $C_7$-Alkinyl, die Gruppe —COO—niederes Alkyl, Hydroxy— $C_2$- bis $C_7$-Alkyl oder die Gruppe $-(CH_2)_n-$COO—niederes Alkyl oder $-(CH_2)_n-$CO—$NR_{91}R_{92}$ bedeuten, und n, $R_{91}$ und $R_{92}$ obige Bedeutung besitzen, reduziert, oder

t) eine Verbindung der Formel I, worin A die obige Gruppe (a) bedeutet, in ein pharmazeutisch annehmbares Säureadditionssalz überführt.

Die verschiedenen erfindungsgemäßen Verfahrensvarianten und die Herstellung der darin verwendeten Ausgangsstoffe werden, im Sinne eines Beispiels, durch die folgenden Schemata illustriert:

Schema I

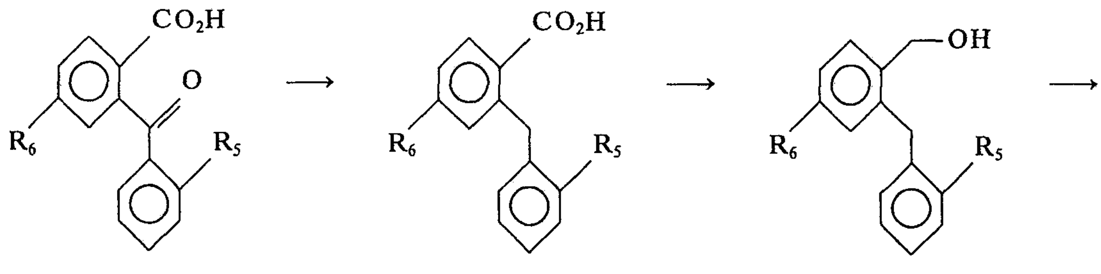

(II) (III) (IV)

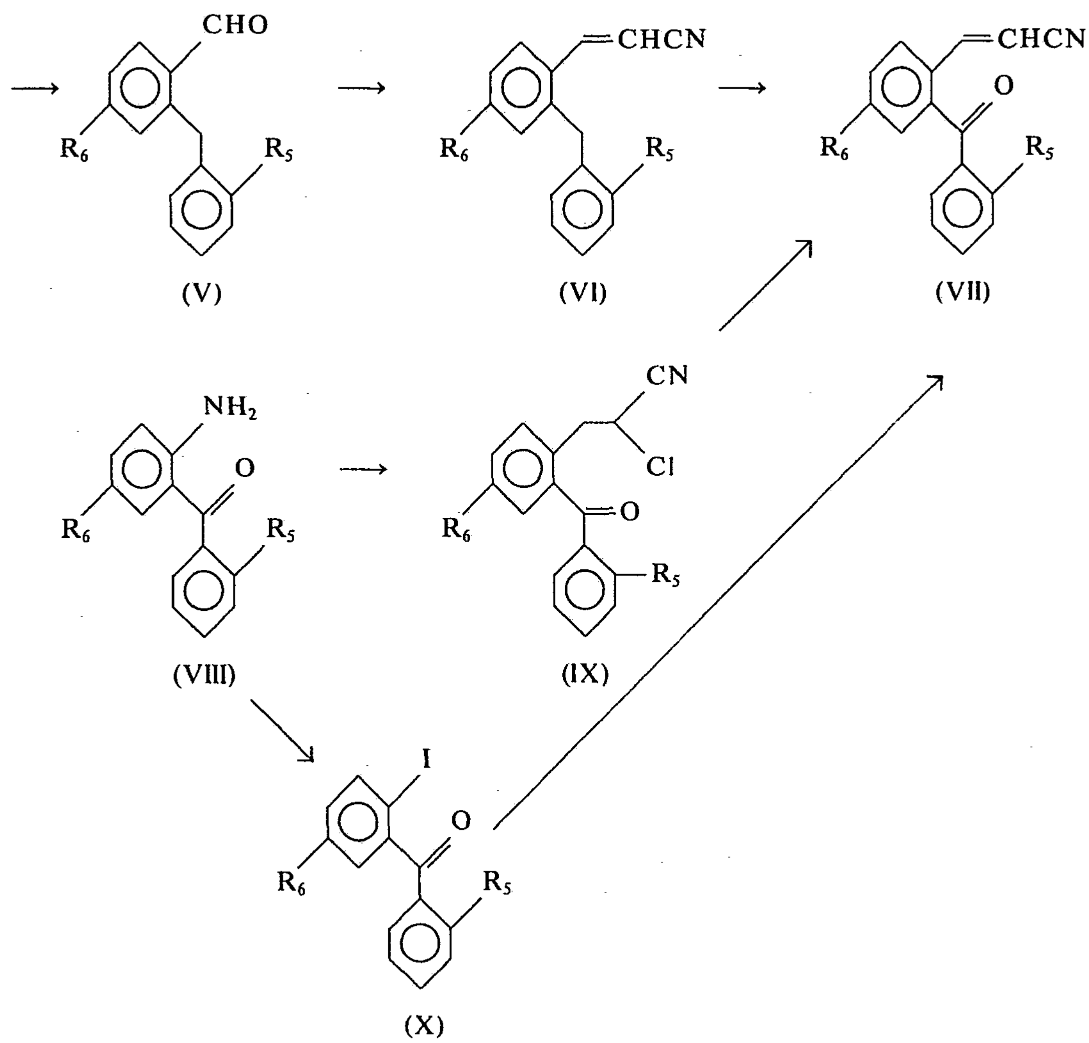

(V) (VI) (VII) (VIII) (IX) (X)

worin R5 und R6 obige Bedeutung besitzen.

II → III

Eine Verbindung der Formel II, ein bekanntes Ausgangsmaterial, kann mit Zinkstaub und Kupfersulfat in konzentriertem Ammoniak umgesetzt werden. Die Reaktionstemperatur variiert in einem Bereich von etwa Raumtemperatur bis 100° C und beträgt vorzugsweise 100° C.

III →

Eine Verbindung der Formel III kann in einem ätherischen Lösungsmittel, wie Diäthyläther oder Tetrahydrofuran, mit einem Metallhydrid, wie Lithiumaluminiumhydrid, oder einem Boran umgesetzt werden. Die Reaktionstemperatur variiert in einem Bereich von etwa —78° C bis Raumtemperatur und beträgt vorzugsweise 0° C.

IV → V

Eine Verbindung der Formel IV kann mit Pyridiniumchlorochromat, Mangandioxid oder einem anderen geeigneten Oxydationsmittel umgesetzt werden, wobei man Methylenchlorid als Lösungsmittel verwendet. Die Reaktionstemperatur variiert in einem Bereich von etwa 0° C bis Siedetemperatur des Lösungsmittels und liegt vorzugsweise bei etwa Raumtemperatur.

V → VI

Eine Verbindung der Formel V kann in einem ätherischen Lösungsmittel, wie Tetrahydrofuran, in Gegenwart einer starken Base, wie Natriumhydrid, Natriumamid, etc., mit Diäthylcyanomethylphosphonat umgesetzt werden. Die Reaktionstemperatur variiert in einem Bereich von etwa 0° C bis Raumtemperatur und liegt vorzugsweise bei etwa Raumtemperatur.

VI → VII

Eine Verbindung der Formel VI kann in einer Mischung aus Essigsäure und Methylenchlorid mit Chromtrioxid oder einem von Chromtrioxid abgeleiteten Oxydationsmittel umgesetzt werden. Die Reaktionstemperatur variiert in einem Bereich von etwa 0° C bis etwa 90° C und liegt vorzugsweise bei Raumtemperatur.

VIII → IX

Eine Verbindung der Formel VIII, ein bekannten Ausgangsmaterial, kann in Gegenwart von Acetonitril, einem niederen Alkylnitril und Kupferchlorid mitAcrylnitril umgesetzt werden. Die Reaktionstemperatur variiert in einem Bereich von etwa 0° C bis etwa 40° C und liegt vorzugsweise bei etwa Raumtemperatur.

IX → VII

Eine Verbindung der obigen Formel IX kann mit einer Mischung aus einem Alkalimetall-, z. B. Lithium-, Natrium- oder Kalium-, -carbonat und einem -bicarbonat, vorzugsweise mit einer Mischung aus einem Teil Kaliciumcarbonat und drei Teilen Kaliumbicarbonat, umgesetzt werden. Unter den geeigneten Lösungsmitteln sind Dimethylsulfoxid, Dimethylformamid oder $C_1$- bis $C_4$-Alkohole, z. B. Methanol. Die Reaktionstemperatur variiert in einem Bereich von etwa 20° C bis 50° C und liegt vorzugsweise bei Raumtemperatur. Bei der obigen Reaktion handelt es sich um eine dem Fachmann an sich bekannte Dehydrohalogenierung.

VIII → X

Man kann eine Verbindung der Formel VIII unter Verwendung von Natriumnitrit in Schwefelsäure diazotieren und das erhaltene Diazoniumsalz durch Ausfällen des entsprechenden Tetrafluoroborates isolieren. Durch anschließendes Behandeln einer Aufschlämmung dieses Salzes in Wasser mit wäßrigem Kaliumjodid bei Raumtemperatur erhält man ein Jobdenzophenon der Formel X.

X → VII

Eine Verbindung der Formel X kann unter Verwendung von Acetonitril oder einem aromatischen Kohlenwasserstoff, wie Toluol, als Lösungsmittel und in Gegenwart eines Palladium(II)salzes, wie dem Acetat, Chlorid, etc., mit Acrylnitril umgesetzt werden. Die Reaktionstemperatur variiert von etwa 60° C bis Siedetemperatur des Lösungsmittels und liegt vorzugsweise bei der Siedetemperatur.

Schema II

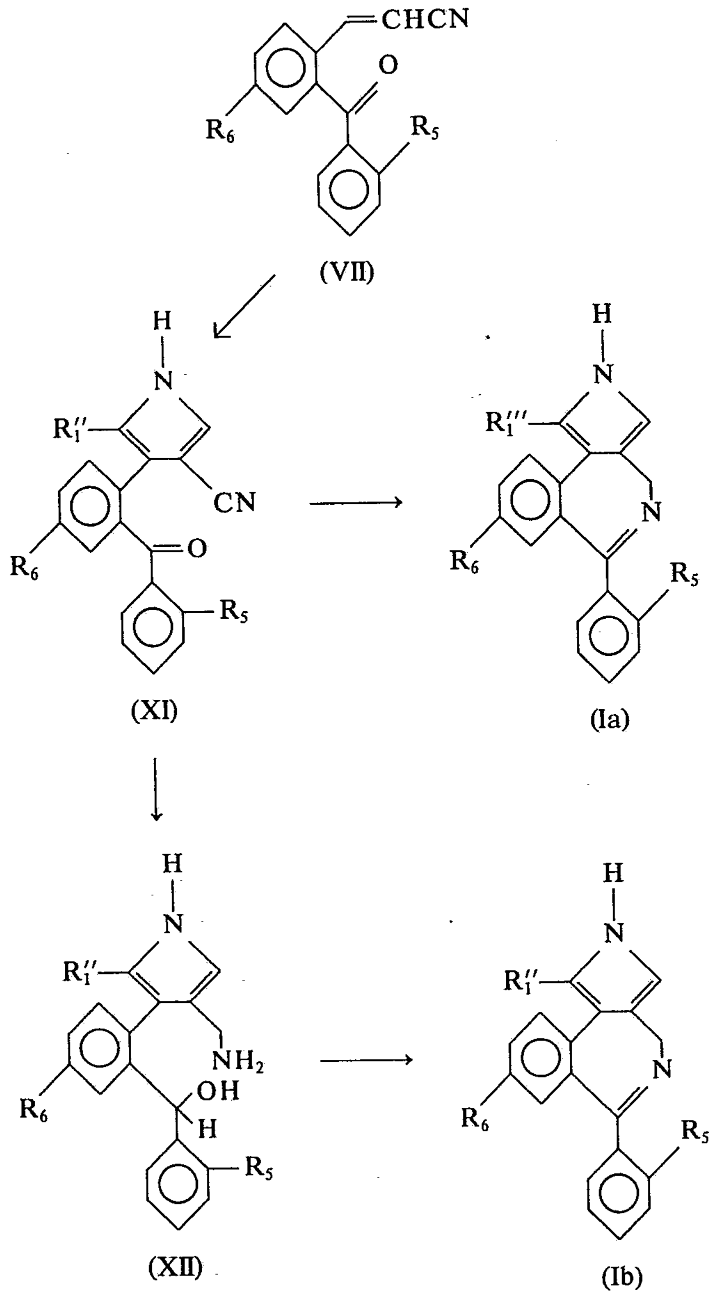

(VII) (XI) (Ia) (XII) (Ib)

worin $R_1''$ Wasserstoff, niederes Alkyl, $C_3$- bis $C_7$-Alkenyl oder $C_3$- bis $C_7$-Alkinyl und $R_1'''$ Wasserstoff oder niederes Alkyl bedeuten und $R_5$ und $R_6$ obige Bedeutung besitzen.

VII → XI

Eine Verbindung der Formel VII kann in Gegenwart von Natriumhydrid und unter Verwendung einer Mischung aus Dimethylsulfoxid und einem Äther, wie Diäthyläther, Dioxan oder Tetrahydrofuran, als Lösungsmittel mit einem $\alpha$-Tosyl-$C_2$- bis $C_8$-Alkylisocyanid, einem $\alpha$-Tosyl-$C_4$- bis $C_8$-Alkenylisocyanid oder einem $\alpha$-Tosyl-$C_4$- bis $C_8$-Alkinylisocyanid umgesetzt werden. Die Reaktionstemperatur variiert in einem Bereich von etwa 0° C bis etwa 40° C und liegt vorzugsweise bei Raumtemperatur. Die $\alpha$-Tosylalkylisocyanide können nach van Leusen et al., Tetrahedron Letters, 3487 (1975) hergestellt werden. Die Verbindungen der Formel XI sind ebenfalls Gegenstand der vorliegenden Erfindung.

XI → Ia

Eine Verbindung der Formel XI kann bei Drucken von etwa Atmosphärendruck bis 5 Atmosphären, in Gegenwart eines Übergangsmetallkatalysators, wie Raney-Nickel, und unter Verwendung von Eisessig als Lösungsmittel mit Wasserstoff umgesetzt werden. Das so erhaltene, der Formel B entsprechende Amin cyclisiert spontan zum Azepinring. Die Reaktionstemperatur liegt bei etwa Raumtemperatur.

Das zuerst gebildete offenkettige Amin wird nicht isoliert, sondern man läßt es in situ zum Produkt der Formel Ia cyclisieren.

XI → XII

Eine Verbindung der Formel XI kann in einem ätherischen Lösungsmittel, wie Tetrahydrofuran, mit einem Metallhydrid-Reduktionsmittel, wie Lithiumaluminiumhydrid umgesetzt werden. Die Reaktionstemperatur variiert in einem Bereich von etwa −20° C bis Raumtemperatur und beträgt vorzugsweise 0° C. Die Verbindungen der Formel XII sind ebenfalls Gegenstand der vorliegenden Erfindung.

XII → Ib

Eine Verbindung der Formel XII kann in einem ätherischen Lösungsmittel, wie Tetrahydrofuran, oder einem Lösungsmittel, wie Toluol, mit Mangandioxid umgesetzt werden. Das so erhaltene Amin der Formel B cyclisiert spontan zum Azepinring. Die Reaktionstemperatur variiert in einem Bereich von etwa Raumtemperatur bis Siedetemperatur des Lösungsmittels und beträgt vorzugsweise 40° C. Die Verbindungen der Formel B sind ebenfalls Gegenstand der vorliegenden Erfindung.

Schema III

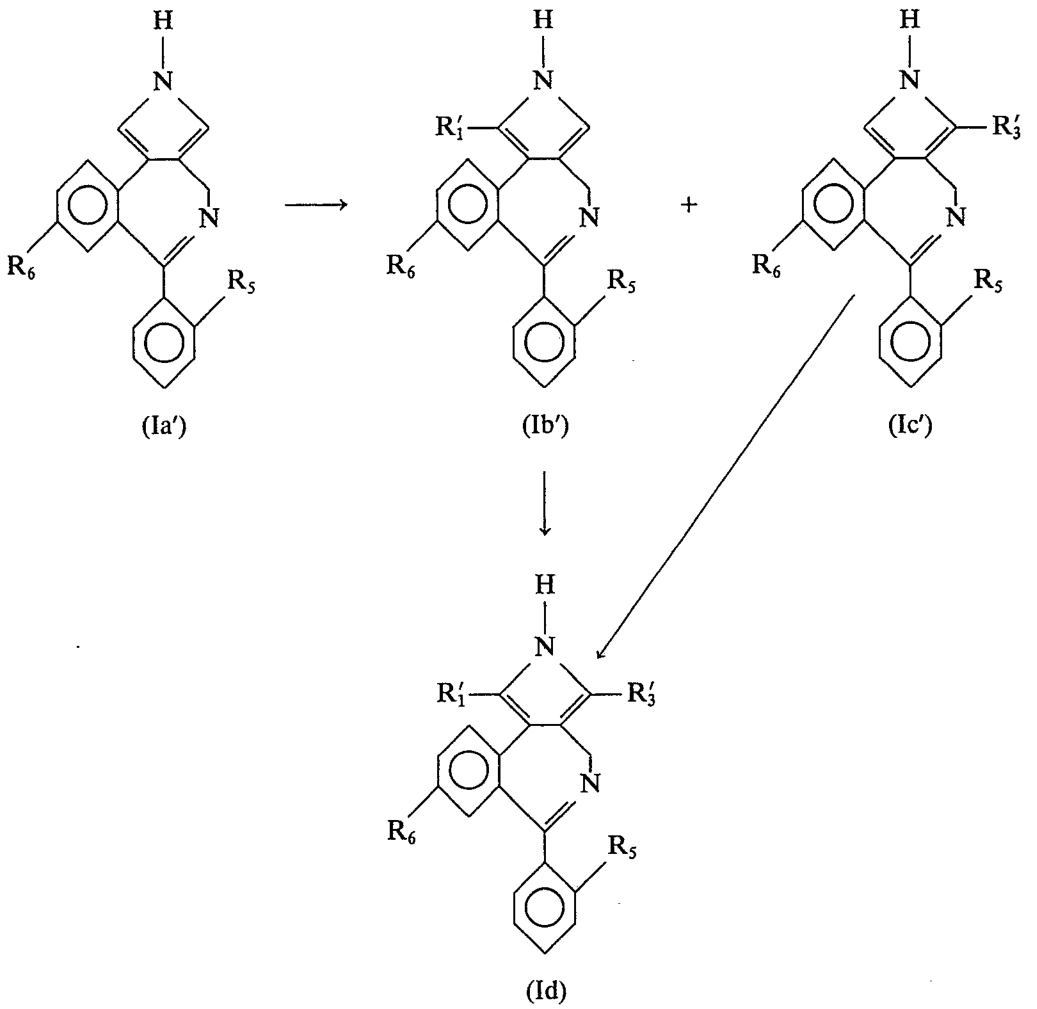

(Ia') (Ib') + (Ic')

(Id)

worin $R_1'$ und $R_3'$ niederes Alkyl, $C_3$- bis $C_7$-Alkenyl oder $C_3$- bis $C_7$-Alkinyl bedeuten und $R_5$ und $R_6$ obige Bedeutung besitzen.

Ia' → Ib' + Ic'

Eine Verbindung der Formel Ia' kann zwischen —78° C und etwa 0° C, vorzugsweise bei etwa —20° C, mit einem Äquivalent einer starken Base, wie Lithiumdiisopropylamid umgesetzt werden. Das erhaltene Anion kann mit dem gewünschten Alkylierungs-, Alkenylierungs- oder Alkinylierungsmittel, wie einem niederen Alkyl-, $C_3$- bis $C_7$-Alkenyl- oder $C_3$- bis $C_7$-Alkinylhalogenid oder -sulfonat, umgesetzt werden. Man erhält eine Mischung von Isomeren der Formeln Ib' und Ic', das mittels Säulenchromatographie aufgetrennt werden kann.

Ib' → Id und Ic' → Id

Die Reaktionsbedingungen sind dieselben wie für die Reaktion einer Verbindung der Formel Ia' zu einer Verbindung der Formel Ib' und Ic'. Es können die gleichen oder verschiedene Alkylierungs-, Alkenylierungs- oder Alkinylierungsmittel verwendet werden, so daß man symmetrisch oder nichtsymmetrisch substituierte Verbindungen erhält.

Schema IV

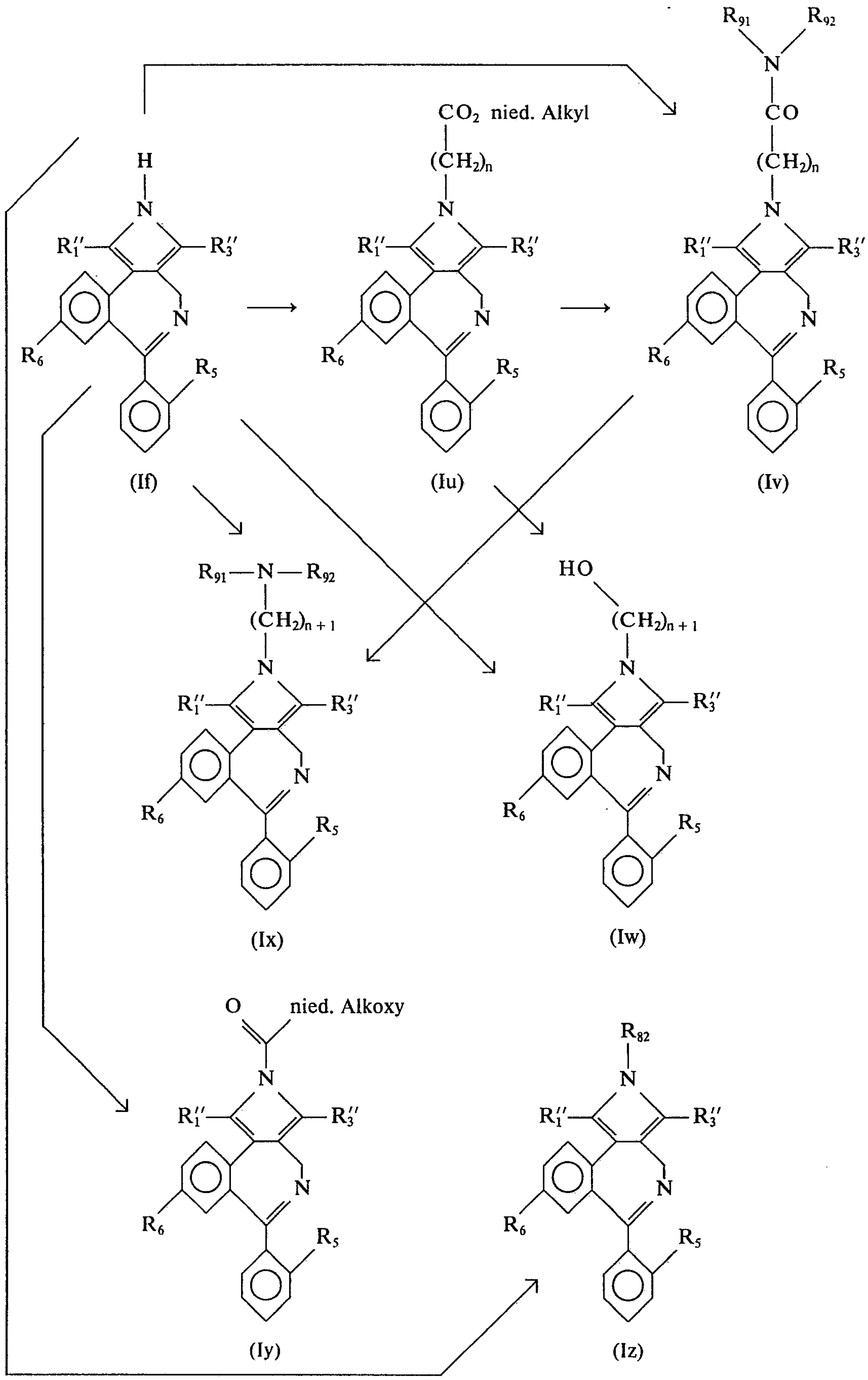

(If) (Iu) (Iv) (Ix) (Iw) (Iy) (Iz)

worin $R_5$ und $R_6$ obige Bedeutung besitzen, $R_2''$ und $R_3''$ je Wasserstoff, niederes Alkyl, $C_3$- bis $C_7$-Alkenyl oder $C_3$- bis $C_7$-Alkinyl, n die Zahl 1 bis 6, $R_{91}$ und $R_{92}$ je Wasserstoff oder niederes Alkyl und $R_{82}$ niederes Alkyl, $C_3$- bis $C_7$-Alkenyl oder $C_3$- bis $C_7$-Alkinyl bedeuten.

If → Iu

Eine Verbindung der Formel If kann in einem polaren Lösungsmittel, wie Dimethylsulfoxid oder Dimethylformamid, und in Gegenwart eines Alkalimetallalkoxides mit einem Haloester, wie Äthylbromacetat und Äthyl-3-brompropionat umgesetzt werden. Die Reaktionstemperatur variiert in einem Bereich von etwa —20° C bis Raumtemperatur und beträgt vorzugsweise 0° C. Erwünschtenfalls kann man das erhaltene Endprodukt in einem wäßrigen ätherischen Lösungsmittel, wie wäßrigem Tetrahydrofuran, mit einem Alkalimetallcarbonat oder -hydroxid behandeln. Durch anschließende Zugabe einer starken Mineralsäure erhält man die entsprechende Carbonsäure.

Iu → Iv

Eine Verbindung der Formel Iu kann in einem $C_1$- bis $C_4$-Alkohol mit Ammoniak oder einem mono- oder di-substituierten niederen Alkylamin und einer katalytischen Menge des entsprechenden Hydrochlorides umgesetzt werden. Die Reaktion wird gewöhnlich bei 100° C unter Verwendung einer Druckapparatur durchgeführt, um die flüchtigen Reaktanden zurückzuhalten.

If → Ix

Eine Verbindung der Formel If kann mit einer Verbindung der Formel

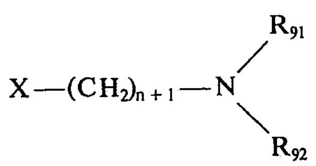

worin $R_{91}$ und $R_{92}$ Wasserstoff oder niederes Alkyl, und X Halogen bedeuten und n obige Bedeutung besitzt, umgesetzt werden, und zwar in Gegenwart eines Alkalimetallalkoxides und in einem polaren Lösungsmittel, wie Dimethylsulfoxid oder Dimethylformamid. Die Reaktionstemperatur variiert in einem Bereich von etwa —20° C bis Raumtemperatur und liegt vorzugsweise bei Raumtemperatur.

If → Iy

Eine Verbindung der Formel If kann zuerst mit einem Alkalimetallalkoxid und anschließend mit einem Haloformat, wie Methylchloroformat, etc., in einem polaren Lösungsmittel, wie Dimethylsulfoxid oder Dimethylformamid umgesetzt werden. Die Reaktionstemperatur variiert in einem Bereich von etwa —20° C bis Raumtemperatur und beträgt vorzugsweise 0° C.

If → Iz

Eine Verbindung der Formel If kann in einem polaren aprotischen Lösungsmittel, z. B. in Dimethylformamid oder in Dimethylsulfoxid, zuerst mit einem Alkalimetallalkoxid, wie Kalium- oder Natriummethoxid, und anschließend mit einem Alkylierungs-, Alkenylierungs- oder Alkinylierungsmittel umgesetzt werden, d. h. mit einem niederen Alkyl-, $C_3$- bis $C_7$-Alkenyl- oder mit einem $C_3$- bis $C_7$-Alkinylhalogenid oder -sulfonat. Die Reaktionstemperatur variiert in einem Bereich von 0° C bis Raumtemperatur und beträgt vorzugsweise 0° C.

Iv → Ix

Eine Verbindung der Formel Iv kann in einem ätherischen Lösungsmittel, wie Tetrahydrofuran, mit einem Metallhydrid-Reduktionsmittel, wie Lithiumaluminiumhydrid umgesetzt werden. Die Reaktionstemperatur variiert in einem Bereich von etwa —20° C bis Raumtemperatur und beträgt vorzugsweise 0° C.

If → Iw

Eine Verbindung der Formel If kann in Gegenwart eines Alkalimetallalkoxides und von Dimethylformamid oder Dimethylsulfoxid mit einer Verbindung der Formel

$$X-(CH_2)_{n+1}-OR_{80}$$

worin $R_{80}$ eine Hydroxy-Schutzgruppe und X Halogen bedeuten, und n obige Bedeutung besitzt, umgesetzt werden.

Unter den geeigneten Hydroxy-Schutzgruppen sind Tetrahydropyranyläther. Durch anschließendes Behandeln mit wäßriger Säure erhält man das Endprodukt.

If → Iv

Eine Verbindung der Formel If kann in Gegenwart eines Alkalimetallalkoxides und von Dimethylformamid oder Dimethylsulfoxid mit einer Verbindung der Formel

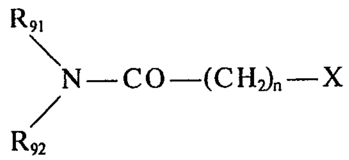

worin $R_{91}$, $R_{92}$ und n obige Bedeutung besitzen und X Halogen bedeutet, umgesetzt werden.

Schema V

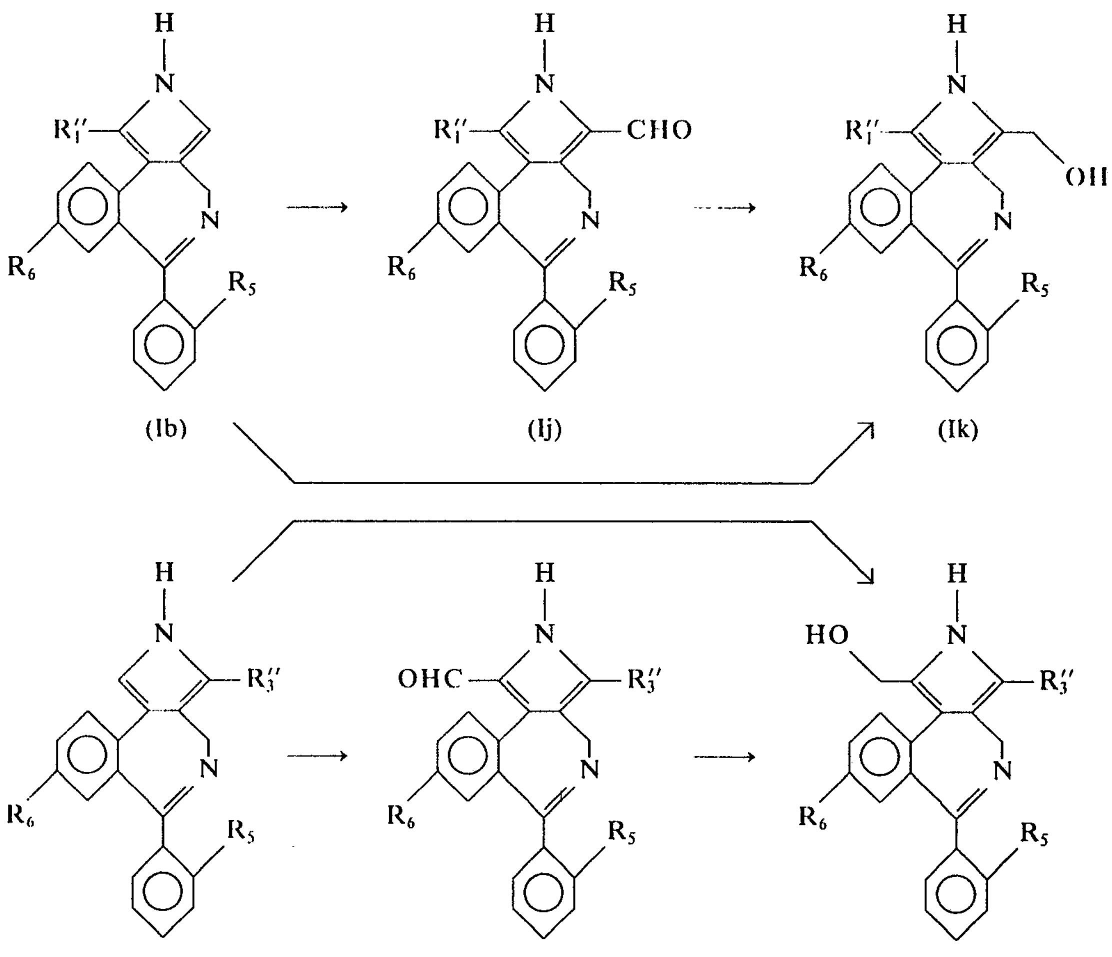

(Ib) (Ij) (Ik)

(Ic) (Il) (Im)

worin $R_1''$, $R_3''$, $R_5$ und $R_6$ obige Bedeutung besitzen.

Ib → Ij und Ic → Il

Die Verbindungen der Formeln Ib und Ic können in Abwesenheit oder in Anwesenheit eines Lösungsmittels, wie Methylenchlorid, mit einer Mischung aus Dimethylformamid und Phosphorylchlorid (Phosgen, Thionylchlorid oder Oxalylchlorid können ebenfalls verwendet werden) umgesetzt werden. Die Reaktion wird gewöhnlich bei etwa 0° C durchgeführt.

Ij → Ik und Il → Im

Die Verbindungen der Formeln Ij und Il können in einem $C_1$- bis $C_4$-Alkohol, vorzugsweise in Äthanol, mit Natriumborhydrid umgesetzt werden, wobei man zwischen —20° C und Raumtemperatur, vorzugsweise bei 0° C, arbeitet.

Ib → Ik und Ic → Im

Die Verbindungen der Formeln Ib und Ic können in einem $C_1$- bis $C_4$-Alkohol, vorzugsweise in Methanol, und in Gegenwart eines Alkalimetallcarbonates mit Paraformaldehyd umgesetzt werden. Die Reaktionstemperatur variiert in einem Bereich von etwa 0° C bis Siedetemperatur des Lösungsmittels und liegt vorzugsweise bei Raumtemperatur.

Schema VI

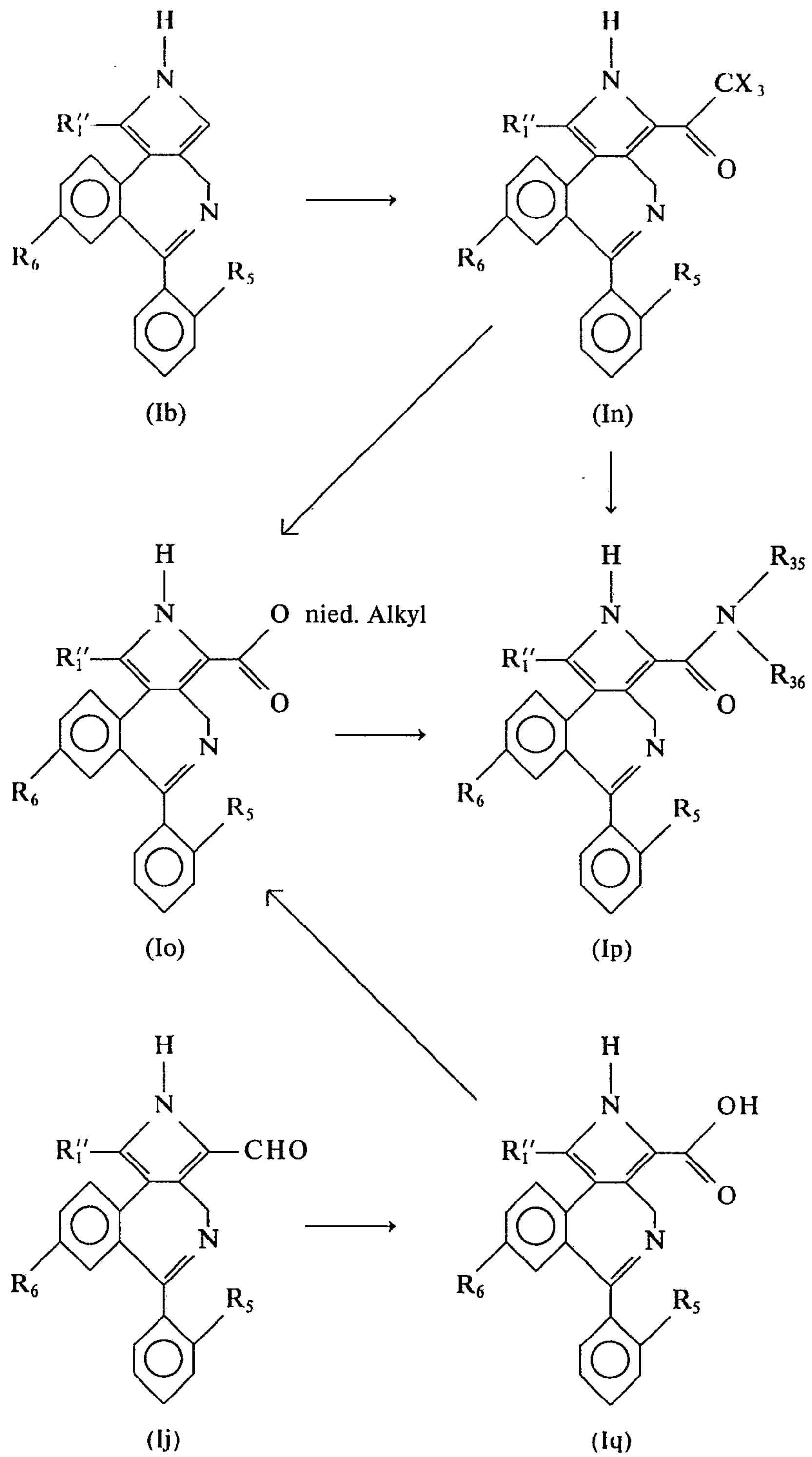

(Ib) (In) (Io) (Ip) (Ij) (Iq)

worin $R_{35}$ und $R_{36}$ je Wasserstoff oder niederes Alkyl und X Halo bedeuten, und $R_1''$ und $R_3''$ obige Bedeutung besitzen.

Schema VI a

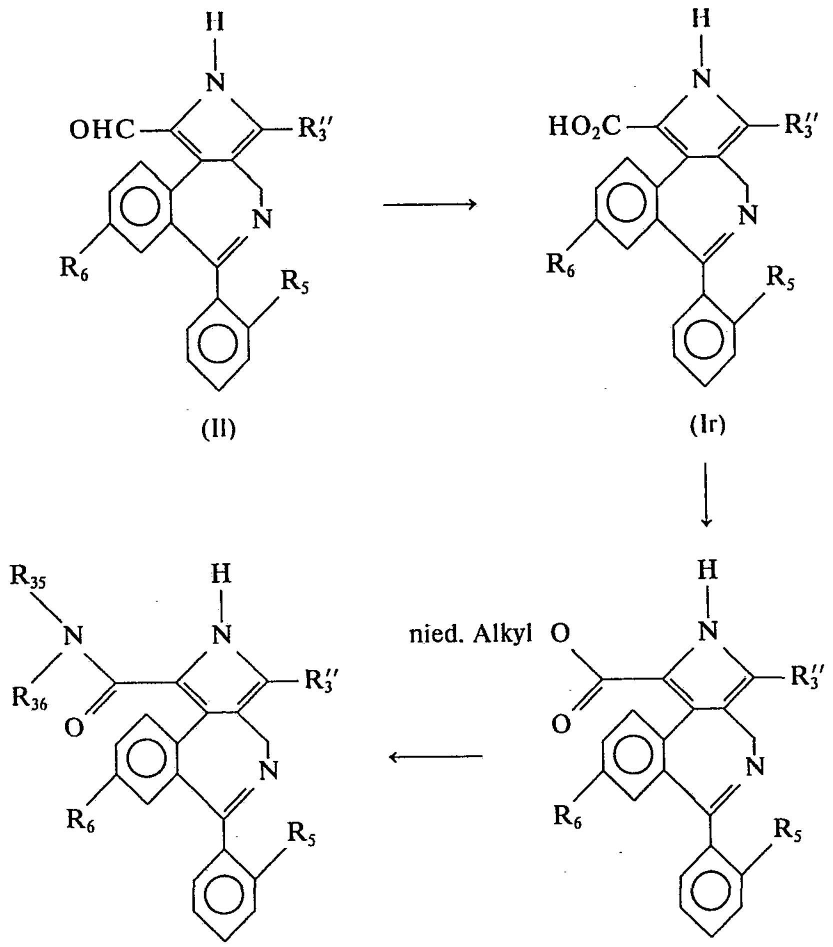

(II) (Ir) (Is) (It)

worin $R_{35}$ und $R_{36}$ je Wasserstoff oder niederes Alkyl und X Halo bedeuten, und $R_1''$ und $R_3''$ obige Bedeutung besitzen.

Ib → In

Eine Verbindung der Formel Ib kann in einem inerten Lösungsmittel, wie Methylenchlorid oder Diäthyläther, mit einem Trihaloacetylhalogenid umgesetzt werden. Die Reaktionstemperatur variiert in einem Bereich von Raumtemperatur bis Siedetemperatur des Lösungsmittels und liegt vorzugsweise bei der Siedetemperatur des Lösungsmittels.

In → Io

Eine Verbindung der Formel In kann mit einem Alkalimetallalkoxid umgesetzt werden und zwar unter Verwendung des entsprechenden Alkohols als Lösungsmittel. Die Reaktionstemperatur liegt gewöhnlich zwischen 0° C und Raumtemperatur.

In → Ip

Eine Verbindung der Formel In kann in einem $C_1$- bis $C_4$-Alkohol mit Ammoniak oder einem entsprechenden Amin umgesetzt werden. Die Reaktionstemperatur variiert in einem Bereich von Raumtemperatur bis 100° C und beträgt vorzugsweise 100° C. In jenen Fällen, in welchen die Reaktanden Siedepunkte besitzen, welche niedriger sind als die Reaktionstemperatur, verwendet man eine Druckapparatur, um die Reaktanden zurückzuhalten.

Ij → Iq und Il → Ir

Verbindungen der Formeln Ij und Il können in einer Mischung aus Wasser und einem mit Wasser mischbaren, inerten Lösungsmittel, wie Aceton, Tetrahydrofuran, etc., mit Kaliumpermanganat umgesetzt werden. Die Reaktionstemperatur variiert in einem Bereich von etwa 0°C bis 60°C und liegt vorzugsweise bei Raumtemperatur.

Iq → Io und Ir → It

Verbindungen der Formeln Iq und Ir können in einem inerten Lösungsmittel, wie Methylenchlorid, oder einem tiefsiedenden Äther, wie Diäthyläther, Tetrahydrofuran, etc., mit einem Diazoalkan, wie Diazomethan oder Diazoäthan, umgesetzt werden. Die Reaktion wird gewöhnlich zwischen etwa 0°C und Raumtemperatur durchgeführt.

Andererseits kann man eine Verbindung der Formel Iq und Ir mit dem entsprechenden Alkohol und einer katalytischen Menge einer starken Mineralsäure, wie Schwefelsäure, oder einer organischen Säure, wie p-Toluolsulfonsäure, umsetzen. Die Reaktionstemperatur variiert in einem Bereich von Raumtemperatur bis Siedetemperatur des Alkohols und liegt vorzugsweise bei Raumtemperatur.

Io → Ip und It → Is

Die Verbindungen der Formeln Io und It können, gegebenenfalls in einem $C_1$- bis $C_4$-Alkohol, mit Ammoniak oder einem mono- oder di-niederen Alkylamin und einer katalytischen Menge des entsprechenden Hydrochlorides umgesetzt werden. Die Reaktionstemperatur variiert in einem Bereich von Raumtemperatur bis 100°C und beträgt vorzugsweise 100°C. In jenen Fällen, in denen die Reaktanden oder Lösungsmittel Siedepunkte besitzen, welche niedriger als 100°C sind, verwendet man eine Druckapparatur, um die Reaktanden zurückzuhalten.

Schema VII

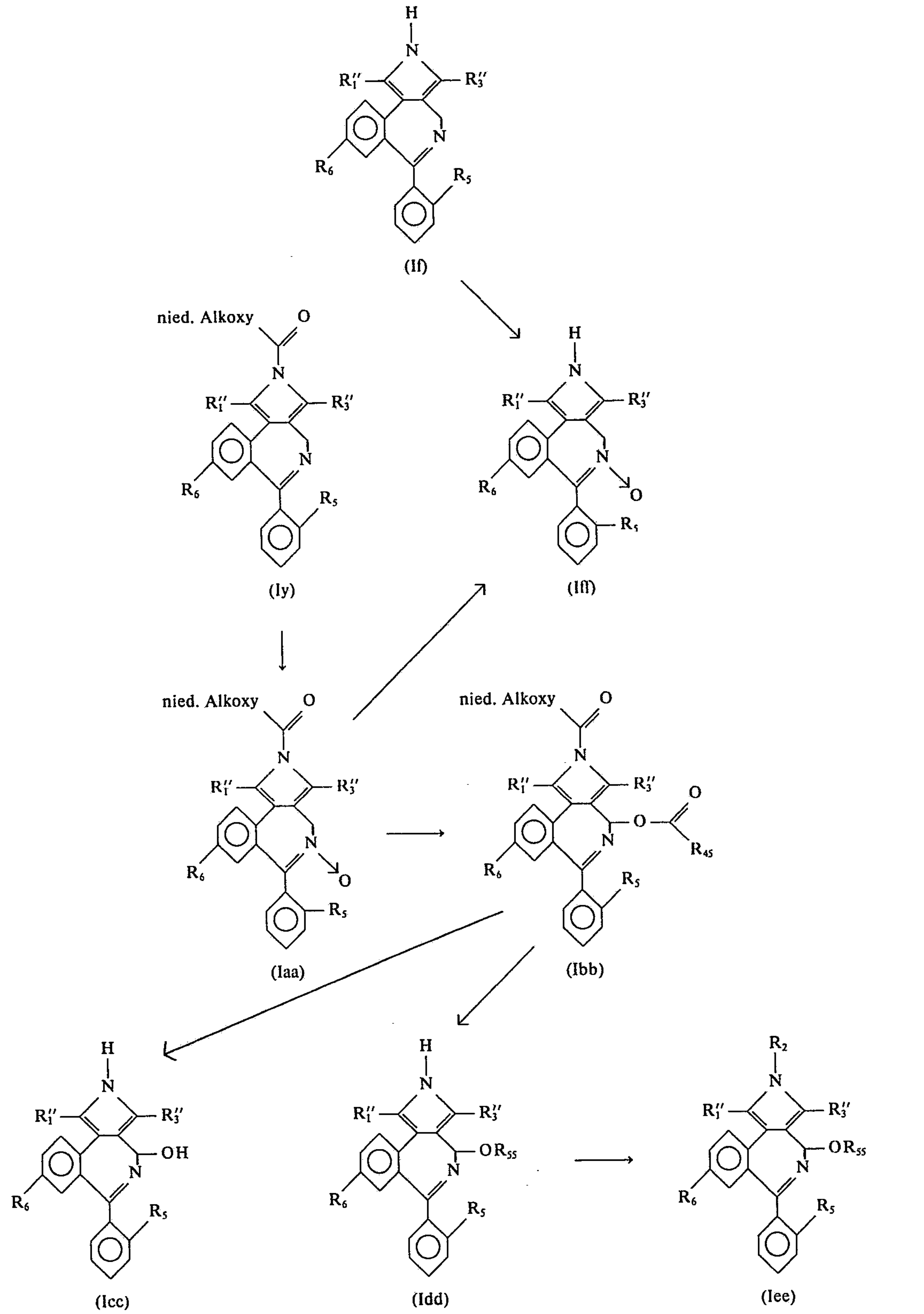

(If) (Iy) (Iff) (Iaa) (Ibb) (Icc) (Idd) (Iee)

worin $R_{45}$ niederes Alkyl oder Trifluormethyl und $R_{55}$ geradkettiges niederes Alkyl bedeuten, und $R_2$, $R_1''$, $R_3''$, $R_5$ und $R_6$ obige Bedeutung besitzen.

Iy → Iaa

Eine Verbindung der Formel Iy kann in einem inerten Lösungsmittel, wie Methylenchlorid, mit einer Persäure, wie m-Chlorperbenzoesäure, Pertrifluoressigsäure, etc., umgesetzt werden. Die Reaktionstemperatur variiert in einem Bereich von 0°C bis Siedetemperatur des Lösungsmittels und liegt vorzugsweise bei Raumtemperatur.

Iaa → Ibb

Eine Verbindung der Formel Iaa kann mit einem von einer geeigneten Carbonsäure abgeleiteten Carbonsäureanhydrid, wie Essigsäure- oder Trifluoressigsäureanhydrid, umgesetzt werden. Die Reaktion wird vorzugsweise bei etwa Siedetemperatur des gewählten Anhydrids durchgeführt, beträgt jedoch nicht mehr als 130° C.

Ibb → Icc

Eine Verbindung der Formel Ibb kann in einem wäßrigen, ätherischen Lösungsmittel, z. B. einer Mischung aus Wasser und Dioxan oder Tetrahydrofuran, mit einem Alkalimetallhydroxid, z. B. mit Natrium- oder Kaliumhydroxid, umgesetzt werden. Die Reaktion kann in einem Bereich von etwa 0° C bis Raumtemperatur, wobei Raumtemperatur bevorzugt wird, durchgeführt werden.

Ibb → Idd

Eine Verbindung der Formel Ibb kann in einer Mischung aus einem $C_1$- bis $C_7$-geradkettigen Alkohol und einem Äther, z. B. Tetrahydrofuran oder Dioxan, mit einem Alkalimetallhydroxid oder -alkoxid, z. B. mit Natrium- oder Kaliumhydroxid oder -methoxid, umgesetzt werden. Man arbeitet dabei in einem Temperaturbereich von etwa 0° C bis Raumtemperatur, vorzugsweise bei 0° C.

Idd → Iee

Eine Verbindung der Formel Idd kann anschließend in Analogie zu den im Zusammenhang mit Reaktionsschema IV beschriebenen Reaktionsschritten umgesetzt werden, d. h. in Analogie zu den Reaktionsschritten If → Iu; If → Iv; If → Iw; Iu → Iv; Iu → Iw; If → Ix; If → Iy; If → Iz und Iv → IX.

If → Iff

Eine Verbindung der Formel If kann in einem inerten Lösungsmittel, wie Methylenchlorid, mit einer Persäure, wie m-Chlorperbenzoesäure, Pertrifluoressigsäure, etc., umgesetzt werden. Die Reaktionstemperatur variiert in einem Bereich von etwa −20° C bis Raumtemperatur und beträgt vorzugsweise 0° C. Weil dies im obigen Formelschema nicht gezeigt ist, ist noch zu erwähnen, daß auch die Verbindungen der Formeln Iu, Iv, Iw und Iz die obige Reaktion eingehen können, d. h. in 5-Stellung oxydiert werden können.

Iaa → Iff

Eine Verbindung der Formel Iaa kann in einer Mischung aus einem $C_1$- bis $C_7$-geradkettigen Alkohol und einem Äther, z. B. Tetrahydrofuran, mit einem Alkalimetallhydroxid oder -carbonat, z. B. Natriumhydroxid oder Kaliumcarbonat umgesetzt werden. Die Reaktion wird in einem Temperaturbereich von etwa 0° C bis Siedetemperatur des Lösungsmittels durchgeführt, wobei man vorzugsweise bei Raumtemperatur arbeitet.

Schema VIII

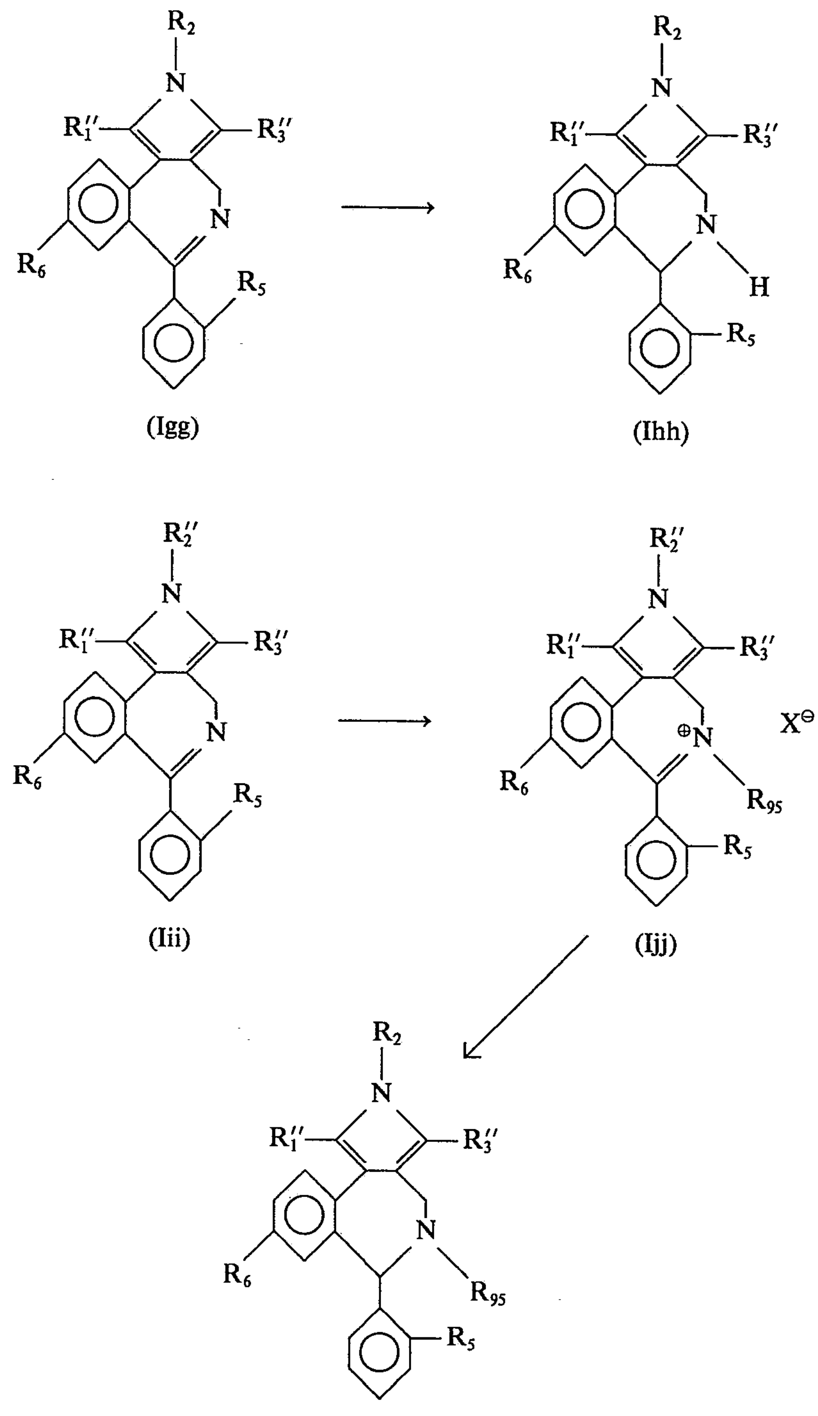

(Igg) (Ihh)

(Iii) (Ijj)

(Ikk)

worin $R_{95}$ niederes Alkyl bedeutet, $R_2''$ dieselbe Bedeutung besitzt wie $R_2$, wobei $R_2''$ nicht Amino-, mono- oder di-niederes Alkylamino—$C_2$- bis $C_7$-Alkyl bedeutet, $R_1''$, $R_2$, $R_3''$, $R_5$ und $R_6$ obige Bedeutung besitzen, und X ein Halogenid- oder Sulfonatrest bedeutet.

Igg → Ihh

Eine Verbindung der Formel Igg kann in einem eine Mineralsäure enthaltenden $C_1$- bis $C_3$-Alkohol mit einem Reduktionsmittel, wie Natriumcyanoborhydrid, oder mit Zink in Essigsäure umgesetzt werden. Die Reaktionstemperatur variiert in einem Bereich von —20° C bis Raumtemperatur und beträgt vorzugsweise 0° C.

Iii → Ijj

Eine Verbindung der Formel Iii kann in einem polaren aprotischen Lösungsmittel, wie Dimethylformamid oder Dimethylsulfoxid, mit einem niederen Alkylhalogenid oder -sulfonat umgesetzt werden. Die Reaktionstemperatur variiert in einem Bereich von 0° C bis 40° C und liegt vorzugsweise bei Raumtemperatur. Wenn $R_2''$ in einer Verbindung der Formel Iii Wasserstoff bedeutet und die Alkylierung in Gegenwart einer Base durchgeführt wird, dann hat $R_2''$ in einer Verbindung der Formel Ijj die gleiche Bedeutung wie $R_{95}''$.

Ijj → Ikk

Eine Verbindung der Formel Ijj kann in einem $C_1$- bis $C_3$-Alkohol mit einem Reduktionsmittel, wie Natriumborhydrid oder Natriumcyanoborhydrid, umgesetzt werden. Die Reaktionstemperatur variiert in einem Bereich von —20° C bis Raumtemperatur und liegt vorzugsweise bei Raumtemperatur. Bedeutet $R_2''$ ein $C_2$- bis $C_7$-Carbonsäureamid (Iv), so kann man ein stärkeres Reduktionsmittel, wie Lithiumaluminiumhydrid, in einem anderen Lösungsmittel, wie Tetrahydrofuran, verwenden, um Verbindungen der Formel Ikk zu erhalten, worin $R_2$ Amino- oder mono- oder di-niederes Alkylamino—$C_2$- bis $C_7$-Alkyl bedeutet. Die Reaktionstemperatur variiert in einem Bereich von —78° C bis Raumtemperatur und beträgt vorzugsweise 0° C.

Verbindungen der Formel I' sind bevorzugt, insbesondere solche, worin $R_3$ Wasserstoff und $R_5$ und $R_6$ Halogen bedeuten (ganz besonders solche, worin $R_5$ Chlor oder Fluor und $R_6$ Chlor bedeutet), $R_1$ Wasserstoff oder niederes Alkyl bedeutet, $R_2$ obige Bedeutung besitzt und $R_4$ Wasserstoff bedeutet.

Ganz besonders bevorzugte Verbindungen sind:

8-Chlor-6-(2-chlorphenyl)-2 H,4 H-pyrrolo[3,4-d] [2]benzazepin,
8-Chlor-6-(2-fluorphenyl)-2 H,4 H-pyrrolo[3,4-d] [2]benzazepin,
8-Chlor-6-(2-chlorphenyl)-1-methyl-2 H,4 H-pyrrolo[3,4] [2]benzazepin,
8-Chlor-6-(2-chlorphenyl)-2-methyl-2 H,4 H-pyrrolo[3,4-d] [2]benzazepin,
8-Chlor-6-(2-fluorphenyl)-2 H,4 H-pyrrolo[3,4-d] [2]benzazepin-2-äthanol und
8-Chlor-6-(2-chlorphenyl)-2 H,4 H-pyrrolo[3,4-d] [2]benzazepin-2-carbonsäure-methylester.

Der Ausdruck »pharmazeutisch annehmbare Salze« umfaßt sowohl Salze mit anorganischen als auch Salze mit organischen, pharmazeutisch annehmbaren starken Säuren, wie Schwefelsäure, Salzsäure, Salpetersäure, Methansulfonsäure und p-Toluolsulfonsäure. Im Hinblick auf den Stand der Technik und der Natur der Verbindung, von welcher ein Salz gewünscht wird, können solche Salze von jedem Fachmann leicht hergestellt werden.

Die erfindungsgemäßen Pyrrolo[3,4-d] [2]benzazepine sind wertvolle Wirkstoffe und besitzen sedative und anxiolytische Eigenschaften. Diese Verbindungen können in Form herkömmlicher pharmazeutischer Präparate verwendet werden; diese Stoffe können z. B. mit herkömmlichen organischen oder anorganischen, inerten, für die parenterale oder enterale Verabreichung geeigneten, pharmazeutischen Trägern, wie z. B. Wasser, Gelatine, Lactose, Stärke, Magnesiumstearat, Talk, pflanzliche Öle, Gummi arabicum, Polyalkylenglykole, Vaselin oder dergleichen, vermischt werden. Sie können in herkömmlichen pharmazeutische Dosierungsformen verabreicht werden, z. B. als feste Formen, z. B. als Tabletten, Dragées, Kapseln, Suppositorien oder dergleichen, oder in flüssigen Formen, z. B. als Lösungen, Suspensionen oder Emulsionen. Außerdem können die pharmazeutischen Präparate, welche einen erfindungsgemäßen Stoff enthalten, herkömmlichen pharmazeutischen Nachbehandlungsmaßnahmen, wie Sterilisation, unterworfen werden und können herkömmliche pharmazeutische Hilfsstoffe, wie Konservierungsmittel, Stabilisierungsmittel, Netzmittel, Emulgiermittel, Salze, zur Veränderung des osmotischen Druckes, oder Puffer enthalten. Die Präparate können auch andere therapeutisch wertvolle Substanzen enthalten.

Eine geeignete pharmazeutische Dosierungseinheit kann etwa 1 bis 500 mg eines erfindungsgemäßen Benzazepins enthalten. Für die orale Verabreichung wird ein Dosierungsbereich von etwa 1 bis 100 mg und für die parenterale Verabreichung ein Dosierungsbereich von etwa 1 bis 50 mg bevorzugt. Die spezifische Dosierung richtet sich jedoch in jedem Fall nach den individuellen Bedürfnissen und dem Urteil der Person, die die erfindungsgemäßen Produkte verabreicht bzw. deren Verabreichung überwacht. Es sei an dieser Stelle betont, daß die obigen Dosierungsangaben lediglich als Beispiele dienen und in keiner Weise den Umfang der vorliegenden Erfindung oder deren Anwendbarkeit einschränken sollen.

Der in der vorliegenden Beschreibung verwendete Ausdruck »Dosierungseinheit« bezeichnet diskrete pharmazeutische Einheiten, welche zusammen mit dem benötigten pharmazeutischen Verdünnungsmittel, Träger oder Vehikel eine vorbestimmte Menge eines aktiven erfindungsgemäßen Produktes enthalten und welche als Einzeldosis in einem Säuger den gewünschten therapeutischen Effekt bewirkt.

Die folgenden, in wohlbekannten Versuchen ermittelten pharmakologischen Daten erläutern die pharmakologischen Eigenschaften der erfindungsgemäßen Pyrrolo-benzazepine.

| Verbindung | Kampftest | Test an der geneigten Ebene | Test an der unanästhesierten Katze |
|---|---|---|---|
| 8-Chlor-6-phenyl-2 H,4 H-pyrrolo[3,4-d] [2]benzazepin | 25 mg/kg | 400 mg/kg | 2,5 mg/kg |
| Toxizität ($DL_{50}$) = >1000 mg/kg (PO) | | | |
| 8-Chlor-6-(2-chlorphenyl)-2 H,4 H-pyrrolo[3,4-d] [2]benzazepin | 1 mg/kg | 400 mg/kg | 0,25 mg/kg |
| Toxizität ($DL_{50}$) = >1000 mg/kg (PO) | | | |
| 8-Chlor-6-(2-fluorphenyl)-1-methyl-2 H,4 H-pyrrolo-[3,4-d] [2]benzazepinmethansulfonat | 2 mg/kg | 400 mg/kg | 2,5 mg/kg |
| Toxzität ($DL_{50}$) = >1000 mg/kg (PO) | | | |

Es folgt eine kurze Beschreibung der obigen Versuche:

## Kampftest

Man bringt zwei Mäuse unter ein umgekehrtes 1-Liter-Becherglas auf ein Gitter, durch welches ihnen Schläge auf die Füße versetzt werden. Dadurch geraten die Tiere in einen Erregungszustand, welcher sich in gelegentlichen kurzen Zweikämpfen äußert. Man wählt Mäusepaare, welche während einer 2minütigen Versuchsperiode mindestens fünfmal gekämpft haben, verabreicht ihnen die Versuchssubstanz auf oralem Wege und wiederholt den Versuch nach 1 Stunde. Hierbei verwendet man logarithmisch ansteigende Dosen bis zu maximal 100 mg/kg. Man definiert als 100-proz. hemmende Dosis diejenige Dosis, welche bei 3 von 3 Mäusepaaren einen Kampf verhindert.

## Test an der geneigten Ebene

Man verabreicht jeweils 6 Mäusen die Prüfsubstanz (maximale Dosis ist 500 mg/kg) und beobachtet sodann während 4 Stunden das Verhalten der Mäuse an der geneigten Ebene auf paralytische Erscheinungen, die ein Abgleiten von der Ebene verursachen. Wird eine Wirkung beobachtet, so verwendet man weitere Dosen, bis man mindestens zwei Dosen hat, bei welchen einige, jedoch nicht alle Tiere von der geneigten Ebene abgleiten. Dosen, bei welchen die Tiere auf Grund von toxischen Erscheinungen oder Erregungszuständen abgleiten, werden für die Berechnung des $PD_{50}$-Wertes nicht berücksichtigt.

Versuch an der wachen Katze

Man behandelt Katzen auf oralem Wege und beobachtet, ob Symptome auftreten (gewöhnlich Ataxie). Zunächst verwendet man eine Katze und eine Dosis von 50 mg/kg. Wenn sich eine Wirkung zeigt, so variiert man die Dosen und benützt bis zu 3 Kapseln pro Dosis. Die Resultate zeigen die minimale wirksame Dosis.

Die folgenden Beispiele illustrieren die vorliegende Erfindung; sie sollen sie jedoch in keiner Weise einschränken. Sofern nichts anderes angegeben ist, sind alle Temperaturen in Celsiusgraden angegeben.

Beispiel 1

a) Man versetzt eine Lösung von 5,0 g Kupfersulfat in 3 l konzentriertem Ammoniak mit 300 g (4,6 Mol) aktiviertem Zinkstaub und 100 g (0,42 Mol) 2-Benzoyl-4-chlorbenzoesäure, erhitzt die Mischung während 3 Tagen unter Rückfluß zum Sieden, wobei man das Volumen durch Zugabe von konzentriertem Ammoniak konstant hält, kühlt die Mischung ab und entfernt das überschüssige Zink durch Filtration. Man säuert das Filtrat durch Zugabe von konzentrierter Salzsäure auf pH 3 an, filtriert den erhaltenen Niederschlag ab und trocknet zum konstanten Gewicht. Man erhält 2-Benzyl-4-chlorbenzoesäure als weißen Festkörper vom Schmelzpunkt 142—144° C.

b) Man versetzt eine auf 0° gekühlte Lösung von 28,4 g (0,75 Mol) Lithiumaluminiumhydrid in 800 ml Äther tropfenweise mit einer Lösung von 85,1 g (0,345 mMol) 2-Benzyl-4-chlorbenzoesäure in 250 ml Äther, läßt die Mischung auf Raumtemperatur erwärmen, rührt während 2 Stunden und zerstört das überschüssige Lithiumaluminiumhydrid durch Zugabe von 28,5 ml Wasser, 28,5 ml 10-proz. wäßrige Natronlauge und 85,5 ml Wasser. Man entfernt den erhaltenen Niederschlag durch Filtration, trocknet das Filtrat über Natriumsulfat und entfernt den Äther unter vermindertem Druck. Man erhält 2-Benzyl-4-chlorbenzylalkohol als farbloses Öl, das beim Stehenlassen kristallisiert und einen Schmelzpunkt von 46,5—49° aufweist.

c) Man versetzt eine Suspension von 238 g (1,1 Mol) Pyridiniumchlorochromat in 800 ml Methylenchlorid mit 79,3 g (0,34 Mol) 2-Benzyl-4-chlorbenzylalkohol, rührt die Mischung während 2 Stunden bei Raumtemperatur und fällt die Chromsalze durch Zugabe von 2,4 l einer 1 : 1-Mischung von Äther und Petroläther aus. Man filtriert durch Kieselgur und entfernt das Lösungsmittel unter vermindertem Druck. Man erhält 2-Benzyl-4-chlorbenzaldehyd als gelbes Öl, das ohne weitere Reinigung in die nächste Stufe eingesetzt wird.

d) Man versetzt eine Suspension von 10,5 g (0,437 Mol) Natriumhydrid (frei von Mineralöl) in 1,2 l Tetrahydrofuran tropfenweise mit 58,4 g (0,328 Mol) Diäthylcyanomethylphosphonat und, nach Beendigung der Wasserstoffentwicklung (nach etwa 60 Minuten), tropfenweise mit einer Lösung von 69,4 g (0,3 Mol) 2-Benzyl-4-chlorbenzaldehyd in 75 ml Tetrahydrofuran. Man rührt die Mischung über Nacht bei Raumtemperatur, dekantiert die Tetrahydrofuranlösung ab und dampft bei Raumtemperatur ein. Man verteilt den Rückstand zwischen 2 l Wasser und 1,5 l Äther, trennt die Ätherphase ab, wäscht mit Wasser und trocknet über Natriumsulfat. Nach Entfernen des Äthers unter vermindertem Druck erhält man 3-(2-Benzyl-4-chlorphenyl)-2-propennitril als gelbes Öl, das ohne weitere Reinigung in die nächste Stufe eingesetzt wird.

e) Man rührt eine Mischung von 28,8 g (0,14 Mol) 3-(2-Benzyl-4-chlorphenyl)-2-propennitril, 50 g (0,5 Mol) Chromtrioxid, 100 ml Methylenchlorid und 300 ml Essigsäure über Nacht bei Raumtemperatur, zerstört das überschüssige Chromtrioxid durch langsame Zugabe von 30 ml Äthanol, verdünnt die erhaltene Mischung mit 800 ml Wasser und schüttelt mit 500 ml Äther aus. Man wäscht die Ätherlösung mit Wasser, gesättigter wäßriger Natriumbicarbonatlösung und gesättigter wäßriger Kochsalzlösung, trocknet über wasserfreiem Natriumsulfat und dampft unter vermindertem Druck ein, wobei man 3-(2-Benzoyl-4-chlorphenyl)-2-propennitril als gelbes Öl erhält, das ohne weitere Reinigung in die nächste Stufe eingesetzt wird.

Eine Probe dieses Stoffes wird durch präparative Schichtchromatographie an einer 2 mm dicken Kieselgelschicht unter Entwickeln mit einer 1 : 1-Mischung von Methylenchlorid und Pentan gereinigt. Man erhält dabei 3-(2-Benzoyl-4-chlorphenyl)-2-propennitril als weißen Festkörper vom Schmelzpunkt 87—89°.

Dieser Stoff kann auch wie folgt hergestellt werden:

d') Man gibt unter Rühren eine Lösung von 92,7 g (0,4 Mol) 2-Amino-5-chlorbenzophenon in 250 ml Acetonitril zu einer Mischung aus 70 g (0,52 Mol) Kupferchlorid, 65 g (0,63 Mol) t-Butylnitrit, 500 ml Acrylnitril und 500 ml Acetonitril, rührt nach beendeter Zugabe noch während 2 Stunden bei Raumtemperatur, verdünnt die erhaltene Mischung mit 80 ml 6 N-Salzsäure und 1,5 l Wasser, schüttelt mit Äther aus und trocknet die Ätherlösung über wasserfreiem Natriumsulfat. Nach Eindampfen der Ätherlösung unter vermindertem Druck erhält man ein braunes Öl, das $\alpha$-Dichlor-2-(benzoyl)-benzolpropannitril und 2,5-Dichlorbenzophenon enthält. Durch Behandeln dieses Öls mit einer Mischung aus Äther und Petroläther erhält man das gewünschte Produkt als bräunlichen Festkörper.

Durch Umkristallisieren einer kleinen Portion aus einer Mischung aus Äther und Petroläther erhält

man α,4-Dichlor-2-(benzoyl)-benzolpropannitril als hellgelbe Nadeln vom Schmelzpunkt 69—71°.

e′) Man rührt eine Mischung aus 50,9 g (0,168 Mol) α,4-Dichlor-2-(benzoyl)-benzolpropannitril, 17 g (0,14 Mol) Kaliumcarbonat, 50,9 g (0,5 Mol) Kaliumbicarbonat und 510 ml Dimethylsulfoxid während 48 Stunden bei Raumtemperatur, verdünnt die erhaltene Mischung mit 1,5 l Wasser und filtriert den ausgefallenen Festkörper ab. Durch Umkristallisieren aus einer Mischung von Methylenchlorid und Äther erhält man 3-(2-Benzoyl-4-chlorphenyl)-2-propennitril als weißliche Prismen vom Schmelzpunkt 89—91°.

f) Man gibt eine Mischung von 10,7 g (40 mMol) 3-(2-Benzoyl-4-chlorphenyl)-2-propennitril, 5,3 g (38 mMol) Tosylmethylisocyanid, 75 ml Dimethylsulfoxid und 150 ml Äther tropfenweise zu einer Suspension von 3,7 g (77 mMol) Natriumhydrid (50-proz. Öldispersion) in 170 ml Äther, rührt nach beendeter Zugabe noch während 2 Stunden, verdünnt die erhaltene Mischung mit Wasser und trennt die ätherische Phase ab. Nach Ausschütteln der wäßrigen Phase mit Äther werden die vereinigten organischen Auszüge mit Wasser gewaschen, über wasserfreiem Natriumsulfat getrocknet und unter vermindertem Druck zu einem dunkelgrünen Öl eingedampft. Man reinigt dieses Öl durch Säulenchromatographie an 800 g Kieselgel unter Eluieren mit 5% Äther in Methylenchlorid und erhält 4-(2-Benzoyl-4-chlorphenyl)-1 H-pyrrol-3-carbonnitril als weißliche Prismen vom Schmelzpunkt 175—177°.

g) Man hydriert eine Mischung aus 4,0 g (13 mMol) 4-(2-Benzoyl-4-chlorphenyl)-1 H-pyrrol-3-carbonitril, 4 g Raney-Nickel und 300 ml Essigsäure während 4 Stunden in einem Parr-Apparat, filtriert das Raney-Nickel ab und verdünnt das Filtrat mit 400 ml Eiswasser. Man neutralisiert die Essigsäure mit Natriumbicarbonat, extrahiert die erhaltene Lösung mit Methylenchlorid, wäscht die Methylenchloridlösung mit Wasser und trocknet über Natriumsulfat. Durch Eindampfen dieser Lösung erhält man 8-Chlor-6-phenyl-2 H,4 H-pyrrolo[3,4-d] [2]benzazepin als gelben Festkörper, der nach Umkristallisieren aus Methylenchlorid/Äther einen weißen Festkörper vom Schmelzpunkt 203—206° liefert.

Beispiel 2

a) α,4-Dichlor-2-(2-fluorbenzoyl)-benzolpropannitril wird auf die gleiche Weise hergestellt wie α,4-Dichlor-2-(benzoyl)-benzolpropannitril. Man erhält hellgelbe Prismen vom Schmelzpunkt 94—95°.

b) 3-[2-(2-Fluorbenzoyl)-4-chlorphenyl]-2-propennitril wird auf die gleiche Weise hergestellt wie 3-(2-Benzoyl-4-chlorphenyl)-2-propennitril. Man erhält weißliche Prismen vom Schmelzpunkt 137—139°.

Dieser Stoff kann auch wie folgt hergestellt werden:

b′) Man erhitzt eine Lösung von 5,0 g (14 mMol) 5-Chlor-2′-fluor-2-jodbenzophenon, 2 ml (14,3 mMol) Triäthylamin, 2 ml (30 mMol) Acrylnitril und 35 mg (1,5 mMol) Palladiumacetat während 16 Stunden unter einer Argonatmosphäre und unter Rückfluß zum Sieden. Die Mischung wird anschließend mit 100 ml 1 N-Salzsäure verdünnt. Man filtriert den erhaltenen Niederschlag ab, wäscht mit Äther und trocknet an der Luft. Man erhält 3-[2-(2-Fluorbenzoyl)-4-chlorphenyl]-2-propennitril als weißlichen Festkörper vom Schmelzpunkt 130—133°.

c) 4-[2-(2-Fluorbenzoyl)-4-chlorphenyl]-1 H-pyrrol-3-carbonitril wird auf die gleiche Weise hergestellt wie 4-(2-Benzoyl-4-chlorphenyl)-1 H-pyrrol-3-carbonitril. Man erhält weißliche Prismen vom Schmelzpunkt 177—179°.

d) Man hydriert eine Mischung aus 3,0 g (9 mMol) 4-[2-(2-Fluorbenzoyl)-4-chlorphenyl]-1 H-pyrrol-3-carbonitril, ca. 3 g Raney-Nickel und 150 ml Eisessig während 6 Stunden in einem Parr-Aparat bei einem Druck von 3,45 bar, filtriert das Raney-Nickel ab und entfernt die Essigsäure unter vermindertem Druck, wobei man ein gelbes Öl erhält. Man gießt das gelbe Öl auf Eis, stellt mit Ammoniak basisch und schüttelt mit Methylenchlorid aus. Man trocknet die Methylenchloridlösung über wasserfreiem Natriumsulfat und dampft unter vermindertem Druck ein. Durch Umkristallisieren der erhaltenen bräunlichen Kristalle aus einer Mischung von Äther und Methylenchlorid erhält man 8-Chlor-6-(2-fluorphenyl)-2 H,4 H-pyrrolo[3,4-d] [2]benzazepin als crèmefarbene Prismen vom Schmelzpunkt 197—199°.

Beispiel 3

Das Methansulfonatsalz von 8-Chlor-6-(2-fluorphenyl)-2 H,4 H-pyrrolo[3,4-d] [2]benzazepin kann hergestellt werden, indem man äquimolare Mengen dieses Benzazepins und Methansulfonsäure in Methanol löst und das entstandene Salz durch Zugabe von Äther ausfällt. Durch Umkristallisieren des erhaltenen Salzes aus einer Mischung von Methanol und Äther erhält man 8-Chlor-6-(2-fluorphenyl)-2 H,4 H-pyrrolo[3,4-d] [2]-benzazepin-methansulfonat als orangefarbene Prismen vom Schmelzpunkt 220—221°.

Beispiel 4

a) α,4-Dichlor-2-(2-chlorbenzoyl)-benzolpropannitril wird auf die gleiche Weise hergestellt wie α,4-Dichlor-2-(2-chlorbenzoyl)-benzolpropannitril. Man erhält weißliche Prismen vom Schmelzpunkt 102—102°.

b) 3-[2-(2-Chlorbenzoyl)-4-chlorphenyl]-2-propennitril wird auf die gleiche Weise hergestellt wie 3-(2-Benzoyl-4-chlorphenyl)-2-propennitril. Man erhält weißliche Prismen vom Schmelzpunkt 137—139°.

c) 4-[2-(2-Chlorbenzoyl)-4-chlorphenyl]-1 H-pyrrol-3-carbonitril wird auf die gleiche Weise hergestellt wie 4-(2-Benzoyl-4-chlorphenyl)-1 H-pyrrol-3-carbonitril. Man erhält weißliche Prismen vom Schmelzpunkt 182—184°.

d) 8-Chlor-6-(2-chlorphenyl)-2 H,4 H-pyrrolo[3,4-d] [2]-benzazepin wird auf die gleiche Weise hergestellt wie 8-Chlor-6-(2-fluorphenyl)-2 H,4 H-pyrrolo[3,4-d] [2]benzazepin. Man erhält crèmefarbene Prismen vom Schmelzpunkt 204—206° C.

Beispiel 5

Das Methansulfonatsalz von 8-Chlor-6-(2-chlorphenyl)-2 H,4 H-pyrrolo[3,4-d] [2]benzazepin wird hergestellt, indem man äquimolare Mengen des Benzazepins und Methansulfonsäure in Mathanol löst und das entstandene Salz durch Zugabe von Äther ausfällt. Durch Umkristallisieren aus einer Mischung von Mathanol und Äther erhält man 8-Chlor-6-(2-chlorphenyl)-2 H,4 H-pyrrolo[3,4-d] [2]benzazepin-methansulfonat als orangefarbene Prismen vom Schmelzpunkt 239—241°.

Beispiel 6

a) Man gibt eine Mischung von 34,5 g (121 mMol) 3-[2-(2-Fluorbenzoyl)-4-chlorphenyl]-2-propennitril, 25,3 g (121 mMol) 1-Tosyläthylisocyanid, 200 ml Dimethylsulfoxid und 400 ml Äther tropfenweise zu einer Suspension von 8,9 g (184 mMol) Natriumhydrid (50-proz. Dispersion in Mineralöl) in 150 ml Äther, rührt nach beendeter Zugabe noch während 2 Stunden, verdünnt die erhaltene Mischung mit Wasser und trennt die ätherische Phase ab. Die wäßrige Lösung wird mit Äther ausgeschüttelt. Man wäscht die vereinigten Ätherlösungen mit Wasser, trocknet über wasserfreiem Natriumsulfat und dampft unter vermindertem Druck ein. Das erhaltene dunkle Öl kristallisiert aus Äther und liefert 4-[2-(2-Fluorbenzoyl)-4-chlorphenyl]-5-methyl-1 H-pyrrol-3-carbonitril als weißliche Kristalle.

Durch Umkristallisieren einer Probe dieses Materials aus einer Mischung von Methylenchlorid und Äther erhält man farblose Prismen vom Schmelzpunkt 201—202°.

b) 8-Chlor-6-(2-fluorphenyl)-1-methyl-2 H,4 H-pyrrolo[3,4-d]-[2]benzazepin wird auf die gleiche Weise hergestellt wie 8-Chlor-6-(2-fluorphenyl)-2 H,4 H-pyrrorolo[3,4-d] [2]benzazepin. Man erhält farblose Prismen vom Schmelzpunkt 226—227°.

Beispiel 7

Das Methansulfonatsalz von 8-Chlor-6-(2-fluorphenyl)-1-methyl-2 H,4 H-pyrrolo[3,4-d] [2]benzazepin wird hergestellt, indem man äquimolare Mengen des Benzazepins und Methansulfonsäure in Methanol löst und das entstandene Salz durch Zugabe von Äther ausfällt. Durch Umkristallisieren des Salzes aus einer Mischung von Methanol und Äther erhält man 8-Chlor-6-(2-fluorphenyl)-1-methyl-2 H,4 H-pyrrolo-[3,4-d] [2]benzazepin-methansulfonat als orangefarbene Prismen vom Schmelzpunkt 259—261.

Beispiel 8

Man gibt 0,6 ml Phosphoroxychlorid zu einer mit Eiswasser gekühlten Lösung von 0,6 g (2,05 mMol) 8-Chlor-6-phenyl-2 H,4 H-pyrrolo[3,4-d] [2]benzazepin in 8 ml Dimethylformamid, rührt die Mischung unter Kühlen während 1 Stunde und gießt anschließend auf 10-proz. wäßrige Natriumcarbonatlösung. Man filtriert das ausgefallene Material ab, löst es in Methylenchlorid, trocknet die erhaltene Lösung und dampft ein. Man chromatographiert den Rückstand an 30 g Kieselgel unter Verwendung von 20% (v/v) Essigester in Methylenchlorid als Elutionsmittel. Das weniger polare 8-Chlor-6-phenyl-4 H-pyrrolo[3,4-d] [2]benzazepin-3-carboxaldehyd wird aus Äther kristallisiert und aus Essigester/Hexan umkristallisiert und hat dann einen Schmelzpunkt von 225—226°.

Durch weiteres Eluieren erhält man das langsamer wandernde 8-Chlor-6-phenyl-2 H,4 H-pyrrolo[3,4-d] [2]benzazepin-1-carboxaldehyd, das aus Essigester kristallisiert wird und dann einen Schmelzpunkt von 241—243° aufweist.

### Beispiel 9

Man gibt 5 ml Phosphoroxychlorid zu einer mit Eiswasser gekühlten Lösung von 3 g (0,013 Mol) 8-Chlor-6-phenyl-2 H,4 H-pyrrolo[3,4-d] [2]benzazepin in 40 ml Dimethylformamid, rührt unter Kühlen während 1 Stunde und gießt die Reaktionsmischung anschließend auf 10proz. wäßrige Natriumcarbonatlösung. Man filtriert das ausgefallene Material ab, nimmt es in Methylenchlorid auf, trocknet die erhaltene Lösung und dampft ein. Man nimmt den Rückstand in 100 ml Äthanol auf, versetzt mit 0,8 g Natriumborhydrid und rührt während 30 Minuten bei Raumtemperatur. Anschließend wird das Lösungsmittel teilweise unter vermindertem Druck abgedampft, worauf man den Rückstand zwischen Methylenchlorid und Wasser verteilt. Die organische Phase wird getrocknet und eingedampft; und der erhaltene Rückstand wird an 250 g Kieselgel unter Verwendung von Methylenchlorid/Essigwasser (2 : 3) chromatographiert.

Das zuerst eluierte 8-Chlor-6-phenyl-2 H,4 H-pyrrolo-[3,4-d] [2]benzazepin-1-methanol wird aus Essigester kristallisiert und liefert weißliche Kristalle vom Schmelzpunkt 208—210°.

Das polarere 8-Chlor-6-phenyl-4 H-pyrrolo[3,4-d] [2]-benzazepin-3-methanol wird aus Essigester kristallisiert und liefert weißliche Kristalle vom Schmelzpunkt 216—218°.

### Beispiel 10

Man versetzt eine Lösung von 1,4 g (12,4 mMol) Kalium-t-butoxid in 30 ml trockenem Dimethylformamid mit 2,4 g (8,2 mMol) 8-Chlor-6-phenyl-2 H,4 H-pyrrolo[3,4-d] [2]benzazepin, kühlt die Mischung auf 0° ab, rührt während 10 Minuten versetzt mit 1,3 ml (11,7 mMol) Äthyl-bromacetat und rührt die erhaltene Mischung während 45 Minuten bei 0°. Man verdünnt mit Wasser, schüttelt mit Äther aus, wäscht die Ätherlösung mit Wasser, trocknet über Natriumsulfat und dampft unter vermindertem Druck ein. Man reinigt das erhaltene gelbe Öl durch Säulenchromatographie an 70 g Kieselgel unter Eluieren mit Methylenchlorid/Äther (9 : 1) und erhält als Hauptkomponente 8-Chlor-6-phenyl-2 H,4 H-pyrrolo[3,4-d] [2]benzazepin-2-essigsäure-äthylester als gelbes Öl.

Eine zweite Komponente besteht aus nicht umgesetztem Ausgangsmaterial mit einem Schmelzpunkt von 202—204°.

### Beispiel 11

Eine Mischung aus 2,3 g (6,5 mMol) 8-Chlor-6-phenyl-2 H,4 H-pyrrolo[3,4-d] [2]benzazepin-2-essigsäure-äthylester und 20 ml methanolischem Ammoniak (ca. 20%, v/v) wird in einem Autoklaven über Nacht erhitzt (Dampfbad). Nach Entfernen des Lösungsmittels unter vermindertem Druck erhält man einen hellgelben Festkörper, der nach Umkristallisieren aus Äthanol/Hexan 8-Chlor-6-phenyl-2 H,4 H-pyrrolo[3,4-d] [2]benzazepin-2-acetamid als weißen Festkörper vom Schmelzpunkt 236—237° liefert.

### Beispiel 12

Man rührt eine Mischung aus 3,4 g (11 mMol) 8-Chlor-6-(2-fluorphenyl)-1-methyl-2 H,4 H-pyrrolo[3,4-d] [2]benzazepin, 2,6 g (14 mMol) Trichloracetylchlorid und 100 ml Methylenchlorid während 12 Stunden bei Raumtemperatur, filtriert das ausgefallene Material ab und suspendiert es in Methylenchlorid. Man stellt mit Ammoniak basisch, trennt die Methylenchloridlösung ab, trocknet sie über wasserfreiem Natriumsulfat und dampft unter vermindertem Druck ein. Man erhält 8-Chlor-6-(2-fluorphenyl)-1-methyl-3-[(trichlormethyl)-carbonyl]-2 H,4 H-pyrrolo[3,4-d] [2]benzazepin als farblosen Festkörper vom Schmelzpunkt 222—225°. Eine Probe dieses Materials wird aus einer Mischung von Methylenchlorid und Äther umkristallisiert und liefert farblose Prismen vom Schmelzpunkt 222—223°.

### Beispiel 13

Man erhitzt eine Mischung aus 3,0 g (6,4 mMol) 8-Chlor-6-(2-fluorphenyl)-1-methyl-3-[(trichlormethyl)carbonyl]2 H,4 H-pyrrolo[3,4-d] [2]benzazepin, 0,5 ml (2 mMol) einer 4 M-Lösung von Natriummethoxid in Methanol und 100 ml Methanol während 30 Minuten auf 40°, dampft anschließend unter vermindertem Druck ein und verteilt den Rückstand zwischen Methylenchlorid und Wasser. Die Methylenchloridlösung wird über wasserfreiem Natriumsulfat getrocknet und unter vermindertem Druck eingedampft. Man erhält 8-Chlor-6-(2-fluorphenyl)-1-methyl-2 H,4 H-pyrrolo[3,4-d] [2]benzazepin-3 carbonsäure-methylester als bräunliche Nadeln vom Schmelzpunkt 228—231°. Durch Umkristallisieren aus Methylenchlorid erhält man farblose Nadeln vom Schmelzpunkt 229—231°.

Beispiel 14

Man erhitzt während 48 Stunden eine Mischung aus 1,0 g (2,1 mMol) 8-Chlor-6-(2-fluorphenyl)1-1methyl-3-[(trichlormethyl)carbonyl-2 H,4 H-pyrrolo[3,4-d] [2]benzazepin und 70 ml mit Methylamin gesättigtem Methanol in einem Bombenrohr auf dem Dampfbad. Man kühlt die Mischung ab und entfernt das Methanol unter vermindertem Druck, wobei man einen Festkörper erhält. Dieser wird zwischen Methylenchlorid und Wasser verteilt, worauf man die Methylenchloridphase abtrennt, über wasserfreiem Natriumsulfat trocknet und unter vermindertem Druck eindampft. Man erhält 8-Chlor-6-(2-fluorphenyl)-N,1-dimethyl-2 H,4 H-pyrrolo[3,4-d] [2]benzazepin-3-carboxamid als bräunliche Nadeln vom Schmelzpunkt 252—255°. Durch Umkristallisieren aus Methylenchlorid erhält man farblose Nadeln vom Schmelzpunkt 266—268°.

Beispiel 15

Man gibt 20 ml (12,5 mMol) einer 0,62M-Lösung von Lithiumdiisopropylamid in Tetrahydrofuran mittels einer Spritze tropfenweise zu einer auf —20° gekühlten Lösung von 3,1 g (9,6 mMol) 8-Chlor-6-(2-fluorphenyl)-2 H,4 H-pyrrolo[3,4-d] [2]benzazepin in 75 ml trockenem Tetrahydrofuran. Man rührt die Lösung während 5 Minuten bei —20°, versetzt anschließend mit 5,0 ml (55 mMol) Allylbromid, läßt die Mischung auf Raumtemperatur erwärmen und rührt während 2 Stunden. Man versetzt anschließend mit Wasser, schüttelt mit Äther aus, wäscht die Ätherlösung mit Wasser, trocknet über wasserfreiem Natriumsulfat und dampft unter vermindertem Druck zu einem bernsteinfarbenen Öl ein. Man reinigt den Rückstand durch Säulenchromatographie an 70 g Kieselgel unter Eluieren mit 5% Äther in Methylenchlorid und erhält 8-Chlor-6-(2-fluorphenyl)-3-(2-propenyl)-2 H,4 H-pyrrolo[3,4-d] [2]benzazepin als weniger polare Komonente. Durch Umkristallisieren aus einer Mischung von Äther und Petroläther erhält man crèmefarbene Prismen vom Schmelzpunkt 135—140° (Schaumbildung).

Das Methansulfonatsalz dieser Verbindung kann hergestellt werden, indem man äquimolare Mengen der Base und Methansulfonsäure in Methanol löst und das entstandene Salz durch Zugabe von Äther ausfällt. Durch Umkristallisieren aus einer Mischung von Methanol und Äther erhält man 8-Chlor-6-(2-fluorphenyl)-3-(2-propenyl)-2 H,4 H-pyrrolo[3,4-d] [2]benzazepin-methansulfonat als orangefarbene Prismen vom Schmelzpunkt 243—244°.

Schließlich kann man noch Ausgangsmaterial vom Schmelzpunkt 226—227° eluieren, das identisch ist mit authentischen Material.

Beispiel 16

Man gibt in einer Portion 0,7 g (2,2 mMol) 8-Chlor-6-(2-fluorphenyl)-2 H,4 H-pyrrolo[3,4-d] [2]benzazepin zu einer auf 0° gekühlten Lösung von 0,3 g (2,6 mMol) Kalium-t-butoxid in 30 ml trockenem Dimethylformamid, rührt während 15 Minuten, versetzt mit 1,0 ml (11 mMol) Allylbromid und läßt die Mischung auf Raumtemperatur erwärmen. Man versetzt mit Wasser, schüttelt mit Methylenchlorid aus, wäscht die Methylenchloridlösung mit Wasser, trocknet über wasserfreiem Natriumsulfat und dampft unter vermindertem Druck zu einem gelben Öl ein. Man reinigt dieses Öl durch Säulenchromatographie an 20 g Kieselgel unter Eluieren mit 5% Äther in Methylenchlorid und erhält 8-Chlor-6-(2-fluorphenyl)-2-(2-propenyl)-4 H-pyrrolo[3,4-d] [2]benzazepin als gelbes Öl. Durch Kristallisieren aus einer Mischung von Äther und Petroläther erhält man einen bräunlichen Festkörper vom Schmelzpunkt 100—102°, der nach Umkristallisieren aus einer Mischung von Äther und Petroläther farblose Prismen vom Schmelzpunkt 104—106° liefert.

Beispiel 17

In einer Portion gibt man 0,7 g (2,2 mMol) 8-Chlor-6-(2-fluorphenyl)-2 H,4 H-pyrrolo[3,4-d] [2]benzazepin zu einer auf 0° gekühlten Lösung von 0,3 g (2,6 mMol) Kalium-t-butoxid in 30 ml trockenem Dimethylformamid. Man rührt während 15 Minuten und versetzt anschließend mit 1,0 ml (16 mMol) Methyljodid und läßt die Mischung auf Raumtemperatur erwärmen. Man versetzt mit Wasser, extrahiert mit Methylenchlorid, wäscht die Methylenchloridphase mit Wasser, trocknet über wasserfreiem Natriumsulfat und dampft unter vermindertem Druck zu einem gelben Öl ein. Man reinigt durch Säulenchromatographie an 20 g Kieselgel unter Eluieren mit 5% Äther in Methylenchlorid und erhält 8-Chlor-6-(2-fluorphenyl)-2-methyl-2 H,4 H-pyrrolo[3,4-d] [2]benzazepin als bräunlichen Festkörper vom Schmelzpunkt 142—144°. Durch Umkristallisieren aus einer Mischung von Äther und Petroläther erhält man crèmefarbene Prismen vom Schmelzpunkt 144—146°.

## Beispiel 18

a) Man gibt eine Mischung von 3,1 g (11 mMol) 3-[2-(2-Fluorbenzoyl)-4-chlorphenyl]-2-propennitril, 3,0 g (12 mMol) 1-Tosyl-3-butenyl-isocyanid (Am. M. van Leusen, R. J. Bouma and O. Possel, Tetrahedron Letters, 3487 (1975)) und 25 ml Dimethylsulfoxid in 50 ml Äther tropfenweise zu einer Suspension von 0,8 g (16,5 mMol) Natriumhydrid (50proz. Mineralöldispersion) in 100 ml Äther. Nach beendeter Zugabe rührt man noch während 2 Stunden, verdünnt anschließend mit Wasser und trennt die ätherische Phase ab. Man schüttelt die wäßrige Phase mit Äther aus, wäscht die vereinigten ätherischen Auszüge mit Wasser, trocknet über wasserfreiem Natriumsulfat und konzentriert unter vermindertem Druck.

Man reinigt das erhaltene dunkle Öl durch Säulenchromatographie an 150 g Kieselgel unter Eluieren mit 5% Äther in Methylenchlorid und erhält 4-[4-Chlor-2-(2-fluorbenzoylphenyl]-5-(2-propenyl)-1 H-pyrrol-3-carbonitril als farblose Nadeln vom Schmelzpunkt 142—144°. Durch Umkristallisieren aus einer Mischung von Äther und Petroläther erhält man farblose Nadeln vom Schmelzpunkt 142—144°.

b) Man rührt eine Mischung aus 0,7 g (2 mMol) 4-[4-Chlor-2-(2-fluorbenzoyl)phenyl]-5-(2-propenyl)-1 H-pyrrol-3-carbonitril und 0,7 g (18 mMol) Lithiumaluminiumhydrid in 50 ml Tetrahydrofuran während 48 Stunden bei Raumtemperatur, zerstört das überschüssige Lithiumaluminiumhydrid durch tropfenweise Zugabe von gesättigter wäßriger Kaliumnatriumtartratlösung, verdünnt die Mischung mit Methylenchlorid und trennt die Methylenchloridphase ab. Diese wird über wasserfreiem Natriumsulfat getrocknet und unter vermindertem Druck eingedampft, wobei man 4-Aminomethyl-4-/4-chlor-2-[(2-fluorphenyl)hydroxymethyl]phenyl/-2-(2-propenyl)-1 H-pyrrol erhält. Da in diesem Molekül die freie Drehbarkeit eingeschränkt ist, handelt es dabei um eine Mischung, wie man mittels Dünnschichtchromatographie und NMR-Spektrum zeigen kann.

Man erhitzt eine Mischung aus 150 mg (0,4 mMol) 4-Aminomethyl-4-/4-chlor-2-[(2-fluorphenyl)hydroxymethyl]phenyl/-2-(2-propenyl)-1 H-pyrrol und 0,6 g (7,3 mMol) Mangandioxid in 30 ml Tetrahydrofuran während 2 Stunden unter Rückfluß zum Sieden. Die Mischung wird abgekühlt und durch Kieselgur filtriert. Nach Eindampfen des Filtrates unter vermindertem Druck wird das erhaltene rote Öl durch Säulenchromatographie an 10 g Kieselgel unter Eluieren mit 5% Äther in Methylenchlorid gereinigt. Man erhält 8-Chlor-6-(2-fluorphenyl)-1-(2-propenyl)-2 H,4 H-pyrrolo[3,4-d] [2]benzazepin als bräunlichen Festkörper vom Schmelzpunkt 212—214°. Durch Umkristallisieren aus einer Mischung von Äther und Methylenchlorid erhält man farblose Prismen vom Schmelzpunkt 213—215°, welche in jeder Beziehung mit authentischem Material identisch sein.

## Beispiel 19

Man rührt eine Mischung aus 1,0 g (3 mMol) 4-[4-Chlor-3-(2-fluorbenzoyl)phenyl]-5-methyl-1 H-pyrrol-3-carbonitril, 1,0 g (26 mMol) Lithiumaluminiumhydrid und 100 ml Tetrahydrofuran während 24 Stunden bei Raumtemperatur, zerstört das überschüssige Lithiumaluminiumhydrid durch tropfenweise Zugabe von gesättigter wäßriger Kaliumnatriumtartratlösung, verdünnt die Mischung mit Methylenchlorid und trennt die Methylenchloridphase ab. Diese wird über wasserfreiem Natriumsulfat getrocknet und unter vermindertem Druck eingedampft, wobei man 4-Aminomethyl-3-/4-chlor-2-[(2-fluorphenyl)hydroxymethyl]phenyl/-3-methyl-1 H-pyrrol erhält. Es handelt sich dabei um eine Mischung, da die freie Drehbarkeit in diesem Molekül eingeschränkt ist. Dies kann mittels Dünnschichtchromatographie und NMR-Spektrum gezeigt werden.

Man erhitzt eine Mischung aus 150 mg (0,43 mMol) 4-Aminomethyl-3-/4-Chlor-2-[(2-fluorphenyl)hydroxymethyl]phenyl/-2-methyl-1 H-pyrrol, 600 mg (7,3 mMol) Mangandioxid und 30 ml Tetrahydrofuran während 2 Stunden unter Rückfluß zum Sieden. Anschließend wird die Mischung abgekühlt und durch Kieselgur filtriert. Man dampft das Filtrat unter vermindertem Druck ein, wobei man ein bernsteinfarbenes Öl erhält, das mittels Säulenchromatographie an 10 g Kieselgel unter Eluieren mit 5% Äther in Methylenchlorid gereinigt wird. Der dabei erhaltene crèmefarbene Festkörper liefert nach Umkristallisieren aus Äther 8-Chlor-6-(2-fluorphenyl)-1-methyl-2 H,4 H-pyrrolo[3,4-d] [2]benzazepin als farblose Nadeln vom Schmelzpunkt 226—227°. Dieses Material ist in jeder Beziehung identisch mit authentischem Material.

## Beispiel 20

Man gibt 7 ml (4,2 mMol) einer 0,62 M-Lösung von Lithiumdiisopropylamid in Tetrahydrofuran mittels einer Spritze tropfenweise zu einer auf —20° gekühlten Lösung von 1,3 g (4,0 mMol) 8-Chlor-6-(2-fluorphenyl)-1-methyl-2 H,4 H-pyrrolo[3,4-d] [2]benzazepin in 30 ml trockenem Tetrahydrofuran. Man rührt die erhaltene Lösung während 5 Minuten bei —20°, versetzt anschließend mit 2,4 ml (25 mMol) 80proz. Propargylbromid in Toluol, läßt die Mischung auf Raumtemperatur erwärmen und rührt noch während 2 Stunden. Man versetzt mit Wasser und extrahiert die Mischung mit Äther. Die Ätherphase wird mit Wasser gewaschen, über trockenem Natriumsulfat getrocknet und unter vermindertem Druck zu einem bernsteinfarbenen Schaum eingedampft. Man reinigt dieses Material mittels Säulenchromatographie an 20 g Kieselgel unter Eluieren mit 5% Äther in Methylenchlorid und erhält 8-Chlor-6-(2-fluorphenyl)-1-methyl-3-(2-propinyl)-2 H,4 H-pyrrolo[3,4-d] [2]benzazepin als Schaum. Durch Umkristallisieren aus einer Mischung von Äther und Petroläther erhält man crèmefarbene Prismen vom Schmelzpunkt 179—180°.

Beispiel 21

a) Man gibt eine Mischung von 33,9 g (0,11 Mol) 3-[2-(2-Chlorbenzoyl)-4-chlorphenyl]-2-propennitril und 20 g (0,96 mMol) 1-Tosyläthylisocyanid in einer Mischung aus 150 ml Dimethylsulfoxid und 190 ml Äther tropfenweise zu einer Suspension von 4,6 g (0,1 Mol) Natriumhydrid (50proz. Dispersion in Mineralöl) in 100 ml Äther, welche sich in einem Wasserbad mit Raumtemperatur befindet. Man rührt noch während 2 Stunden bei Raumtemperatur, verdünnt die Mischung anschließend mit 1,2 l Wasser und 40 ml 1 N-Salzsäure und schüttelt die Methylenchlorid aus. Man wäscht die Methylenchloridlösung mit Wasser, trocknet über wasserfreiem Natriumsulfat und dampft unter vermindertem Druck ein. Man kristallisiert das erhaltene dunkelgrüne Öl aus einer Mischung von Äther und Petroläther und erhält 4-[4-Chlor-2-(2-chlorbenzoyl)phenyl]-5-methyl-1 H-pyrrol-3-carbonitril als bräunliche Kristalle vom Schmelzpunkt 206—208°. Durch Umkristallisieren aus Äther erhält man farblose Kristalle vom Schmelzpunkt 210—211°.

Durch Kristallisieren des aus den Mutterlaugen erhaltenen Materials aus Äther erhält man 6-Chlor-8-chlorphenyl)-1,8-dihydro-8-hydroxy-2-methylideno[2,1-b]pyrrol-3-carbonitril vom Schmelzpunkt 221—225°. Durch Umkristallisieren aus Äther erhält man hellgelbe Prismen vom Schmelzpunkt 232—237°.

b) Man hydriert eine Mischung aus 8,5 g (24 mMol) 4-[4-Chlor-2-(2-chlorbenzoyl)phenyl]-5-methyl-1 H-pyrrol-3-carbonitril, 1 Löffel Raney-Nickel und 250 ml Eisessig über Nacht in einem Parr-Apparat bei einem Druck von 3,8 bar. Man filtriert den Katalysator ab, entfernt die Essigsäure unter vermindertem Druck, verdünnt den Rückstand mit Wasser und stellt mit konzentriertem Ammoniak basisch. Das ausgefallene Material wird abfiltriert und in Tetrahydrofuran aufgenommen. Man trocknet über wasserfreiem Natriumsulfat und dampft unter vermindertem Druck ein. Durch Kristallisieren des Rückstandes aus einer Mischung von Äther und Petroläther erhält man 8-Chlor-6-(2-chlorphenyl)-1-methyl-2 H,4 H-pyrrolo[3,4-d] [2]benzazepin als weißliche Kristalle vom Schmelzpunkt 219—222°. Durch Umkristallisieren aus einer Mischung von Äther und Methylenchlorid erhält man farblose Kristalle vom Schmelzpunkt 221—225°.

Beispiel 22

8-Chlor-6-(2-chlorphenyl)-2-methyl-2 H,4 H-pyrrolo[3,4-d] [2]benzazepin wird auf die gleiche Weise hergestellt wie 8-Chlor-6-(2-fluorphenyl)-2-methyl-2 H,4 H-pyrrolo[3,4-d] [2]benzazepin (Beispiel 17). Man erhält farblose Prismen vom Schmelzpunkt 167—168°.

Das Methansulfonatsalz von 8-Chlor-6-(2-chlorphenyl)-2-methyl-2 H,4 H-pyrrolo[3,4-d] [2]benzazepin wird hergestellt, indem man äquimolare Mengen der Base und Methansulfonsäure in Methanol löst, und das entstandene Salz durch Zugabe von Äther ausfällt. Durch Umkristallisieren aus einer Mischung von Methanol und Äther erhält man 8-Chlor-6-(2-chlorphenyl)-2-methyl-2 H,4 H-pyrrolo[3,4-d] [2]benzazepin-methansulfonat als orangefarbene Prismen vom Schmelzpunkt 200—203°.

Beispiel 23

In einer Portion gibt man 2,0 g (6,5 mMol) 8-Chlor-6-(2-fluorphenyl)-2 H,4 H-pyrrolo[3,4-d] [2]benzazepin zu 0,9 g (8 mMol) Kalium-t-butoxid in 35 ml Dimethylformamid, das man vorher auf 0° gekühlt hat. Man rührt während 15 Minuten, versetzt mit 3 ml (9 mMol) einer 3 M-Lösung von Diäthylaminoäthylchlorid in Toluol, läßt auf Raumtemperatur erwärmen, rührt während 2 Stunden, verdünnt die erhaltene Mischung mit Wasser und extrahiert mit Methylenchlorid. Die Methylenchloridlösung wird mit Wasser gewaschen, über wasserfreiem Natriumsulfat getrocknet und unter vermindertem Druck eingedampft. Den Rückstand reinigt man durch Säulenchromatographie an 25 g Kieselgel unter Eluieren mit Methylenchlorid/Äther (4 : 1) und erhält 8-Chlor-2-[2-[2-(diäthylamino)äthyl-6-(2-fluorphenyl)-2 H,4 H-pyrrolo[3,4-d] [2]benzazepin als weißlichen Festkörper vom Schmelzpunkt 110—112°. Man löst den weißen Rückstand in 10 ml einer 1,4 M-Lösung von Chlorwasserstoff in Methanol und verdünnt mit Äther. Man filtriert den erhaltenen Niederschlag ab und kristallisiert den orangefarbenen Festkörper aus einer Mischung von Methylenchlorid und Äther um. Man erhält 8-Chlor-2-[2-(diäthylamino)äthyl]-6-(2-fluorphenyl)-2 H,4 H-pyrrolo[3,4-d] [2]benzazepin-dihydrochlorid als orangefarbene Kristalle vom Schmelzpunkt 229—231°.

Beispiel 24

In einer Portion gibt man 2,0 g (6,4 mMol) 8-Chlor-6-(2-fluorphenyl)-2 H,4 H-pyrrolo[3,4-d] [2]benzazepin zu einer auf 0° gekühlten Lösung von 0,9 g (8 mMol) Kalium-t-butoxid in 30 ml Dimethylformamid, rührt während 15 Minuten, versetzt mit 0,7 ml (7,5 mMol) Methylbromacetat, rührt die erhaltene Mischung während 5 Minuten, verdünnt mit Wasser und extrahiert mit Äther. Die Ätherlösung wird mit Wasser gewaschen, über wasserfreiem Natriumsulfat getrocknet und unter vermindertem Druck zur Trockene eingedampft. Man reinigt den Rückstand durch Säulenchromatographie und erhält 8-Chlor-6-(2-fluorphenyl)-2 H,4 H-pyrrolo[3,4-d] [2]benzazepin-2-essigsäure-methylester als hellgelbes Öl.

Beispiel 25

In einer Portion gibt man 6,0 g (18,3 mMol) 8-Chlor-6-(2-chlorphenyl)-2 H,4 H-pyrrolo[3,4-d] [2]benzazepin zu einer auf 0° gekühlten Lösung von 3,0 g (26,5 mMol) Kalium-t-butoxid in 50 ml Dimethylformamid, versetzt, nachdem alles gelöst ist, mit 1,8 ml (2,3 g; 24,5 mMol) Methylchlorformat und rührt die erhaltene Mischung während 15 Minuten. Man verdünnt mit 150 ml Wasser, filtriert den erhaltenen Niederschlag ab und nimmt diesen in Methylenchlorid auf. Man wäscht die Methylenchloridlösung mit Wasser, trocknet über wasserfreiem Natriumsulfat und dampft unter vermindertem Druck ein. Man kristallisiert den Rückstand aus Äther und erhält einen weißlichen Festkörper. Durch Umkristallisieren aus einer Mischung von Methylenchlorid und Äther erhält man 8-Chlor-6-(2-chlorphenyl)-2 H,4 H-pyrrolo[3,4-d] [2]benzazepin-2-carbonsäuremethylester als weißliche feine Nadeln vom Schmelzpunkt 185—186° (Zersetzung).

Beispiel 26

In einer Portion gibt man 2,0 g (6,4 mMol) 8-Chlor-6-(2-fluorphenyl)-2 H,4 H-pyrrolo[3,4-d] [2]benzazepin zu einer auf 0° gekühlten Lösung von 0,9 g (8,0 mMol) Kalium-t-butoxid in 30 ml Dimethylformamid, rührt während 5 Minuten und versetzt mit 2,0 ml (32 mMol) Methyljodid. Man rührt noch während 1 Stunde, versetzt mit Wasser und extrahiert die erhaltene Mischung mit Äther. Die Ätherlösung wird mit Wasser gewaschen, über wasserfreiem Natriumsulfat getrocknet und unter vermindertem Druck zu einem wachsartigen Festkörper eingedampft. Man reinigt den Rückstand durch Säulenchromatographie an Kieselgel unter Eluieren mit 5% Äther in Methylenchlorid und erhält 8-Chlor-6-(2-fluorphenyl)-2,5-dimethyl-2 H,4 H-pyrrolo[3,4-d] [2]benzazepin-5-iumjodid als farblose Kristalle vom Schmelzpunkt 142—143°.

Die wäßrige Diamethylformamidlösung wird mit Methylenchlorid ausgeschüttelt. Die Methylenchloridphase wird mit Wasser gewaschen, über wasserfreiem Natriumsulfat getrocknet und unter vermindertem Druck zur Trockene eingedampft. Durch Verreiben des Rückstandes mit einer Mischung aus Methanol und Äther erhält man 8-Chlor-6-(2-fluorphenyl)-2,5-dimethyl-2 H,4 H-pyrrolo[3,4-d] [2]benzazepin-5-iumjodid als orangefarbenen Festkörper. Durch Umkristallisieren aus einer Mischung von Methanol und Äther erhält man orange Prismen vom Schmelzpunkt 228—231°.

Beispiel 27

In einer Portion gibt man 3,0 g (9,2 mMol) 8-Chlor-6-(2-chlorphenyl)-2 H,4 H-pyrrolo[3,4-d] [2]benzazepin zu einer auf 0° gekühlten Lösung von 1,35 g (12 mMol) Kalium-t-butoxid in 30 ml Dimethylformamid, rührt während 15 Minuten und versetzt mit 5 ml (80 mMol) Methyljodid. Man erwärmt die Mischung auf Raumtemperatur, rührt während 4 Stunden, verdünnt mit Wasser und extrahiert mit Methylenchlorid. Die Methylenchloridlösung wird mit Wasser gewaschen, über wasserfreiem Natriumsulfat getrocknet und unter vermindertem Druck zu einem roten Öl eingedampft. Durch Zugabe von wenig Mathanol induziert man die Kristallisation und erhält einen gelben Festkörper. Durch Umkristallisieren aus einer Mischung von Methanol und Äther erhält man 8-Chlor-6-(2-chlorphenyl)-2,5-dimethyl-2 H,4 H-pyrrolo[3,4-d] [2]benzazepin-5-iumjodid als feine orangefarbene Nadeln vom Schmelzpunkt 195—197°.

Beispiel 28

Portionsweise gibt man 0,2 g (52 mMol) Natriumborhydrid zu einer auf 0° gekühlten Lösung von 3,0 g (6,3 mMol) 8-Chlor-6-(2-chlorphenyl)-2,5-dimethyl-2 H,4 H-pyrrolo[3,4-d] [2]benzazepin-5-iumjodid in 50 ml Methanol, rührt die Mischung während 20 Minuten bei 0° und entfernt anschließend das Methanol unter vermindertem Druck. Den Rückstand verteilt man zwischen Methylenchlorid und Wasser, wäscht die Methylenchloridphase mit Wasser und gesättigter Kochsalzlösung, trocknet über wasserfreiem Natriumsulfat und dampft unter vermindertem Druck zu einem hellgelben Öl ein. Durch Kristallisieren aus Äther erhält man 8-Chlor-6-(2-chlorphenyl)-2,5-dimethyl-5,6-dihydro-2 H,4 H-pyrrolo[3,4-d] [2]benzazepin als farblose Kristalle vom Schmelzpunkt 154—155°.

Beispiel 29

Man gibt eine Lösung von 2,3 g (6,0 mMol) 8-Chlor-6-(2-fluorphenyl)-2 H,4 H-pyrrolo[3,4-d] [2]benzazepin-2-essigsäure-methylester in 25 ml Tetrahydrofuran tropfenweise zu einer auf —78° gekühlten Lösung von 0,5 g (13 mMol) Lithiumaluminiumhydrid in 30 ml Tetrahydrofuran, läßt die Mischung auf 0° erwärmen und rührt während 2 Stunden. Man zerstört das überschüssige Lithiumaluminiumhydrid durch Zugabe von 0,6 ml Wasser, 0,6 ml 10proz. Natronlauge und 2,0 ml Wasser. Nach Abfiltrieren vom ausgefallenen Material wird das Filtrat unter vermindertem Druck zur Trockene eingedampft. Den Rückstand kristallisiert man aus Äther und erhält einen weißen Festkörper, den man aus einer Mischung von Äther und Petroläther umkristallisiert. Man erhält 8-Chlor-6-(2-fluorphenyl)-2 H,4 H-pyrrolo[3,4-d] [2]benzazepin-2-äthanol als farblose Prismen vom Schmelzpunkt 145—147°.

Beispiel 30

Über einen Zeitraum von 4 Stunden gibt man in je 5 Portionen 0,5 g (7,9 mMol) Natriumcyanoborhydrid und 35 ml einer 1 M-Lösung von Methansulfonsäure in Methanol zu einer auf 0° gekühlten Lösung von 2,0 g (6,1 mMol) 8-Chlor-6-(2-chlorphenyl)-2 H,4 H-pyrrolo[3,4-d] [2]benzazepin in 50 ml Methanol. Nach beendeter Zugabe rührt man noch über Nacht bei Raumtemperatur, stellt anschließend mit 40proz. wäßriger Natronlauge basisch und verdünnt mit Wasser. Der erhaltene Niederschlag wird abfiltriert. Durch Umkristallisieren des weißen Festkörpers aus einer Mischung von Äther und Methylenchlorid erhält man 8-Chlor-6-(2-chlorphenyl)-5,6-dihydro-2 H,4 H-pyrrolo[3,4-d] [2]benzazepin als farblose Kristalle vom Schmelzpunkt 211—212°.

Beispiel 31

Man erhitzt eine Mischung aus 0,7 g (2,2 mMol) 8-Chlor-6-(2-fluorphenyl)-2 H,4 H-pyrrolo[3,4-d] [2]benzazepin, 2,0 ml (18 mMol) Trichloracetylchlorid und 20 ml Methylenchlorid während 7 Tagen unter Rückfluß zum Sieden. Der erhaltene Niederschlag wird abfiltriert und zwischen Methylenchlorid und gesättigter wäßriger Natriumbicarbonatlösung verteilt. Die Methylenchloridlösung trocknet man über wasserfreiem Natriumsulfat. Nach Eindampfen unter vermindertem Druck erhält man 1-[8-Chlor-6-(2-fluorphenyl)-2 H,4 H-pyrrolo[3,4-d] [2]benzazepin-3-yl]-2,2,2-trichloräthanon als weißen Festkörper vom Schmelzpunkt 240—245° (Zersetzung).

Beispiel 32

Man gibt eine Lösung von 3,0 ml (33 mMol) Phosphoroxychlorid in 20 ml Methylenchlorid tropfenweise zu einer auf 0° gekühlten Lösung von 3,0 g (9,1 mMol) 8-Chlor-6-(2-chlorphenyl)-2 H,4 H-pyrrolo[3,4-d] [2]benzazepin in 40 ml Dimethylformamid, rührt noch während 2 Stunden bei 0° und gießt anschließend auf eine gesättigte wäßrige Natriumcarbonatlösung. Man extrahiert mit Methylenchlorid, wäscht die Methylenchloridlösung mit Wasser, trocknet sie über wasserfreiem Natriumsulfat und dampft unter vermindertem Druck zur Trockene ein. Den Rückstand reinigt man durch Säulenchromatographie an 100 g Kieselgel unter Eluieren mit 50% Essigester in Methylenchlorid (v/v) und erhält 8-Chlor-6-(2-chlorphenyl)-2 H,4 H-pyrrolo[3,4-d] [2]benzazepin-3-carboxaldehyd als leicht rosafarbenen Festkörper vom Schmelzpunkt 270—274°. Umkristallisieren aus einer Mischung von Essigester und Methylenchlorid liefert pfirsichfarbene Prismen vom Schmelzpunkt 276—277°.

Beispiel 33

Man gibt eine Lösung von 1,5 g (9,5 mMol) Kaliumpermanganat in 150 ml 50proz. wäßrigem Aceton tropfenweise zu einer Lösung von 1,6 g (4,5 mMol) 8-Chlor-6-(2-chlorphenyl)-2 H,4 H-pyrrolo[3,4-d] [2]benzazepin-3-carboxaldehyd in 100 ml Aceton, verdünnt die erhaltene Mischung nach 2,5 Stunden mit einer gesättigten wäßrigen Natriumbisulfitlösung, neutralisiert durch Zugabe von Essigsäure und extrahiert mit Methylenchlorid. Die Methylenchloridlösung wird über wasserfreiem Natriumsulfat getrocknet und unter vermindertem Druck zur Trockene eingedampft. Durch Verreiben des Rückstandes mit Methylenchlorid erhält man 8-Chlor-6-(2-chlorphenyl)-2 H,4 H-pyrrolo[3,4-d] [2]benzazepin-3-carbonsäure als gelbe Kristalle vom Schmelzpunkt 216—218°. Durch Umkristallisieren aus

einer Mischung von Äthanol und Äther erhält man das entsprechende Hemiätherat als gelbe Prismen vom Schmelzpunkt 252—254° (Zersetzung).

Beispiel 34

Man gibt eine Lösung von 0,8 g (2,2 mMol) rohem 8-Chlor-6-(2-chlorphenyl)-2 H,4 H-pyrrolo[3,4-d] [2]benzazepin-3-carbonsäure in 20 ml einer Mischung von 50% Tetrahydrofuran in Methylenchlorid zu 20 ml einer 1 M-Lösung von Diazomethan in Äther. Man zerstört das überschüssige Diazomethan durch Zugabe von Essigsäure, wäscht die organische Phase mit gesättigter wäßriger Natriumbicarbonatlösung, trocknet über wasserfreiem Natriumsulfat und dampft unter vermindertem Druck zur Trockene ein. Den Rückstand reinigt man durch Säulenchromatographie an 20 g Kieselgel unter Eluieren mit 20% Äther in Methylenchlorid und erhält ein hellgelbes Öl. Dieses Öl löst man in Methanol, das 1 ml einer 1 M-Lösung von Methansulfonsäure in Methanol enthält, und fällt das entstandene Salz durch Zugabe von Äther aus. Durch Umkristallisieren aus einer Mischung von Methanol und Äther erhält man 8-Chlor-6-(2-chlorphenyl)-2 H,4 H-pyrrolo[3,4-d] [2]benzazepin-3-carbonsäure-methylester-methansulfonat als gelbe Kristalle vom Schmelzpunkt 273—274° (Zersetzung).

Beispiel 35

Man gibt 0,6 ml (6,5 mMol) Phosphoroxychlorid mittels einer Spritze zu einer auf 0° gekühlten Lösung von 0,7 g (2,0 mMol) 8-Chlor-6-(2-chlorphenyl)-1-methyl-2 H,4 H-pyrrolo[3,4-d] [2]benzazepin in 8 ml Dimethylformamid, rührt die erhaltene Mischung während 1 Stunde bei 0° und gießt auf 50 ml gesättigte wäßrige Natriumcarbonatlösung. Man extrahiert mit Methylenchlorid, wäscht die Methylenchloridlösung mit Wasser, trocknet über wasserfreiem Natriumsulfat und dampft unter vermindertem Druck ein. Den erhaltenen roten amorphen Festkörper reinigt man durch Säulenchromatographie an 20 g Kieselgel unter Eluieren mit 40% Äther in Methylenchlorid und erhält einen weißlichen Festkörper, den man aus einer Mischung von Methylenchlorid und Essigester umkristallisiert. Man erhält 8-Chlor-6-(2-chlorphenyl)-1-methyl-2 H,4 H-pyrrolo[3,4-d] [2]benzazepin-3-carboxaldehyd als weißliche Kristalle vom Schmelzpunkt 274—276°.

Beispiel 36

In einer Portion gibt man 0,2 g (5,2 mMol) Natriumborhydrid zu einer auf 0° gekühlten Lösung von 0,9 g 8-Chlor-6-(2-chlorphenyl)-1-methyl-2 H,4 H-pyrrolo[3,4-d] [2]benzazepin-3-carboxaldehyd in 20 ml Methanol, rührt während 30 Minuten bei 0°, versetzt mit Wasser und filtriert den erhaltenen Niederschlag ab. Man erhält 8-Chlor-6-(2-chlorphenyl)-3-(hydroxymethyl)-1-methyl-2 H,4 H-pyrrolo[3,4-d] [2]benzazepin als bräunlichen amorphen Festkörper vom Schmelzpunkt 170—180° (Zersetzung). Dieser Stoff konnte nicht ohne Zersetzung gereinigt werden.

Beispiel 37

Man rührt eine Mischung aus 4,2 g (11 mMol) 8-Chlor-6-(2-chlorphenyl)-2 H,4 H-pyrrolo[3,4-d] [2]benzazepin-2-carbonsäure-methylester, 3,15 g (14,5 mMol) m-Chlorperbenzoesäure und 100 ml Methylenchlorid während 2,5 Stunden bei 0°, wäscht die erhaltene Mischung mit gesättigter wäßriger Natriumbicarbonatlösung und gesättigter Kochsalzlösung, trocknet über wasserfreiem Natriumsulfat und dampft unter vermindertem Druck zur Trockene ein. Man kristallisiert den Rückstand aus Äther und erhält 8-Chlor-6-(2-chlorphenyl)-2 H,4 H-pyrrolo[3,4-d] [2]benzazepin-2-carbonsäure-methylester-5-oxid als weißen Festkörper vom Schmelzpunkt 224—226°. Umkristallisieren aus einer Mischung von Äther und Methylenchlorid liefert farblose Prismen vom Schmelzpunkt 226—227°.

Beispiel 38

Methode A: Man gibt eine Lösung von 4,0 g (20 mMol) 85proz. m-Chlorperbenzoesäure in 50 ml Methylenchlorid tropfenweise zu einer auf 0° gekühlten Lösung von 5,0 g (15 mMol) 8-Chlor-6-(2-chlorphenyl)-2 H,4 H-pyrrolo[3,4-d] [2]benzazepin in 250 ml Methylenchlorid, rührt während 2 Stunden bei 0°, wäscht die erhaltene Mischung mit gesättigter wäßriger Natronbicarbonatlösung, Wasser und gesättigter wäßriger Natriumsulfatlösung und dampft unter vermindertem Druck ein. Das erhaltene Öl kristallisiert man aus Essigester und erhält einen crèmefarbenen Festkörper. Durch Umkristallisieren aus einer Mischung von Methylenchlorid und Äther erhält man 8-Chlor-6-(2-chlorphenyl)-2 H,4 H-pyrrolo[3,4-d] [2]benzazepin-5-oxid als farblose Nadeln vom Schmelzpunkt 233—235°.

Methode B: Man rührt eine Mischung aus 1,0 g (2,4 mMol) 8-Chlor-6-(2-chlorphenyl)-2 H,4 H-pyrrolo[3,4-d] [2]benzazepin-2-carbonsäure-methylester-5-oxid, 2,5 ml einer 3 N-Natronlauge, 10 ml Methanol und 50 ml Tetrahydrofuran während 20 Minuten bei Raumtemperatur, dampft die erhaltene Mischung unter vermindertem Druck ein und verteilt den Rückstand zwischen Wasser und Methylenchlorid. Die Methylenchloridlösung wird mit Wasser gewaschen, über wasserfreiem Natriumsulfat getrocknet und unter vermindertem Druck eingedampft. Man behandelt den Rückstand mit einer Mischung aus Tetrahydrofuran und Äther und erhält einen weißlichen Festkörper vom Schmelzpunkt 239—240°. Durch Umkristallisieren aus einer Mischung von Äther und Methylenchlorid erhält man 8-Chlor-6-(2-chlorphenyl)-2 H,4 H-pyrrolo[3,4-d] [2]benzazepin-5-oxid als feine farblose Nadeln vom Schmelzpunkt 241—242°. Die spektroskopische Analyse zeigt, daß dieser Stoff mit dem obigen identisch ist, obwohl die Schmelzpunkte differieren.

Beispiel 39

Man rührt eine Mischung aus 7,6 g (19 mMol) 8-Chlor-6-(2-chlorphenyl)-2 H,4 H-pyrrolo[3,4-d] [2]benzazepin-2-carbonsäure-methylester-5-oxid und 200 ml Essigsäureanhydrid während 12 Stunden bei 70° und während 5 Stunden bei 105°. Die Acetanhydridlösung wird unter vermindertem Druck eingedampft. Den Rückstand nimmt man in Methylenchlorid auf, wäscht mit gesättigter wäßriger Natriumbicarbonatlösung und gesättigter Kochsalzlösung, trocknet über wasserfreiem Natriumsulfat und dampft unter vermindertem Druck ein. Das erhaltene Öl reinigt man durch Säulenchromatographie an 50 g neutralem Aluminiumoxid unter Eluieren mit 5% Äther in Methylenchlorid und erhält 4-(Acetyloxy)-8-chlor-6-(2-chlorphenyl)-2 H,4 H-pyrrolo[3,4-d] [2]benzazepin-2-carbonsäure-methylester als bräunlichen Festkörper vom Schmelzpunkt 175—177°. Durch Umkristallisieren aus Äther erhält man einen weißlichen Festkörper vom Schmelzpunkt 177—178°.

Beispiel 40

Man rührt eine Mischung aus 1,0 g (2,2 mMol) 4-(Acetyl-oxy)-8-chlor-6-(2-chlorphenyl)-2 H,4 H-pyrrolo[3,4-d] [2]benzazepin-2-carbonsäure-methylester und 2,5 ml 3 N-Natronlauge in einer Mischung aus 25 ml Tetrahydrofuran und 10 ml Methanol während 30 Minuten bei 0°, verdünnt die erhaltene Mischung mit Wasser und extrahiert mit Methylenchlorid. Die Methylenchloridlösung wird mit gesättigter Kochsalzlösung gewaschen, über wasserfreiem Natriumsulfat getrocknet und unter vermindertem Druck zur Trockene eingedampft. Den Rückstand zerreibt man mit einer Mischung aus Äther und Methylenchlorid und erhält einen crèmefarbenen Festkörper vom Schmelzpunkt 215—218°. Durch Umkristallisieren aus einer Mischung von Tetrahydrofuran und Hexan erhält man 8-Chlor-6-(2-chlorphenyl)-4-methoxy-2 H,4 H-pyrrolo[3,4-d] [2]benzazepin als bräunliche Prismen vom Schmelzpunkt 221—224°.

Beispiel 41

In einer Portion gibt man 1,0 g (2,8 mMol) 8-Chlor-6-(2-chlorphenyl)-4-methoxy-2 H,4 H-pyrrolo[3,4-d] [2]benzazepin zu einer auf 0° gekühlten Lösung von 0,35 g (3,0 mMol) Kalium-t-butoxid in 20 ml Dimethylformamid. Man rührt während 15 Minuten, versetzt mit 0,3 ml (4,8 mMol) Methyljodid, rührt die Mischung während 5 Minuten, verdünnt mit Wasser und extrahiert mit Äther. Die Ätherlösung wird mit Wasser gewaschen, über wasserfreiem Natrium Sulfat getrocknet und unter vermindertem Druck eingedampft. Das erhaltene hellgelbe Öl reinigt man durch Säulenchromatographie an 25 g Kieselgel unter Eluieren mit 5% Äther in Methylenchlorid und erhält einen weißlichen Festkörper vom Schmelzpunkt 128—130°. Durch Umkristallisieren aus einer Mischung von Äther und Petroläther erhält man 8-Chlor-2-methyl-4-methoxy-6-(2-chlorphenyl)-2 H,4 H-pyrrolo[3,4-d] [2]benzazepin als weißliche Prismen vom Schmelzpunkt 128—130°.

Beispiel 42

In einer Portion gibt man 1,5 g (4,8 mMol) 8-Chlor-6-(2-fluorphenyl)-2 H,4 H-pyrrolo[3,4-d] [2]benzazepin zu einer auf 0° gekühlten Lösung von 0,6 g (5,3 mMol) Kalium-t-butoxid in 25 ml Dimethylformamid, rührt während 15 Minuten, versetzt mit 5,2 ml (10,4 mMol) einer 2 M-Lösung von Dimethylaminopropylchlorid inToluol, läßt die Mischung auf Raumtemperatur erwärmen und rührt noch während 4 Stunden. Man verdünnt mit Wasser, extrahiert mit Methylenchlorid, wäscht die Methylenchloridlösung mit Wasser, trocknet über wasserfreiem Natriumsulfat und dampft unter vermindertem Druck ein. Man reinigt den Rückstand durch Säulenchromatographie an 25 g Kieselgel unter Eluieren mit Methylenchlorid/Äther (4 : 1) und Methanol/Äther/Methylenchlorid (1 : 2 : 7) und erhält ein farbloses Öl. Dieses Öl nimmt man in einem Überschuß an methanolischem Chlorwasserstoff auf und dampft die erhaltene Lösung unter vermindertem Druck ein. Durch Kristallisieren des Rückstandes aus einer Mischung von Isopropanol und Äther erhält man 8-Chlor-2-[3-(dimethylamino)propyl]-6-(2-fluorphenyl)-2 H,4 H-pyrrolo[3,4-d] [2]benzazepin-dihydrochlorid als orangefarbener Festkörper vom Schmelzpunkt 258—260°.

Beispiel 43

8-Chlor-2-[2-(dimethylamino)äthyl]-6-(2-fluorphenyl)-2 H,4 H-pyrrolo[3,4-d] [2]benzazepin-dihydrochlorid wird auf die gleiche Weise hergestellt wie 8-Chlor-2-[2-(dimethylamino)propyl]-6-(2-fluorphenyl)-2 H,4 H-pyrrolo[3,4-d] [2]benzazepin-dihydrochlorid. Man erhält gelbe Kristalle vom Schmelzpunkt 264—266°.

Beispiel 44

In einer Portion gibt man 2,1 g (6,4 mMol) 8-Chlor-6-(2-chlorphenyl)-2 H,4 H-pyrrolo[3,4-d] [2]benzazepin zu einer auf 0° gekühlten Lösung von 0,8 g (7,0 mMol) Kalium-t-butoxid in 35 ml Dimethylformamid, rührt während 15 Minuten, versetzt mit 2,5 ml (7,5 mMol) einer 3 M-Lösung von Diäthylaminoäthylchlorid in Toluol, läßt die erhaltene Mischung auf Raumtemperatur erwärmen und rührt noch während 3 Stunden. Man verdünnt die Mischung mit Wasser und extrahiert mit Methylenchlorid. Die Methylenchloridlösung wird mit Wasser gewaschen, über wasserfreiem Natriumsulfat getrocknet und unter vermindertem Druck eingedampft. Man reinigt den Rückstand durch Säulenchromatographie an 25 g Kieselgel unter Eluieren mit Methylenchlorid/Äther (4 : 1) und erhält 8-Chlor-2-[2-(diäthylamino)äthyl]-6-(2-chlorphenyl)-2 H,4 H-pyrrolo[3,4-d] [2]benzazepin als weißlichen Festkörper vom Schmelzpunkt 130—131°.

Beispiel 45

Über einen Zeitraum von 30 Minuten gibt man 0,3 g (5,3 mMol) Natriumcyanoborhydrid portionenweise zu einer Lösung von 0,3 g (0,75 mMol) 8-Chlor-2-[2-(diäthylamino)äthyl]-6-(2-fluorphenyl)-2 H,4 H-pyrrolo[3,4-d] [2]benzazepin-dihydrochlorid. Während der Reaktion werden insgesamt 6,0 ml (8,4 mMol) einer 1,4 M-Lösung von Chlorwasserstoff in Methanol zugefügt, um einen sauren pH aufrechtzuerhalten. Man entfernt das Methanol unter vermindertem Druck und verteilt den Rückstand zwischen Ammoniak und Äther. Die Ätherlösung trocknet man über wasserfreiem Natriumsulfat und dampft unter vermindertem Druck zur Trockene ein. Den Rückstand löst man in einem Überschuß an methanolischem Chlorwasserstoff und 50 ml Isopropanol. Durch Eindampfen der Lösung unter vermindertem Druck erhält man 8-Chlor-2-[2-(diäthylamino)äthyl]-5,6-dihydro-6-(2-fluorphenyl)-2 H,4 H-pyrrolo[3,4-d] [2]benzazepin-dihydrochlorid als weißen Festkörper. Durch Umkristallisieren aus einer Mischung von Äther und Methanol erhält man farblose Nadeln vom Schmelzpunkt 265—270° (Zersetzung).

Beispiel A

Herstellung von Tabletten (Naßgranulierung)

| Bestandteile | mg/Tabl. | mg/Tabl. | mg/Tabl. | mg/Tabl. |
|---|---|---|---|---|
| 1. 8-Chlor-6-(2-chlorphenyl)-2 H,4 H-pyrrolo[3,4-d] [2]benzazepin oder 8-Chlor-1-methyl-6-(2-fluorphenyl)-2 H,4 H-pyrrolo[3,4-d] [2]benzazepin | 1 | 5 | 10 | 25 |
| 2. Lactose | 202 | 232 | 261 | 280 |
| 3. modifizierte Stärke | 25 | 35 | 45 | 55 |
| 4. vorgelatinisierte Stärke | 20 | 25 | 30 | 35 |
| 5. destill. Wasser q. s. | — | — | — | — |
| 6. Magnesiumstearat | 2 | 3 | 4 | 5 |
| Tablettengewicht | 250 | 300 | 350 | 400 |

Verfahren

Man vermischt die Bestandteile 1—4 in einem geeigneten Mixer, granuliert mit genügend destilliertem Wasser, um eine geeignete Konsistenz zu erhalten, vermahlt und trocknet in einem geeigneten Ofen. Anschließend vermahlt und mischt man während 3 Minuten mit dem Magnesiumstearat und verpreßt auf einer Presse zu Tabletten geeigneter Größe.

Beispiel B

Herstellung von Tabletten (direkte Verpressung)

| Bestandteile | | mg/Tabl. | mg/Tabl. | mg/Tabl. | mg/Tabl. |
|---|---|---|---|---|---|
| 1. | 8-Chlor-6-(2-chlorphenyl)-2 H,4 H-pyrrolo[3,4-d] [2]benzazepin oder 8-Chlor-1-methyl-6-(2-fluorphenyl)-2 H,4 H-pyrrolo[3,4-d] [2]benzazepin | 1 | 5 | 10 | 25 |
| 2. | Lactose | 221 | 217 | 212 | 181 |
| 3. | Avicel | 45 | 45 | 45 | 55 |
| 4. | Stärke für die direkte Verpressung | 30 | 30 | 30 | 35 |
| 5. | Magnesiumstearat | 3 | 3 | 3 | 4 |
| | Tablettengewicht | 300 | 300 | 300 | 300 |

Verfahren

Man vermischt den Wirkstoff mit der gleichen Menge Lactose, vermischt das ganze mit den Bestandteilen 3 und 4 und den Rest der Lactose, versetzt anschließend mit dem Magnesiumstearat und mischt während 3 Minuten. Die Masse wird auf einer geeigneten Presse zu Tabletten entsprechender Größe verpreßt.

Beispiel C

Herstellung von Kapseln

| Bestandteile | | mg/Kapsel | mg/Kapsel | mg/Kapsel | mg/Kapsel |
|---|---|---|---|---|---|
| 1. | 8-Chlor-6-(2-chlorphenyl)2 H,4 H-pyrrolo[3,4-d] [2]-benzazepin oder 8-Chlor-1-methyl-6-(2-fluorphenyl)-2 H,4 H-pyrrolo-[3,4-d] [2]benzazepin | 1 | 5 | 10 | 25 |
| 2. | Lactose | 203 | 293,5 | 328 | 372,5 |
| 3. | Stärke | 30 | 35 | 40 | 30 |
| 4. | Talk | 15 | 15 | 20 | 20 |
| 5. | Aerosol OT | 1 | 1,5 | 2 | 2,5 |
| | Kapselabfüllgewicht | 250 | 350 | 400 | 450 |

Verfahren

Man vermischt die Bestandteile 1, 2, 3 und 5 in einem geeigneten Mixer, mahlt, versetzt mit dem Talk und mischt gut. Man füllt auf einer geeigneten Maschine in Kapseln entsprechender Größe ab.

**Patentansprüche für die Vertragsstaaten: BE, CH, DE, FR, GB, IT, LI, LU, NL, SE**

1. Pyrrolo[3,4-d] [2]benzazepine der allgemeinen Formel

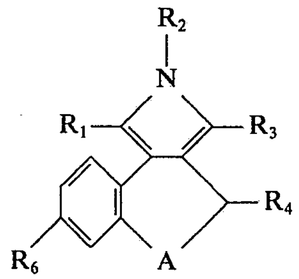

(I)

worin A eine der Gruppen

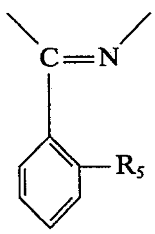 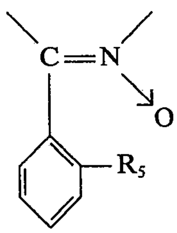 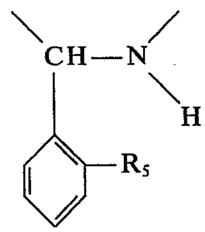 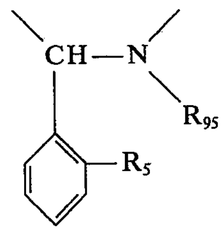 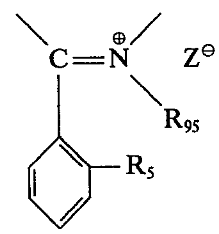

(a) (b) (c) (d) und (e)

$R_1$ Wasserstoff, niederes Alkyl, $C_3$- bis $C_7$-Alkenyl, $C_3$- bis $C_7$-Alkinyl, Hydroxymethyl oder die Gruppe $-COR_{11}$, $R_{11}$ Wasserstoff, Hydroxy, niederes Alkoxy oder Amino oder mono- oder di-niederes Alkylamino, $R_3$ Wasserstoff, niederes Alkyl, $C_3$- bis $C_7$-Alkenyl, $C_3$- bis $C_7$-Alkinyl, Hydroxymethyl oder die Gruppe $-COR_{111}$, $R_{111}$ Wasserstoff, Hydroxy, Trihalomethyl, niederes Alkoxy oder Amino oder mono- oder di-niederes Alkylamino, $R_2$ Wasserstoff, niederes Alkyl, $C_3$- bis $C_7$-Alkenyl, $C_3$- bis $C_7$-Alkinyl, die Gruppe $-COO-$niederes Alkyl, Hydroxy$-C_2$- bis $C_7$-Alkyl, Amino- oder mono- oder di-niederes Alkylamino$-C_2$- bis $C_7$-Alkyl oder die Gruppe $-C_1$- bis $C_6$-Alkyl$-COR_{21}$, $R_{21}$ Hydroxy, niederes Alkoxy, Amino oder mono- oder di-niederes Alkylamino, $R_4$ Wasserstoff, geradkettiges niederes Alkoxy, Hydroxy oder die Gruppe $-OCOR$, R Wasserstoff, $C_1$- bis $C_6$-Alkyl, niederes Haloalkyl oder Phenyl, $R_5$ Halogen mit einer Ordnungszahl, welche nicht größer als 35 ist, oder Wasserstoff, $R_6$ Halogen mit einer Ordnungszahl, welche nicht größer als 35 ist, $R_{95}$ niederes Alkyl und $Z^{\ominus}$ das Anion einer Säure bedeuten, mit der Maßgabe, daß die als »nieder« bezeichneten Reste höchstens 7 C-Atome aufweisen und den folgenden Maßgaben:

(A) wenn einer der Reste $R_1$ und $R_3$ Hydroxymethyl oder die Gruppe $-COR_{11}$ oder $-COR_{111}$ bedeutet, wobei $R_{11}$ und $R_{111}$ obige Bedeutung besitzen, dann bedeuten der verbleibende Rest Wasserstoff, niederes Alkyl, $C_3$- bis $C_7$-Alkenyl oder $C_3$- bis $C_7$-Alkinyl und $R_2$ Wasserstoff;
(B) wenn $R_4$ $-OCOR$ bedeutet, dann bedeuten $R_1$ und $R_3$ Wasserstoff, niederes Alkyl, $C_3$- bis $C_7$-Alkenyl oder $C_3$- bis $C_7$-Alkinyl und $R_2$ die Gruppe $-CO-$niederes Alkoxy;
(C) wenn $R_4$ Hydroxy bedeutet, dann bedeuten $R_1$ und $R_3$ Wasserstoff, niederes Alkyl, $C_3$- bis $C_7$-Alkenyl oder $C_3$- bis $C_7$-Alkinyl und $R_2$ Wasserstoff;
(D) wenn $R_4$ geradkettiges niederes Alkoxy bedeutet, dann bedeuten $R_1$ und $R_3$ Wasserstoff, niederes Alkyl, $C_3$- bis $C_7$-Alkenyl oder $C_3$- bis $C_7$-Alkinyl;
(E) wenn A die obige Gruppe (b), (c), (d) oder (e) bedeutet, dann bedeutet $R_4$ Wasserstoff; und
(F) wenn A die obige Gruppe (b) oder (e) bedeutet, dann besitzt $R_2$ eine von Amino- oder mono oder di-niederes Alkylamino$-C_2$- bis $C_7$-Alkyl verschiedene Bedeutung;

und pharmazeutisch annehmbare Säureadditionssalze von Verbindungen der Formel I, worin A die Gruppe (a) bedeutet.

2. Verbindungen gemäß Anspruch 1, dadurch gekennzeichnet, daß $R_2$ eine andere Bedeutung als Carboxy—$C_1$- bis $C_6$-Alkyl besitzt, mit den folgenden Maßgaben:

(i) wenn A die Gruppe (a) und $R_4$ geradkettiges niederes Alkoxy bedeuten, dann bedeutet $R_2$ Wasserstoff oder niederes Alkyl; und
(ii) wenn A die Gruppe (b) bedeutet, dann bedeutet $R_2$ Wasserstoff oder die Gruppe —COO—niederes Alkyl.

3. Verbindungen gemäß Anspruch 1 oder 2, dadurch gekennzeichnet, daß A die Gruppe (a) bedeutet.
4. Verbindungen gemäß Anspruch 3, dadurch gekennzeichnet, daß $R_3$ Wasserstoff, $R_5$ und $R_6$ je Halogen, $R_1$ Wasserstoff oder niederes Alkyl und $R_4$ Wasserstoff bedeuten.
5. Verbindungen gemäß Anspruch 4, dadurch gekennzeichnet, daß $R_5$ Chlor oder Fluor und $R_6$ Chlor bedeuten.
6. 8-Chlor-6-(2-chlorphenyl)-2 H,4 H-pyrrolo[3,4-d] [2]benzazepin.
7. 8-Chlor-6-(2-fluorphenyl)-2 H,4 H-pyrrolo[3,4-d] [2]benzazepin.
8. 8-Chlor-6-(2-chlorphenyl)-1-methyl-2 H,4 H-pyrrolo[3,4-d] [2]benzazepin.
9. 8-Chlor-6-(2-chlorphenyl)-2-methyl-2 H,4 H-pyrrolo[3,4-d] [2]benzazepin.
10. 8-Chlor-6-(2-fluorphenyl-2 H,4 H-pyrrolo[3,4-d] [2]benzazepin-2-äthanol.
11. 8-Chlor-6-(2-chlorphenyl)-2 H,4 H-pyrrolo[3,4-d] [2]benzazepin-2-carbonsäuremethylester.
12. Verbindungen der allgemeinen Formel

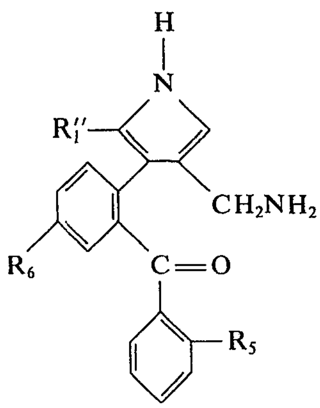

(B)

worin $R_5$ und $R_6$ die in Anspruch 1 angegebene Bedeutung besitzen, und $R_1''$ Wasserstoff, niederes Alkyl, $C_3$- bis $C_7$-Alkenyl oder $C_3$- bis $C_7$-Alkinyl bedeutet.
13. Verbindungen der allgemeinen Formel

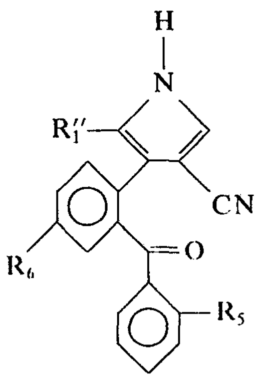

(XI)

worin $R_5$ und $R_6$ die in Anspruch 1 angegebene Bedeutung besitzen, und $R_1''$ Wasserstoff, niederes Alkyl, $C_3$- bis $C_7$-Alkenyl oder $C_3$- bis $C_7$-Alkinyl bedeutet.

14. Verbindungen der allgemeinen Formel

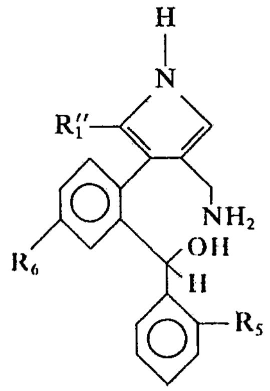

(XII)

worin $R_5$ und $R_6$ die in Anspruch 1 angegebene Bedeutung besitzen, und $R_1''$ Wasserstoff, niederes Alkyl, $C_3$- bis $C_7$-Alkenyl oder $C_3$- bis $C_7$-Alkinyl bedeutet.

15. Verbindungen gemäß einem der Ansprüche 1—11 als pharmazeutische Wirkstoffe.

16. Verbindungen gemäß einem der Ansprüche 1—11 als sedativ und anxiolytisch wirksame Stoffe.

17. Verfahren zur Herstellung von Verbindungen gemäß einem der Ansprüche 1—11, dadurch gekennzeichnet, daß man

a) eine Verbindung der allgemeinen Formel

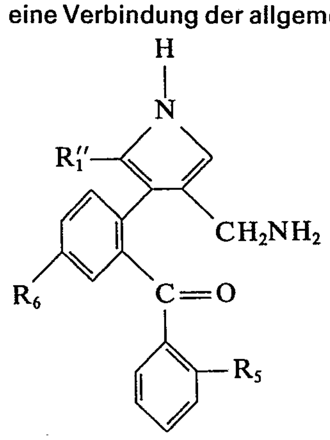

(B)

worin $R_5$ und $R_6$ obige Bedeutung besitzen, und $R_1''$ Wasserstoff, niederes Alkyl, $C_3$- bis $C_7$-Alkenyl oder $C_3$- bis $C_7$-Alkinyl bedeutet, cyclisiert, oder

b) eine Verbindung der in Anspruch 1 definierten Formel I, worin A die in Anspruch 1 angegebene Gruppe (a), $R_2$ und $R_4$ je Wasserstoff, und einer der Reste $R_1$ und $R_3$ Wasserstoff und der andere Wasserstoff, niederes Alkyl, $C_3$- bis $C_7$-Alkenyl oder $C_3$- bis $C_7$-Alkinyl bedeuten, in der 1- und/oder 3-Stellung niederalkyliert, $C_3$- bis $C_7$-alkenyliert oder $C_3$- bis $C_7$-alkinyliert, oder

c) eine Verbindung der in Anspruch 1 definierten Formel I, worin A die in Anspruch 1 angegebene Gruppe (a), $R_1$ und $R_3$ je Wasserstoff, niederes Alkyl, $C_3$- bis $C_7$-Alkenyl oder $C_3$- bis $C_7$-Alkinyl und $R_2$ Wasserstoff bedeuten, in der 2-Stellung mit einer niederen Alkyl-, $C_3$- bis $C_7$-Alkenyl-, $C_3$- bis $C_7$-Alkinyl- oder mit einer Hydroxy—$C_2$- bis $C_7$-Alkylgruppe, oder mit der Gruppe —COO—niederes Alkyl, $-(CH_2)_n-COO-$ niederes Alkyl, $-(CH_2)_n-CO-NR_{91}R_{92}$ oder $-(CH_2)_{n+1}-NR_{91}R_{92}$, worin n die Zahl 1 bis 6 und $R_{91}$ und $R_{92}$ je Wasserstoff oder niederes Alkyl bedeuten, substituiert, oder

d) eine Verbindung der in Anspruch 1 definierten Formel I, worin A die in Anspruch 1 angegebene Gruppe (a), $R_1$ und $R_3$ je Wasserstoff, niederes Alkyl, $C_3$- bis $C_7$-Alkenyl oder $C_3$- bis $C_7$-Alkinyl und $R_2$ die Gruppe $-(CH_2)_nCOO-$ niederes Alkyl oder $-(CH_2)_n-CO-NR_{91}R_{92}$ bedeuten und n, $R_{91}$ und $R_{92}$ obige Bedeutung besitzen, reduziert, oder

e) eine Verbindung der in Anspruch 1 definierten Formel I, worin A die in Anspruch 1 angegebene Gruppe (a), $R_1$ und $R_3$ je Wasserstoff, niederes Alkyl, $C_3$- bis $C_7$-Alkenyl oder $C_3$- bis $C_7$-Alkinyl und $R_2$ die Gruppe $-(CH_2)_nCOO-$niederes Alkyl bedeuten und n obige Bedeutung besitzt, in die entsprechende Carbonsäure oder in ein entsprechendes Amid, niederes Alkylamid oder di-niederes Alkylamid umwandelt, oder

f) eine Verbindung der in Anspruch 1 definierten Formel I, worin A die in Anspruch 1 angegebene Gruppe (a), $R_2$ und $R_4$ je Wasserstoff und einer der Reste $R_1$ und $R_3$ Wasserstoff und der andere Wasserstoff, niederes Alkyl, $C_3$- bis $C_7$-Alkenyl oder $C_3$- bis $C_7$-Alkinyl bedeuten, in der 1- oder 3-Stellung formyliert oder hydroxymethyliert, oder

g) eine Verbindung der in Anspruch 1 definierten Formel I, worin A die in Anspruch 1 angegebene Gruppe (a), $R_2$ und $R_4$ je Wasserstoff und einer der Reste $R_1$ und $R_3$ Formyl und der andere Wasserstoff, niederes Alkyl, $C_3$- bis $C_7$-Alkenyl oder $C_3$- bis $C_7$-Alkinyl bedeuten, zur entsprechenden Hydroxymethylverbindung reduziert, oder

h) eine Verbindung der in Anspruch 1 definierten Formel I, worin A die in Anspruch 1 angegebene Gruppe (a), $R_2$, $R_3$ und $R_4$ je Wasserstoff und $R_1$ Wasserstoff, niederes Alkyl, $C_3$- bis $C_7$-Alkenyl oder $C_3$- bis $C_7$-Alkinyl bedeuten, mit einem Trihaloacetylchlorid behandelt, oder

i) eine Verbindung der in Anspruch 1 definierten Formel I, worin A die in Anspruch 1 angegebene Gruppe (a), $R_2$ und $R_4$ je Wasserstoff, $R_1$ Wasserstoff, niederes Alkyl, $C_3$- bis $C_7$-Alkenyl oder $C_3$- bis $C_7$-Alkinyl und $R_3$ Trihaloacetyl bedeuten, mit einem Alkalimetall-niederen Alkoxid oder mit einer Verbindung der Formel $HNR_{35}R_{36}$, worin $R_{35}$ und $R_{36}$ je Wasserstoff oder niederes Alkyl bedeuten, behandelt, oder

j) eine Verbindung der in Anspruch 1 definierten Formel I, worin A die in Anspruch 1 angegebene Gruppe (a), $R_2$ und $R_4$ je Wasserstoff und einer der Reste $R_1$ und $R_3$ Formyl und der andere Wasserstoff, niederes Alkyl, $C_3$- bis $C_7$-Alkenyl oder $C_3$- bis $C_7$-Alkinyl bedeuten, zur entsprechenden Carbonsäure oxydiert, oder

k) eine Verbindung der in Anspruch 1 definierten Formel I, worin A die in Anspruch 1 angegebene Gruppe (a), $R_2$ und $R_4$ je Wasserstoff und einer der Reste $R_1$ und $R_3$ die Gruppe $-COOH$ und der andere Wasserstoff, niederes Alkyl, $C_3$- bis $C_7$-Alkenyl oder $C_3$- bis $C_7$-Alkinyl bedeuten, verestert, oder

l) eine Verbindung der in Anspruch 1 definierten Formel I, worin A die in Anspruch 1 angegebene Gruppe (a), $R_2$ und $R_4$ je Wasserstoff und einer der Reste $R_1$ und $R_3$ niederes Alkoxycarbonyl und der andere Wasserstoff, niederes Alkyl, $C_3$- bis $C_7$-Alkenyl oder $C_3$- bis $C_7$-Alkinyl bedeuten, mit einer Verbindung der Formel $HNR_{35}R_{36}$, worin $R_{35}$ und $R_{36}$ obige Bedeutung besitzen, behandelt, oder

m) eine Verbindung der in Anspruch 1 definierten Formel I, worin A die in Anspruch 1 angegebene Gruppe (a), $R_4$ Wasserstoff, $R_1$ und $R_3$ je Wasserstoff, niederes Alkyl, $C_3$- bis $C_7$-Alkenyl oder $C_3$- bis $C_7$-Alkinyl und $R_2$ Wasserstoff, niederes Alkyl, $C_3$- bis $C_7$-Alkenyl, $C_3$- bis $C_7$-Alkinyl, die Gruppe $-COO-$niederes Alkyl, Hydroxy$-C_2$- bis $C_7$-Alkyl die Gruppe $-(CH_2)_n-COO-$niederes Alkyl oder $-(CH_2)_n-CO-NR_{91}R_{92}$ bedeuten und n, $R_{91}$ und $R_{92}$ obige Bedeutung besitzen, $N_5$-oxydiert, oder

n) eine Verbindung der in Anspruch 1 definierten Formel I, worin A die in Anspruch 1 angegebene Gruppe (b), $R_4$ Wasserstoff, $R_2$ die Gruppe $-COO-$niederes Alkyl und $R_1$ und $R_3$ je Wasserstoff, niederes Alkyl, $C_3$- bis $C_7$-Alkenyl oder $C_3$- bis $C_7$-Alkinyl bedeuten, mit einem den Rest $-COR$ liefernden Acylierungsmittel behandelt, wobei R die in Anspruch 1 angegebene Bedeutung besitzt, oder

o) eine Verbindung der in Anspruch 1 definierten Formel I, worin A die in Anspruch 1 angegebene Gruppe (a), $R_4$ die Gruppe $-OCOR$, $R_2$ die Gruppe $-COO-$niederes Alkyl, $R_1$ und $R_3$ je Wasserstoff, niederes Alkyl, $C_3$- bis $C_7$-Alkenyl oder $C_3$- bis $C_7$-Alkinyl bedeuten und R die in Anspruch 1 angegebene Bedeutung besitzt, oder eine Verbindung der in Anspruch 1 definierten Formel I, worin A die in Anspruch 1 angegebene Gruppe (b), $R_2$ die Gruppe $-COO-$niederes Alkyl, $R_4$ Wasserstoff und $R_1$ und $R_3$ je Wasserstoff, niederes Alkyl, $C_3$- bis $C_7$-Alkenyl oder $C_3$- bis $C_7$-Alkinyl bedeuten, einer alkalischen Hydrolyse unterwirft, oder

p) eine Verbindung der in Anspruch 1 definierten Formel I, worin A die in Anspruch 1 angegebene Gruppe (a), $R_4$ die Gruppe $-OCOR$, $R_2$ die Gruppe $-COO-$niederes Alkyl, $R_1$ und $R_3$ je Wasserstoff, niederes Alkyl, $C_3$- bis $C_7$-Alkenyl, oder $C_3$- bis $C_7$-Alkinyl bedeuten, und R die in Anspruch 1 angegebene Bedeutung besitzt, in Gegenwart eines $C_1$- bis $C_7$-geradkettigen Alkohols mit einem Alkalimetallhydroxid oder -alkoxid behandelt, oder

q) eine Verbindung der in Anspruch 1 definierten Formel I, worin A die in Anspruch 1 angegebene Gruppe (a), $R_4$ Wasserstoff und $R_1$ und $R_3$ je Wasserstoff, niederes Alkyl, $C_3$- bis $C_7$-Alkenyl oder $C_3$- bis $C_7$-Alkinyl bedeuten, reduziert, oder

r) in einer Verbindung der in Anspruch 1 definierten Formel I, worin A die in Anspruch 1 angegebene Gruppe (a), $R_4$ Wasserstoff, $R_1$ und $R_3$ je Wasserstoff, niederes Alkyl, $C_3$- bis $C_7$-Alkenyl oder $C_3$- bis $C_7$-Alkinyl und $R_2$ Wasserstoff, niederes Alkyl, $C_3$- bis $C_7$-Alkenyl, $C_3$- bis $C_7$-Alkinyl, die Gruppe $-COO-$niederes Alkyl, Hydroxy$-C_2$- bis $C_7$-Alkyl oder die Gruppe $-(CH_2)_n-COO-$niederes Alkyl oder $-(CH_2)_n-CO-NR_{91}R_{92}$ bedeuten, und n, $R_{91}$ und $R_{92}$ obige Bedeutung besitzen, die Imingruppe quaternisiert, oder

s) eine Verbindung der in Anspruch 1 definierten Formel I, worin A die in Anspruch 1 angegebene Gruppe (e), $R_4$ Wasserstoff, $R_1$ und $R_3$ je Wasserstoff, niederes Alkyl, $C_3$- bis $C_7$-Alkenyl oder $C_3$- bis $C_7$-Alkinyl und $R_2$ Wasserstoff, niederes Alkyl, $C_3$- bis $C_7$-Alkenyl, $C_3$- bis $C_7$-Alkinyl, die Gruppe $-COO-$niederes Alkyl, Hydroxy$-C_2$- bis $C_7$-Alkyl oder die Gruppe $-(CH_2)_n-COO-$niederes Alkyl oder $-(CH_2)_n-CO-NR_{91}R_{92}$ bedeuten, und n, $R_{91}$ und $R_{92}$ obige Bedeutung besitzen, reduziert, oder

t) eine Verbindung der in Anspruch 1 definierten Formel I, worin A die in Anspruch 1 angegebene Gruppe (a) bedeutet, in ein pharmazeutisch annehmbares Säureadditionssalz überführt.

18. Arzneimittel, enthaltend eine Verbindung gemäß einem der Ansprüche 1—11.

19. Sedative und anxiolytische Mittel, enthaltend eine Verbindung gemäß einem der Ansprüche 1—11.

**Patentansprüche für den Vertragsstaat: AT**

1. Verfahren zur Herstellung von Pyrrolo[3,4—D] [2]benzazepinen der allgemeinen Formel

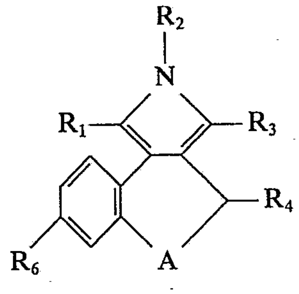

(I)

worin A eine der Gruppen

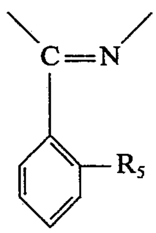 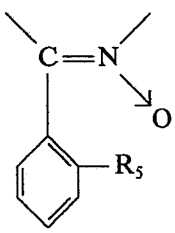 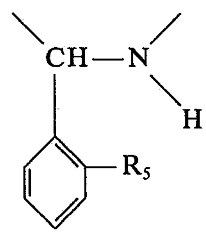 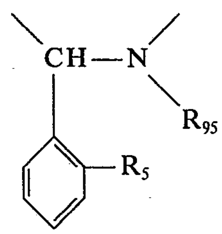 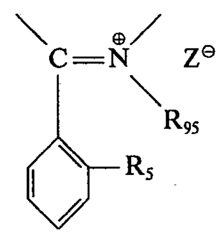

(a) (b) (c) (d) und (e)

$R_1$ Wasserstoff, niederes Alkyl, $C_3$- bis $C_7$-Alkenyl, $C_3$- bis $C_7$-Alkinyl, Hydroxymethyl oder die Gruppe $—COR_{11}$, $R_{11}$ Wasserstoff, Hydroxy, niederes Alkoxy oder Amino oder mono- oder di-niederes Alkylamino, $R_3$ Wasserstoff, niederes Alkyl, $C_3$- bis $C_7$-Alkenyl, $C_3$- bis $C_7$-Alkinyl, Hydroxymethyl oder die Gruppe $—COR_{111}$, $R_{111}$ Wasserstoff, Hydroxy, Trihalomethyl, niederes Alkoxy oder Amino oder mono- oder di-niederes Alkylamino, $R_2$ Wasserstoff, niederes Alkyl, $C_3$- bis $C_7$-Alkenyl, $C_3$- bis $C_7$-Alkinyl, die Gruppe —COO—niederes Alkyl, Hydroxy—$C_2$- bis $C_7$-Alkyl, Amino- oder mono- oder di-niederes Alkylamino—$C_2$- bis $C_7$-Alkyl, oder die Gruppe —$C_1$- bis $C_6$-Alkyl—$COR_{21}$, $R_{21}$ Hydroxy, niederes Alkoxy, Amino oder mono- oder di-niederes Alkylamino, $R_4$ Wasserstoff, geradkettiges niederes Alkoxy, Hydroxy, oder die Gruppe —OCOR, R Wasserstoff, $C_1$- bis $C_6$-Alkyl, niederes Haloalkyl oder Phenyl, $R_5$ Halogen mit einer Ordnungszahl, welche nicht größer als 35 ist, oder Wasserstoff, $R_6$ Halogen mit einer Ordnungszahl, welche nicht größer als 35 ist, $R_{95}$ niederes Alkyl und $Z^{\ominus}$ das Anion einer Säure bedeuten, mit der Maßgabe, daß die als »nieder« bezeichneten Reste höchstens 7 C-Atome aufweisen und den folgenden Maßgaben:

(A) wenn einer der Reste $R_1$ und $R_3$ Hydroxymethyl oder die Gruppe $—COR_{11}$ oder $—COR_{111}$ bedeutet, wobei $R_{11}$ und $R_{111}$ obige Bedeutung besitzen, dann bedeuten der verbleibende Rest Wasserstoff, niederes Alkyl, $C_3$- bis $C_7$-Alkenyl oder $C_3$- bis $C_7$-Alkinyl und $R_2$ Wasserstoff;
(B) wenn $R_4$ —OCOR bedeutet, dann bedeuten $R_1$ und $R_3$ Wasserstoff, niederes Alkyl, $C_3$- bis $C_7$-Alkenyl oder $C_3$- bis $C_7$-Alkinyl und $R_2$ die Gruppe —CO—niederes Alkoxy;
(C) wenn $R_4$ Hydroxy bedeutet, dann bedeuten $R_1$ und $R_3$ Wasserstoff, niederes Alkyl, $C_3$- bis $C_7$-Alkenyl oder $C_3$- bis $C_7$-Alkinyl und $R_2$ Wasserstoff;
(D) wenn $R_4$ geradkettiges niederes Alkoxy bedeutet, dann bedeuten $R_1$ und $R_3$ Wasserstoff, niederes Alkyl, $C_3$- bis $C_7$-Alkenyl oder $C_3$- bis $C_7$-Alkinyl;
(E) wenn A die obige Gruppe (b), (c), (d) oder (e) bedeutet, dann bedeutet $R_4$ Wasserstoff; und
(F) wenn A die obige Gruppe (b) oder (e) bedeutet, dann besitzt $R_2$ eine von Amino- oder mono- oder di-niederes Alkylamino—$C_2$- bis $C_7$-Alkyl verschiedene Bedeutung;

und von pharmazeutisch annehmbaren Säureadditionssalzen von Verbindungen der Formel I, worin A die Gruppe (a) bedeutet, dadurch gekennzeichnet, daß man

a) eine Verbindung der allgemeinen Formel

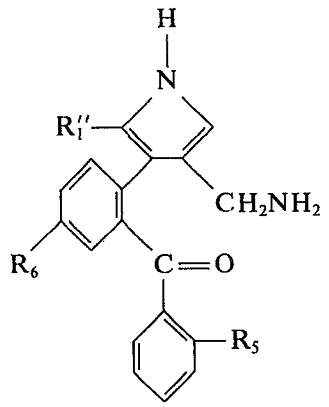

(B)

worin $R_5$ und $R_6$ obige Bedeutung besitzen, und $R_1''$ Wasserstoff, niederes Alkyl, $C_3$- bis $C_7$-Alkenyl oder $C_3$- bis $C_7$-Alkinyl bedeutet, cyclisiert, oder

b) eine Verbindung der Formel I, worin A die obige Gruppe (a), $R_2$ und $R_4$ je Wasserstoff, und einer der Reste $R_1$ und $R_3$ Wasserstoff und der andere Wasserstoff, niederes Alkyl, $C_3$- bis $C_7$-Alkenyl oder $C_3$- bis $C_7$-Alkinyl bedeuten, in der 1- und/oder 3-Stellung niederalkyliert, $C_3$- bis $C_7$-alkenyliert oder $C_3$- bis $C_7$-alkinyliert, oder

c) eine Verbindung der Formel I, worin A die obige Gruppe (a), $R_1$ und $R_3$ je Wasserstoff, niederes Alkyl, $C_3$- bis $C_7$-Alkenyl oder $C_3$- bis $C_7$-Alkinyl und $R_2$ Wasserstoff bedeuten, in der 2-Stellung mit einer niederen Alkyl-, $C_3$- bis $C_7$-Alkenyl-, $C_3$- bis $C_7$-Alkinyl- oder mit einer Hydroxy$-C_2$- bis $C_7$-Alkylgruppe, oder mit der Gruppe $-COO-$niederes Alkyl, $-(CH_2)_n-COO-$niederes Alkyl, $-(CH_2)_n-CO-NR_{91}R_{92}$ oder $-(CH_2)_{n+1}-NR_{91}R_{92}$, worin n die Zahl 1 bis 6 und $R_{91}$ und $R_{92}$ je Wasserstoff oder niederes Alkyl bedeuten, substituiert, oder

d) eine Verbindung der Formel I, worin A die obige Gruppe (a), $R_1$ und $R_3$ je Wasserstoff, niederes Alkyl, $C_3$- bis $C_7$-Alkenyl oder $C_3$- bis $C_7$-Alkinyl und $R_2$ die Gruppe $-(CH_2)_nCOO-$niederes Alkyl oder $-(CH_2)_n-CO-NR_{91}R_{92}$ bedeuten und n, $R_{91}$ und $R_{92}$ obige Bedeutung besitzen, reduziert, oder

e) eine Verbindung der Formel I, worin A die obige Gruppe (a), $R_1$ und $R_3$ je Wasserstoff, niederes Alkyl, $C_3$- bis $C_7$-Alkenyl oder $C_3$- bis $C_7$-Alkinyl und $R_2$ die Gruppe $-(CH_2)_n-COO-$niederes Alkyl bedeuten, und n obige Bedeutung besitzt, in die entsprechende Carbonsäure oder in ein entsprechendes Amid, niederes Alkylamid oder di-niederes Alkylamid umwandelt, oder

f) eine Verbindung der Formel I, worin A die obige Gruppe (a), $R_2$ und $R_4$ je Wasserstoff und einer der Reste $R_1$ und $R_3$ Wasserstoff und der andere Wasserstoff, niederes Alkyl, $C_3$- bis $C_7$-Alkenyl oder $C_3$ bis $C_7$-Alkinyl bedeuten, in der 1- oder 3-Stellung formyliert oder hydroxymethyliert, oder

g) eine Verbindung der Formel I, worin A die obige Gruppe (a), $R_2$ und $R_4$ je Wasserstoff und einer der Reste $R_1$ und $R_3$ Formyl und der andere Wasserstoff, niederes Alkyl, $C_3$- bis $C_7$-Alkenyl oder $C_3$- bis $C_7$-Alkinyl bedeuten, zur entsprechenden Hydroxymethylverbindung reduziert, oder

h) eine Verbindung der Formel I, worin A die obige Gruppe (a), $R_2$, $R_3$ und $R_4$ je Wasserstoff und $R_1$ Wasserstoff, niederes Alkyl, $C_3$- bis $C_7$-Alkenyl oder $C_3$- bis $C_7$-Alkinyl bedeuten, mit einem Trihaloacetylchlorid behandelt, oder

i) eine Verbindung der Formel I, worin A die obige Gruppe (a), $R_2$ und $R_4$ je Wasserstoff, $R_1$ Wasserstoff, niederes Alkyl, $C_3$- bis $C_7$-Alkenyl oder $C_3$- bis $C_7$-Alkinyl und $R_3$ Trihaloacetyl bedeuten, mit einem Alkalimetallniederen Alkoxid oder mit einer Verbindung der Formel $HNR_{35}R_{36}$, worin $R_{35}$ und $R_{36}$ je Wasserstoff oder niederes Alkyl bedeuten, behandelt, oder

j) eine Verbindung der Formel I, worin A die obige Gruppe (a), $R_2$ und $R_4$ je Wasserstoff und einer der Reste $R_1$ und $R_3$ Formyl und der andere Wasserstoff, niederes Alkyl, $C_3$- bis $C_7$-Alkenyl oder $C_3$- bis $C_7$-Alkinyl bedeuten, zur entsprechenden Carbonsäure oxydiert, oder

k) eine Verbindung der Formel I, worin A die obige Gruppe (a), $R_2$ und $R_4$ je Wasserstoff und einer der Reste $R_1$ und $R_3$ die Gruppe $-COOH$ und der andere Wasserstoff, niederes Alkyl, $C_3$- bis $C_7$-Alkenyl oder $C_3$- bis $C_7$-Alkinyl bedeuten, verestert, oder

l) eine Verbindung der Formel I, worin A die obige Gruppe (a), $R_2$ und $R_4$ je Wasserstoff und einer der Reste $R_1$ und $R_3$ niederes Alkoxycarbonyl und der andere Wasserstoff, niederes Alkyl, $C_3$- bis $C_7$-Alkenyl oder $C_3$- bis $C_7$-Alkinyl bedeuten, mit einer Verbindung der Formel $HNR_{35}R_{36}$, worin $R_{35}$ und $R_{36}$ obige Bedeutung besitzen, behandelt, oder

m) eine Verbindung der Formel I, worin A die obige Gruppe (a), $R_4$ Wasserstoff, $R_1$ und $R_3$ je Wasserstoff, niederes Alkyl, $C_3$- bis $C_7$-Alkenyl oder $C_3$- bis $C_7$-Alkinyl und $R_2$ Wasserstoff, niederes Alkyl, $C_3$- bis $C_7$-Alkenyl, $C_3$- bis $C_7$-Alkinyl, die Gruppe $-COO-$niederes Alkyl, Hy-

droxy—$C_2$- bis $C_7$-Alkyl oder die Gruppe $-(CH_2)_n-COO-$niederes Alkyl oder $-(CH_2)_n-CO-NR_{91}R_{92}$ bedeuten und n, $R_{91}$ und $R_{92}$ obige Bedeutung besitzen, $N_5$-oxydiert, oder

n) eine Verbindung der Formel I, worin A die obige Gruppe (b), $R_4$ Wasserstoff, $R_2$ die Gruppe $-COO-$niederes Alkyl und $R_1$ und $R_3$ je Wasserstoff, niederes Alkyl, $C_3$- bis $C_7$-Alkenyl oder $C_3$- bis $C_7$-Alkinyl bedeuten, mit einem den Rest $-COR$ liefernden Acylierungsmittel behandelt, wobei R obige Bedeutung besitzt, oder

o) eine Verbindung der Formel I, worin A die obige Gruppe (a), $R_4$ die Gruppe $-OCOR$, $R_2$ die Gruppe $-COO-$niederes Alkyl, $R_1$ und $R_3$ je Wasserstoff, niederes Alkyl, $C_3$- bis $C_7$-Alkenyl oder $C_3$- bis $C_7$-Alkinyl bedeuten und R obige Bedeutung besitzt, oder eine Verbindung der Formel I, worin A die obige Gruppe (b), $R_2$ die Gruppe $-COO-$niederes Alkyl, $R_4$ Wasserstoff und $R_1$ und $R_3$ je Wasserstoff, niederes Alkyl, $C_3$- bis $C_7$-Alkenyl oder $C_3$- bis $C_7$-Alkinyl bedeuten, einer alkalischen Hydrolyse unterwirft, oder

p) eine Verbindung der Formel I, worin A die obige Gruppe (a), $R_4$ die Gruppe $-OCOR$, $R_2$ die Gruppe $-COO-$niederes Alkyl, $R_1$ und $R_3$ je Wasserstoff, niederes Alkyl, $C_3$- bis $C_7$-Alkenyl oder $C_3$- bis $C_7$-Alkinyl bedeuten und R obige Bedeutung besitzt, in Gegenwart eines $C_1$- bis $C_7$-geradkettigen Alkohols mit einem Alkalimetallhydroxid oder -alkoxid behandelt, oder

q) eine Verbindung der Formel I, worin A die obige Gruppe (a), $R_4$ Wasserstoff und $R_1$ und $R_3$ je Wasserstoff, niederes Alkyl, $C_3$- bis $C_7$-Alkenyl oder $C_3$- bis $C_7$-Alkinyl bedeuten, reduziert, oder

r) in einer Verbindung der Formel I worin A die obige Gruppe (a), $R_4$ Wasserstoff, $R_1$ und $R_3$ je Wasserstoff,niederes Alkyl, $C_3$- bis $C_7$-Alkenyl oder $C_3$- bis $C_7$-Alkinyl und $R_2$ Wasserstoff, niederes Alkyl, $C_3$- bis $C_7$-Alkenyl, $C_3$- bis $C_7$-Alkinyl, die Gruppe $-COO-$niederes Alkyl, Hydroxy—$C_2$- bis $C_7$-Alkyl oder die Gruppe $-(CH_2)_n-COO-$niederes Alkyl oder $-(CH_2)_n-CO-NR_{91}R_{92}$ bedeuten, und n, $R_{91}$ und $R_{92}$ obige Bedeutung besitzen, die Imingruppe quaternisiert, oder

s) eine Verbindung der Formel I, worin A die obige Gruppe (e), $R_4$ Wasserstoff, $R_1$ und $R_3$ je Wasserstoff, niederes Alkyl, $C_3$- bis $C_7$-Alkenyl oder $C_3$- bis $C_7$-Alkinyl und $R_2$ Wasserstoff, niederes Alkyl, $C_3$- bis $C_7$-Alkenyl, $C_3$- bis $C_7$-Alkinyl, die Gruppe $-COO-$niederes Alkyl, Hydroxy—$C_2$- bis $C_7$-Alkyl oder die Gruppe $-(CH_2)_n-COO-$niederes Alkyl oder $-(CH_2)_n-CO-NR_{91}R_{92}$ bedeuten, und n, $R_{91}$ und $R_{92}$ obige Bedeutung besitzen, reduziert, oder

t) eine Verbindung der Formel I, worin A die obige Gruppe (a) bedeutet, in ein pharmazeutisch annehmbares Säureadditionssalz überführt.

2. Verfahren gemäß Anspruch 1 zur Herstellung von Verbindungen der in Anspruch 1 definierten Formel I, worin $R_2$ eine andere Bedeutung als Carboxy—$C_1$- bis $C_6$-Alkyl besitzt, mit den folgenden Maßgaben:

(i) wenn A die Gruppe (a) und $R_4$ geradkettiges niederes Alkoxy bedeuten, dann bedeutet $R_2$ Wasserstoff oder niederes Alkyl; und
(ii) wenn A die Gruppe (b) bedeutet, dann bedeutet $R_2$ Wasserstoff oder die Gruppe $-COO-$niederes Alkyl,

oder von pharmazeutisch annehmbaren Säureadditionssalzen von Verbindungen der Formel I, worin A die Gruppe (a) bedeutet, dadurch gekennzeichnet, daß man diese Verbindungen oder Salze gemäß den Verfahrensvarianten (a), (b), (f), (g), (h), (i), (j), (k), (l), (n), (o), (p), (q), (r) oder (s) herstellt; oder gemäß Verfahrensvariante (c), wobei $R_4$ im Ausgangsmaterial Wasserstoff bedeutet und wobei in dieses Ausgangsmaterial in 2-Stellung ein anderer Substituent eingeführt wird als Hydroxy—$C_2$- bis $C_7$-Alkyl oder $-(CH_2)_n-CO-NR_{91}R_{92}$, oder worin $R_4$ im Ausgangsmaterial geradkettiges niederes Alkoxy bedeutet und wobei dieses Ausgangsmaterial in 2-Stellung durch eine niedere Alkylgruppe substituiert wird; oder gemäß Verfahrensvariante (d), worin $R_4$ im Ausgangsmaterial Wasserstoff bedeutet, und dieses Ausgangsmaterial in ein entsprechendes Amid, niederes Alkylamid oder Di-niederes Alkylamid übergeführt wird; oder gemäß Verfahrensvariante (m), worin $R_2$ im Ausgangsmaterial Wasserstoff oder die Gruppe $-COO-$niederes Alkyl bedeutet.

3. Verfahren gemäß Anspruch 1 oder 2, dadurch gekennzeichnet, daß man Verbindungen der in Anspruch 1 definierten allgemeinen Formel I herstellt, worin A die Gruppe (a) bedeutet.

4. Verfahren gemäß Anspruch 3, dadurch gekennzeichnet, daß man Verbindungen der in Anspruch 1 definierten allgemeinen Formel I herstellt, worin $R_3$ Wasserstoff, $R_5$ und $R_6$ je Halogen, $R_1$ Wasserstoff oder niederes Alkyl und $R_4$ Wasserstoff bedeuten.

5. Verfahren gemäß Anspruch 4, dadurch gekennzeichnet, daß man Verbindungen der in Anspruch 1 definierten allgemeinen Formel I herstellt, worin $R_5$ Chlor oder Fluor und $R_6$ Chlor bedeuten.

6. Verfahren gemäß Anspruch 1, dadurch gekennzeichnet, daß man 8-Chlor-6-(2-chlorphenyl)-2 H,4 H-pyrrolo[3,4-d] [2]benzazepin herstellt.

7. Verfahren gemäß Anspruch 1, dadurch gekennzeichnet, daß man 8-Chlor-6-(2-fluorphenyl)-2 H,4 H-pyrrolo[3,4-d] [2]benzazepin herstellt.

8. Verfahren gemäß Anspruch 1, dadurch gekennzeichnet, daß man 8-Chlor-6-(2-chlorphenyl)-1-methyl-2 H,4 H-pyrrolo[3,4-d] [2]benzazepin herstellt.

9. Verfahren gemäß Anspruch 1, dadurch gekennzeichnet, daß man 8-Chlor-6-(2-chlorphenyl)-2-methyl-2 H,4 H-pyrrolo[3,4 – d] [2]benzazepin herstellt.

10. Verfahren gemäß Anspruch 1, dadurch gekennzeichnet, daß man 8-Chlor-6-(2-fluorphenyl)-2 H,4 H-pyrrolo[3,4-d] [2]benzazepin-2-äthanol herstellt.

11. Verfahren gemäß Anspruch 1, dadurch gekennzeichnet, daß man 8 Chlor 6 (2 chlorphenyl)-2 H,4 H-pyrrolo[3,4-d] [2]benzazepin-2-carbonsäuremethylester herstellt.

**Claims for the Contracting States: BE, CH, DE, FR, GB, IT, LI, LU, NL, SE**

1. Pyrrolo[3,4-d] [2]benzazepines of the general formula

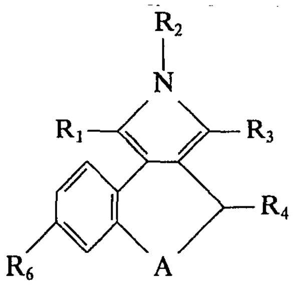

(I)

wherein A signifies one of the groups

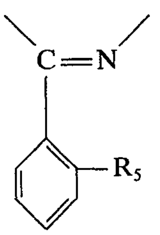 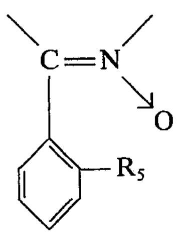 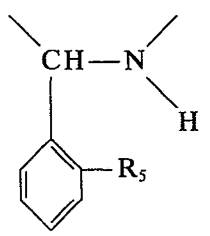 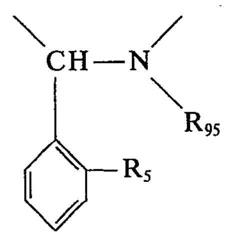 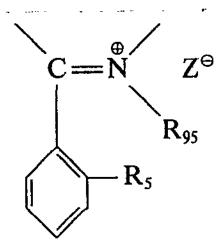

(a) (b) (c) (d) and (e)

$R_1$ signifies hydrogen, lower alkyl, $C_3$- to $C_7$-alkenyl, $C_3$- to $C_7$-alkynyl, hydroxymethyl or the group $-COR_{11}$, $R_{11}$ signifies hydrogen, hydroxy, lower alkoxy or amino or mono- or di-lower alkylamino, $R_3$ signifies hydrogen, lower alkyl, $C_3$- to $C_7$-alkenyl, $C_3$- to $C_7$-alkynyl, hydroxymethyl or the group $-COR_{111}$, $R_{111}$ signifies hydrogen, hydroxy, trihalomethyl, lower alkoxy or amino or mono- or di-lower alkylamino, $R_2$ signifies hydrogen, lower alkyl, $C_3$- to $C_7$-alkenyl, $C_3$- to $C_7$-alkynyl, the group $-COO-$lower alkyl, hydroxy$-C_2$- to $C_7$-alkyl, amino- or mono- or di- lower alkylamino$-C_2$- to $C_7$-alkyl or the group $-C_1$- to $C_6$-alkyl$-COR_{21}$, $R_{21}$ signifies hydroxy, lower alkoxy, amino or mono- or di-lower alkylamino, $R_4$ signifies hydrogen, straight-chain lower alkoxy, hydroxy or the group $-OCOR$, R signifies hydrogen, $C_1$- to $C_6$-alkyl, lower haloalkyl or phenyl, $R_5$ signifies halogen having an atomic number which ist not greater than 35 or hydrogen, $R_6$ signifies halogen having an atomic number which is not greater than 35, $R_{95}$ signifies lower alkyl and $Z^{\ominus}$ signifies the anion of an acid, with the proviso that the residues denoted as »lower« have at most 7 C-atoms and the following provisos:

(A) when one of the residues $R_1$ and $R_3$ signifies hydroxymethyl or the group $-COR_{11}$ or $-COR_{111}$, in which $R_{11}$ and $R_{111}$ have the above significance, then the remaining residue signifies hydrogen, lower alkyl, $C_3$- to $C_7$-alkenyl or $C_3$- to $C_7$-alkynyl and $R_2$ signifies hydrogen;
(B) when $R_4$ signifies $-OCOR$, then $R_1$ and $R_3$ signify hydrogen, lower alkyl, $C_3$- to $C_7$-alkenyl or $C_3$- to $C_7$-alkynyl and $R_2$ signifies the group $-CO-$lower alkoxy;
(C) when $R_4$ signifies hydroxy, then $R_1$ and $R_3$ signify hydrogen, lower alkyl, $C_3$- to $C_7$-alkenyl or $C_3$- to $C_7$-alkynyl and $R_2$ signifies hydrogen;
(D) when $R_4$ signifies straight-chain lower alkoxy, then $R_1$ and $R_3$ signify hydrogen, lower alkyl, $C_3$-to $C_7$-alkenyl or $C_3$- to $C_7$-alkynyl;
(E) when A signifies the above group (b), (c), (d) or (e), then $R_4$ signifies hydrogen; and
(F) when A signifies the above group (b) or (e), then $R_2$ has a significance different from amino- or mono- or di-lower alkylamino$-C_2$- to $C_7$-alkyl;

and pharmaceutically acceptable acid addition salts of compounds of formula I in which A signifies the group (a).

2. Compounds in accordance with claim 1, characterized in that $R_2$ has a significance other than carboxy$-C_1$- to $C_6$-alkyl, with the following provisos:

(i) when A signifies the group (a) and $R_4$ signifies straight-chain lower alkoxy, then $R_2$ signifies hydrogen or lower alkyl; and
(ii) when A signifies the group (b), then $R_2$ signifies hydrogen or the group $-COO-$lower alkyl.

3. Compounds in accordance with claim 1 or 2, characterized in that A signifies the group (a).
4. Compounds in accordance with claim 3, characterized in that $R_3$ signifies hydrogen, $R_5$ and $R_6$ each signify halogen, $R_1$ signifies hydrogen or lower alkyl and $R_4$ signifies hydrogen.
5. Compounds in accordance with claim 4, characterized in that $R_5$ signifies chlorine or fluorine and $R_6$ signifies chlorine.
6. 8-Chloro-6-(2-chlorophenyl)-2 H,4 H-pyrrolo[3,4-d] [2]benzazepine.
7. 8-Chloro-6-(2-fluorophenyl)-2 H,4 H-pyrrolo[3,4-d] [2]benzazepine.
8. 8-Chloro-6-(2-chlorophenyl)-1-methyl-2 H,4 H-pyrrolo[3,4-d] [2]benzazepine.
9. 8-Chloro-6-(2-chlorophenyl)-2-methyl-2 H,4 H-pyrrolo[3,4-d] [2]benzazepine.
10. 8-Chloro-6-(2-fluorophenyl)-2 H,4 H-pyrrolo[3,4-d] [2]benzazepine-2-ethanol.
11. 8-Chloro-6-(2-chlorophenyl)-2 H,4 H-pyrrolo[3,4-d] [2]benzazepine-2-carboxylic acid methyl ester.
12. Compounds of the general formula

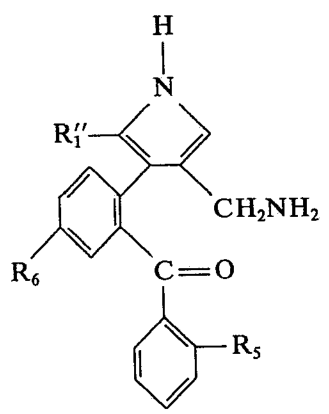

(B)

wherein $R_5$ and $R_6$ have the significance given in claim 1, and $R_1''$ signifies hydrogen, lower alkyl, $C_3$- to $C_7$-alkenyl or $C_3$- to $C_7$-alkynyl.
13. Compounds of the general formula

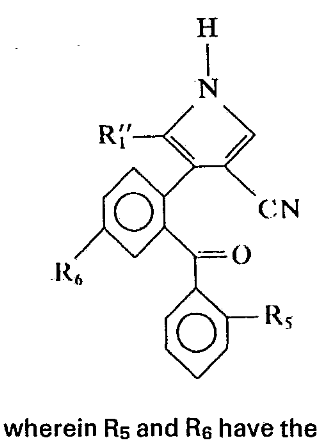

(XI)

wherein $R_5$ and $R_6$ have the significance given in claim 1, and $R_1''$ signifies hydrogen, lower alkyl, $C_3$- to $C_7$-alkenyl or $C_3$- to $C_7$-alkynyl.

14. Compounds of the general formula

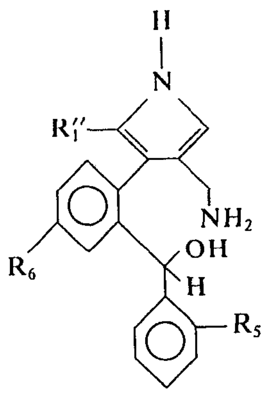

(XII)

wherein $R_5$ and $R_6$ have the significance given in claim 1, and $R_1''$ signifies hydrogen, lower alkyl, $C_3$- to $C_7$-alkenyl or $C_3$- to $C_7$-alkynyl.

15. Compounds in accordance with any one of claims 1—11 as pharmaceutically active substances.

16. Compounds in accordance with any one of claims 1—11 as sedative and anxiolytic active substances.

17. A process for the manufacture of compounds in accordance with any one of claims 1—11, characterized by

a) cyclizing a compound of the general formula

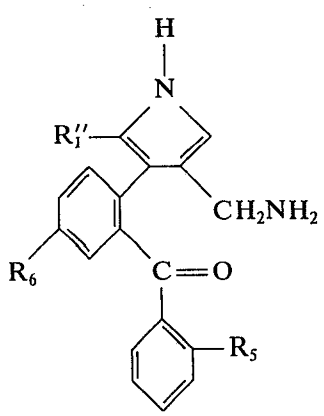

(B)

wherein $R_5$ and $R_6$ have the above significance, and $R_1''$ signifies hydrogen, lower alkyl, $C_3$- to $C_7$-alkenyl or $C_3$- to $C_7$-alkynyl, or

b) lower alkylating, $C_3$- to $C_7$-alkenylating or $C_3$- to $C_7$-alkynylating in the 1- and/or 3-position a compound of formula I defined in claim 1 wherein A signifies the group (a) given in claim 1, $R_2$ and $R_4$ each signify hydrogen, and one of the residues $R_1$ and $R_3$ signifies hydrogen and the other signifies hydrogen, lower alkyl, $C_3$- to $C_7$-alkenyl or $C_3$- to $C_7$-alkynyl, or

c) substituting in the 2-position a compound of formula I defined in claim 1 wherein A signifies the group (a) given in claim 1, $R_1$ and $R_3$ each signify hydrogen, lower alkyl, $C_3$- to $C_7$-alkenyl or $C_3$- to $C_7$-alkynyl and $R_2$ signifies hydrogen with a lower alkyl, $C_3$- to $C_7$-alkenyl, $C_3$- to $C_7$-alkynyl or with a hydroxy—$C_2$- to $C_7$-alkyl group, or with the group —COO—lower alkyl, $-(CH_2)_n-COO-$lower alkyl, $-(CH_2)_n-CO-NR_{91}R_{92}$ or $-(CH_2)_{n+1}-NR_{91}R_{92}$ wherein n signifies the number 1 to 6 and $R_{91}$ and $R_{92}$ each signify hydrogen or lower alkyl, or

d) reducing a compound of formula I defined in claim 1 wherein A signifies the group (a) given in claim 1, $R_1$ and $R_3$ each signify hydrogen, lower alkyl, $C_3$- to $C_7$-alkenyl or $C_3$- to $C_7$-alkynyl and $R_2$ signifies the group $-(CH_2)_nCOO-$lower alkyl or $-(CH_2)_n-CO-NR_{91}R_{92}$ and n, $R_{91}$ and $R_{92}$ have the above significance, or

e) converting a compound of formula I defined in claim 1 wherein A signifies the group (a) given in claim 1, $R_1$ and $R_3$ each signify hydrogen, lower alkyl, $C_3$- to $C_7$-alkenyl or $C_3$- to $C_7$-alkynyl and $R_2$ signifies the group $-(CH_2)_nCOO-$lower alkyl and n has the above significance into the corresponding carboxylic acid or into a corresponding amide, lower alkyl amide or di-lower alkyl amide,

f) formylating or hydroxymethylating in the 1- or 3-position a compound of formula I defined in claim 1 wherein A signifies the group (a) given in claim 1, $R_2$ and $R_4$ each signify hydrogen and one of the residues $R_1$ and $R_3$ signifies hydrogen and the other signifies hydrogen, lower alkyl, $C_3$- to $C_7$-alkenyl or $C_3$- to $C_7$-alkynyl, or

g) reducing a compound of formula I defined in claim 1 wherein A signifies the group (a) given in claim 1, $R_2$ and $R_4$ each signify hydrogen and one of the residues $R_1$ and $R_3$ signifies formyl and the other signifies hydrogen, lower alkyl, $C_3$- to $C_7$-alkenyl or $C_3$- to $C_7$-alkynyl to the corresponding hydroxymethyl compound, or

h) treating a compound of formula I defined in claim 1 wherein A signifies the group (a) given in claim 1, $R_2$, $R_3$ and $R_4$ each signify hydrogen and $R_1$ signifies hydrogen, lower alkyl, $C_3$- to $C_7$-alkenyl or $C_3$- to $C_7$-alkynyl with a trihaloacetyl chloride, or

i) treating a compound of formula I defined in claim 1 wherein A signifies the group (a) given in claim 1, $R_2$ and $R_4$ each signify hydrogen, $R_1$ signifies hydrogen, lower alkyl, $C_3$- to $C_7$-alkenyl or $C_3$- to $C_7$-alkynyl and $R_3$ signifies trihaloacetyl with an alkali metal lower alkoxide or with a compound of the formula $HNR_{35}R_{36}$ wherein $R_{35}$ and $R_{36}$ each signify hydrogen or lower alkyl, or

j) oxidizing a compound of formula I defined in claim 1 wherein A signifies the group (a) given in claim 1, $R_2$ and $R_4$ each signify hydrogen and one of the residues $R_1$ and $R_3$ signifies formyl and the other signifies hydrogen, lower alkyl, $C_3$- to $C_7$-alkenyl or $C_3$- to $C_7$-alkynyl to the corresponding carboxylic acid, or

k) esterifying a compound of formula I defined in claim 1 wherein A signifies the group (a) given in claim 1, $R_2$ and $R_4$ each signify hydrogen and one of the residues $R_1$ and $R_3$ signifies the group $-COOH$ and the other signifies hydrogen, lower alkyl, $C_3$- to $C_7$-alkenyl or $C_3$- to $C_7$-alkynyl, or

l) treating a compound of formula I defined in claim 1 wherein A signifies the group (a) given in claim 1, $R_2$ and $R_4$ each signify hydrogen and one of the residues $R_1$ and $R_3$ signifies lower alkoxycarbonyl and the other signifies hydrogen, lower alkyl, $C_3$- to $C_7$-alkenyl or $C_3$- to $C_7$-alkynyl with a compound of the formula $HNR_{35}R_{36}$ wherein $R_{35}$ and $R_{36}$ have the above significance, or

m) $N_5$-oxidizing a compound of formula I defined in claim 1 wherein A signifies the group (a) given in claim 1, $R_4$ signifies hydrogen, $R_1$ and $R_3$ each signify hydrogen, lower alkyl, $C_3$- to $C_7$-alkenyl or $C_3$- to $C_7$-alkynyl and $R_2$ signifies hydrogen, lower alkyl, $C_3$- to $C_7$-alkenyl, $C_3$- to $C_7$-alkynyl, the group $-COO-$lower alkyl, hydroxy$-C_2$- to $C_7$-alkyl, the group $-(CH_2)_n-COO-$lower alkyl or $-(CH_2)_n-CO-NR_{91}R_{92}$ and n, $R_{91}$ and $R_{92}$ have the above significance, or

n) treating a compound of formula I defined in claim 1 wherein A signifies the group (b) given in claim 1, $R_4$ signifies hydrogen, $R_2$ signifies the group $-COO-$lower alkyl and $R_1$ and $R_3$ each signify hydrogen, lower alkyl, $C_3$- to $C_7$-alkenyl or $C_3$- to $C_7$-alkynyl with an acylating agent yielding the residue $-COR$ in which R has the significance given in claim 1, or

o) subjecting a compound of formula I defined in claim 1 wherein A signifies the group (a) given in claim 1, $R_4$ signifies the group $-OCOR$, $R_2$ signifies the group $-COO-$lower alkyl, $R_1$ and $R_3$ each signify hydrogen, lower alkyl, $C_3$- to $C_7$-alkenyl or $C_3$- to $C_7$-alkynyl and R has the significance given in claim 1 or a compound of formula I defined in claim 1 wherein A signifies the group (b) given in claim 1, $R_2$ signifies the group $-COO-$lower alkyl, $R_4$ signifies hydrogen and $R_1$ and $R_3$ each signify hydrogen, lower alkyl, $C_3$- to $C_7$-alkenyl or $C_3$- to $C_7$-alkynyl to an alkaline hydrolysis, or

p) treating a compound of formula I defined in claim 1 wherein A signifies the group (a) given in claim 1, $R_4$ signifies the group $-OCOR$, $R_2$ signifies the group $-COO-$lower alkyl, $R_1$ and $R_3$ each signify hydrogen, lower alkyl, $C_3$- to $C_7$-alkenyl or $C_3$- to $C_7$-alkynyl and R has the significance given in claim 1 with an alkali metal hydroxide or alkoxide in the presence of a $C_1$- to $C_7$-straight chain alcohol, or

q) reducing a compound of formula I defined in claim 1 wherein A signifies the group (a) given in claim 1, $R_4$ signifies hydrogen and $R_1$ and $R_3$ each signify hydrogen, lower alkyl, $C_3$- to $C_7$-alkenyl or $C_3$- to $C_7$-alkynyl, or

r) quaternizing the imine group in a compound of formula I defined in claim 1 wherein A signifies the group (a) given in claim 1, $R_4$ signifies hydrogen, $R_1$ and $R_3$ each signify hydrogen, lower alkyl, $C_3$- to $C_7$-alkenyl or $C_3$- to $C_7$-alkynyl and $R_2$ signifies hydrogen, lower alkyl, $C_3$- to $C_7$-alkenyl, $C_3$- to $C_7$-alkynyl, the group $-COO-$lower alkyl, hydroxy$-C_2$- to $C_7$-alkyl or the group $-(CH_2)_n-COO-$lower alkyl or $-(CH_2)_n-CO-NR_{91}R_{92}$ and n, $R_{91}$ and $R_{92}$ have the above significance, or

s) reducing a compound of formula I defined in claim 1 wherein A signifies the group (e) given in claim 1, $R_4$ signifies hydrogen, $R_1$ and $R_3$ each signify hydrogen, lower alkyl, $C_3$- to $C_7$-alkenyl or $C_3$- to $C_7$-alkynyl and $R_2$ signifies hydrogen, lower alkyl, $C_3$- to $C_7$-alkenyl, $C_3$- to $C_7$-alkynyl, the group $-COO-$lower alkyl, hydroxy$-C_2$- to $C_7$-alkyl or the group $-(CH_2)_n-COO-$lower alkyl or $-(CH_2)_n-CO-NR_{91}R_{92}$ and n, $R_{91}$ and $R_{92}$ have the above significance, or

t) converting a compound of formula I defined in claim 1 wherein A signifies the group (a) given in claim 1 into a pharmaceutically acceptable acid addition salt.

18. Medicaments containing a compound in accordance with any one of Claims 1—11.

19. Sedative and anxiolytic medicaments containig a compound in accordance with any one of Claims 1—11.

**Claims for the Contracting State: AT**

1. A process for the manufacture of pyrrolo[3,4-d] [2]benzazepines of the general formula

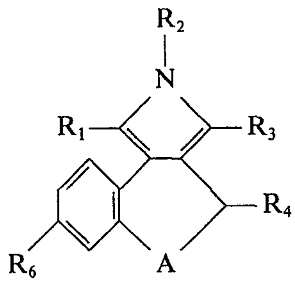

(I)

wherein A signifies one of the groups

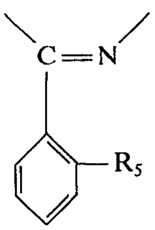 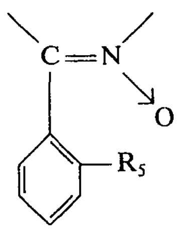 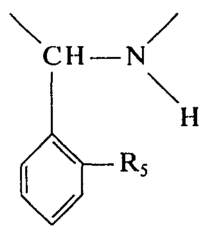 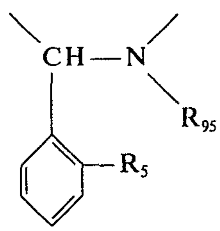 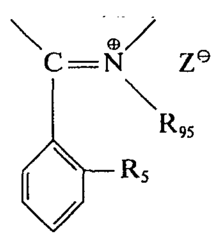

(a) (b) (c) (d) and (e)

$R_1$ signifies hydrogen, lower alkyl, $C_3$- to $C_7$-alkenyl, $C_3$- to $C_7$-alkynyl, hydroxymethyl or the group $-COR_{11}$, $R_{11}$ signifies hydrogen, hydroxy, lower alkoxy or amino or mono- or di-lower alkylamino, $R_3$ signifies hydrogen, lower alkyl, $C_3$- to $C_7$-alkenyl, $C_3$- to $C_7$-alkynyl, hydroxymethyl or the group $-COR_{111}$, $R_{111}$ signifies hydrogen, hydroxy, trihalomethyl, lower alkoxy or amino or mono- or di-lower alkylamino, $R_2$ signifies hydrogen, lower alkyl, $C_3$- to $C_7$-alkenyl, $C_3$- to $C_7$-alkynyl, the group $-COO-$lower alkyl, hydroxy$-C_2$- to $C_7$-alkyl, amino- or mono- or di- lower alkylamino-$C_2$- to $C_7$-alkyl or the group $-C_1$- to $C_6$-alkyl$-COR_{21}$, $R_{21}$ signifies hydroxy, lower alkoxy, amino or mono- or di-lower alkylamino, $R_4$ signifies hydrogen, straight-chain lower alkoxy, hydroxy or the group $-OCOR$, R signifies hydrogen, $C_1$- to $C_6$-alkyl, lower haloalkyl or phenyl, $R_5$ signifies halogen having an atomic number which is not greater than 35 or hydrogen, $R_6$ signifies halogen having an atomic number which is not greater than 35, $R_{95}$ signifies lower alkyl and $Z^{\ominus}$ signifies the anion of an acid, with the proviso that the residues denoted as »lower« have at most 7 C-atoms and the following provisos:

(A) when one of the residues $R_1$ and $R_3$ signifies hydroxymethyl or the group $-COR_{11}$ or $-COR_{111}$, in which $R_{11}$ and $R_{111}$ have the above significance, then the remaining residue signifies hydrogen, lower alkyl, $C_3$- to $C_7$-alkenyl or $C_3$- to $C_7$-alkynyl and $R_2$ signifies hydrogen;
(B) when $R_4$ signifies $-OCOR$, then $R_1$ and $R_3$ signify hydrogen, lower alkyl, $C_3$- to $C_7$-alkenyl or $C_3$- to $C_7$-alkynyl and $R_2$ signifies the group $-CO-$lower alkoxy;
(C) when $R_4$ signifies hydroxy, then $R_1$ and $R_3$ signify hydrogen, lower alkyl, $C_3$- to $C_7$-alkenyl or $C_3$- to $C_7$-alkynyl and $R_2$ signifies hydrogen;
(D) when $R_4$ signifies straight-chain lower alkoxy, then $R_1$ and $R_3$ signify hydrogen, lower alkyl, $C_3$- to $C_7$-alkenyl or $C_3$- to $C_7$-alkynyl;
(E) when A signifies the above group (b), (c), (d) or (e), then $R_4$ signifies hydrogen; and
(F) when A signifies the above group (b) or (e), then $R_2$ has a significance different from amino- or mono- or di-lower alkylamino$-C_2$- to $C_7$-alkyl;

and of pharmaceutically acceptable acid addition salts of compounds of formula I wherein A signifies the group (a), characterized by

a) cyclizing a compound of the general formula

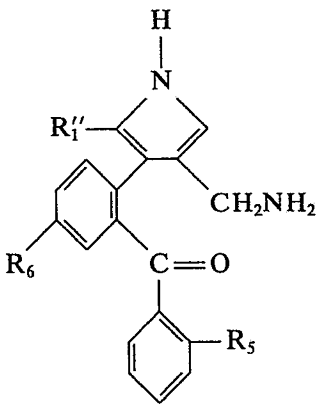

(B)

wherein $R_5$ and $R_6$ have the above significance, and $R_1''$ signifies hydrogen, lower alkyl, $C_3$- to $C_7$-alkenyl or $C_3$- to $C_7$-alkynyl, or

b) lower alkylating, $C_3$- to $C_7$-alkenylating or $C_3$- to $C_7$-alkynylating in the 1- and/or 3-position a compound of formula I wherein A signifies the group (a), above, $R_2$ and $R_4$ each signify hydrogen, and one of the residues $R_1$ and $R_3$ signifies hydrogen and the other signifies hydrogen, lower alkyl, $C_3$- to $C_7$-alkenyl or $C_3$- to $C_7$-alkynyl, or

c) substituting in the 2-position a compound of formula I wherein A signifies the group (a), above, $R_1$ and $R_3$ each signify hydrogen, lower alkyl, $C_3$- to $C_7$-alkenyl or $C_3$- to $C_7$-alkynyl and $R_2$ signifies hydrogen with a lower alkyl, $C_3$- to $C_7$-alkenyl, $C_3$- to $C_7$-alkynyl or with a hydroxy$-C_2$- to $C_7$-alkyl group, or with the group $-COO-$lower alkyl, $-(CH_2)_n-COO-$lower alkyl, $-(CH_2)_n-CO-NR_{91}R_{92}$ or $-(CH_2)_{n+1}-NR_{91}R_{92}$ wherein n signifies the number 1 to 6 and $R_{91}$ and $R_{92}$ each signify hydrogen or lower alkyl, or

d) reducing a compound of formula I wherein A signifies the group (a), above, $R_1$ and $R_3$ each signify hydrogen, lower alkyl, $C_3$- to $C_7$-alkenyl or $C_3$- to $C_7$-alkynyl and $R_2$ signifies the group $-(CH_2)_nCOO-$lower alkyl or $-(CH_2)_n-CO-NR_{91}R_{92}$ and n, $R_{91}$ and $R_{92}$ have the above significance, or

e) converting a compound of formula I wherein A signifies the group (a), above, $R_1$ and $R_3$ each signify hydrogen, lower alkyl, $C_3$- to $C_7$-alkenyl or $C_3$- to $C_7$-alkynyl and $R_2$ signifies the group $-(CH_2)_nCOO-$lower alkyl and n has the above significance into the corresponding carboxylic acid or into a corresponding amide, lower alkyl amide or di-lower alkyl amide, or

f) formylating or hydroxymethylating in the 1- or 3-position a compound of formula I wherein A signifies the group (a), above, $R_2$ and $R_4$ each signify hydrogen and one of the residues $R_1$ and $R_3$ signifies hydrogen and the other signifies hydrogen, lower alkyl, $C_3$- to $C_7$-alkenyl or $C_3$- to $C_7$-alkynyl, or

g) reducing a compound of formula I wherein A signifies the group (a), above, $R_2$ and $R_4$ each signify hydrogen and one of the residues $R_1$ and $R_3$ signifies formyl and the other signifies hydrogen, lower alkyl, $C_3$- to $C_7$-alkenyl or $C_3$- to $C_7$-alkynyl to the corresponding hydroxymethyl compound, or

h) treating a compound of formula I wherein A signifies the group (a), above, $R_2$, $R_3$ and $R_4$ each signify hydrogen and $R_1$ signifies hydrogen, lower alkyl, $C_3$- to $C_7$-alkenyl or $C_3$- to $C_7$-alkynyl with a trihaloacetyl chloride, or

i) treating a compound of formula I wherein A signifies the group (a), above, $R_2$ and $R_4$ each signify hydrogen, $R_1$ signifies hydrogen, lower alkyl, $C_3$- to $C_7$-alkenyl or $C_3$- to $C_7$-alkynyl and $R_3$ signifies trihaloacetyl with an alkali metal lower alkoxide or with a compound of the formula $HNR_{35}R_{36}$ wherein $R_{35}$ and $R_{36}$ each signify hydrogen or lower alkyl, or

j) oxidizing a compound of formula I wherein A signifies the group (a), above, $R_2$ and $R_4$ each signify hydrogen and one of the residues $R_1$ and $R_3$ signifies formyl and the other signifies hydrogen, lower alkyl, $C_3$- to $C_7$-alkenyl or $C_3$- to $C_7$-alkynyl to the corresponding carboxylic acid, or

k) esterifying a compound of formula I wherein A signifies the group (a), above, $R_2$ and $R_4$ each signify hydrogen and one of the residues $R_1$ and $R_3$ signifies the group $-COOH$ and the other signifies hydrogen, lower alkyl, $C_3$- to $C_7$-alkenyl or $C_3$- to $C_7$-alkynyl, or

l) treating a compound of formula I wherein A signifies the group (a), above, $R_2$ and $R_4$ each signify hydrogen and one of the residues $R_1$ and $R_3$ signifies lower alkoxycarbonyl and the other signifies hydrogen, lower alkyl, $C_3$- to $C_7$-alkenyl or $C_3$- to $C_7$-alkynyl with a compound of the formula $HNR_{35}R_{36}$ wherein $R_{35}$ and $R_{36}$ have the above significance, or

m) $N_5$-oxidizing a compound of formula I wherein A signifies the group (a), above, $R_4$ signifies hydrogen, $R_1$ and $R_3$ each signify hydrogen, lower alkyl, $C_3$- to $C_7$-alkenyl or $C_3$- to $C_7$-alkynyl and $R_2$ signifies hydrogen, lower alkyl, $C_3$- to $C_7$-alkenyl, $C_3$- to $C_7$-alkynyl, the group $-COO-$lower

alkyl, hydroxy—$C_2$- to $C_7$-alkyl, the group $-(CH_2)_n-COO-$lower alkyl or $-(CH_2)_n-CO-NR_{91}R_{92}$ and n, $R_{91}$ and $R_{92}$ have the above significance, or

n) treating a compound of formula I wherein A signifies the group (b), above, $R_4$ signifies hydrogen, $R_2$ signifies the group $-COO-$lower alkyl and $R_1$ and $R_3$ each signify hydrogen, lower alkyl, $C_3$- to $C_7$-alkenyl or $C_3$- to $C_7$-alkynyl with an acylating agent yielding the residue $-COR$ in which R has the above significance, or

o) subjecting a compound of formula I wherein A signifies the group (a), above, $R_4$ signifies the group $-OCOR$, $R_2$ signifies the group $-COO-$lower alkyl, $R_1$ and $R_3$ each signify hydrogen, lower alkyl, $C_3$- to $C_7$-alkenyl or $C_3$- to $C_7$-alkynyl and R has the above significance or a compound of formula I wherein A signifies the group (b), above, $R_2$ signifies the group $-COO-$lower alkyl, $R_4$ signifies hydrogen and $R_1$ and $R_3$ each signify hydrogen, lower alkyl, $C_3$- to $C_7$-alkenyl or $C_3$- to $C_7$-alkynyl to an alkaline hydrolysis, or

p) treating a compound of formula I wherein A signifies the group (a), above, $R_4$ signifies the group $-OCOR$, $R_2$ signifies the group $-COO-$lower alkyl, $R_1$ and $R_3$ each signify hydrogen, lower alkyl, $C_3$- to $C_7$-alkenyl or $C_3$- to $C_7$-alkynyl and R has the above significance with an alkali metal hydroxide or alkoxide in the presence of a $C_1$- to $C_7$-straight chain alcohol, or

q) reducing a compound of formula I wherein A signifies the group (a), above, $R_4$ signifies hydrogen and $R_1$ and $R_3$ each signify hydrogen, lower alkyl, $C_3$- to $C_7$-alkenyl or $C_3$- to $C_7$-alkynyl, or

r) quaternizing the imine group in a compound of formula I wherein A signifies the group (a), above, $R_4$ signifies hydrogen, $R_1$ and $R_3$ each signify hydrogen, lower alkyl, $C_3$- to $C_7$-alkenyl or $C_3$- to $C_7$-alkynyl and $R_2$ signifies hydrogen, lower alkyl, $C_3$- to $C_7$-alkenyl, $C_3$- to $C_7$-alkynyl, the group $-COO-$lower alkyl, hydroxy—$C_2$- to $C_7$-alkyl or the group $-(CH_2)_n-COO-$lower alkyl or $-(CH_2)_n-CO-NR_{91}R_{92}$ and n, $R_{91}$ and $R_{92}$ have the above significance, or

s) reducing a compound of formula I wherein A signifies the group (e), above, $R_4$ signifies hydrogen, $R_1$ and $R_3$ each signify hydrogen, lower alkyl, $C_3$- to $C_7$-alkenyl or $C_3$- to $C_7$-alkynyl and $R_2$ signifies hydrogen, lower alkyl, $C_3$- to $C_7$-alkenyl, $C_3$- to $C_7$-alkynyl, the group $-COO-$lower alkyl, hydroxy—$C_2$- to $C_7$-alkyl or the group $-(CH_2)_n-COO-$lower alkyl or $-(CH_2)_n-CO-NR_{91}R_{92}$ and n, $R_{91}$ and $R_{92}$ have the above significance, or

t) converting a compound of formula I wherein A signifies the group (a), above, into a pharmaceutically acceptable acid addition salt.

2. A process in accordance with claim 1 for the manufacture of compounds of formula I defined in claim 1 wherein $R_2$ has a significance other than carboxy—$C_1$- to $C_6$-alkyl, with the following provisos:

(i) when A signifies the group (a) and $R_4$ signifies straight-chain lower alkoxy, then $R_2$ signifies hydrogen or lower alkyl; and

(ii) when A signifies the group (b), then $R_2$ signifies hydrogen or the group $-COO-$lower alkyl.

or of pharmaceutically acceptable acid addition salts of compounds of formula I wherein A signifies the group (a), characterized in that these compounds or salts are manufactured in accordance with process variants (a), (b), (f), (g), (h), (i), (j), (k), (l), (n), (o), (p), (q), (r) or (s); or in accordance with process variant (c), whereby $R_4$ in the starting material signifies hydrogen and whereby a substituent other than hydroxy—$C_2$- to $C_7$-alkyl or $-(CH_2)_n-CO-NR_{91}R_{92}$ is introduced into this starting material in the 2-position, or wherein $R_4$ in the starting material signifies straight-chain lower alkoxy and whereby this starting material is substituted in the 2-position by a lower alkyl group; or in accordance with process variant (d) wherein $R_4$ in the starting material signifies hydrogen and this starting material is converted into a corresponding amide, lower alkyl amide or di-lower alkyl amide; or in accordance with process variant (m) wherein $R_2$ in the starting material signifies hydrogen or the group $-COO-$lower alkyl.

3. A process in accordance with claim 1 or 2, characterized in that there are manufactured compounds of general formula I defined in claim 1 wherein A signifies the group (a).

4. A process in accordance with claim 3, characterized in that there are manufactured compounds of general formula I defined in claim 1 wherein $R_3$ signifies hydrogen, $R_5$ and $R_6$ each signify halogen, $R_1$ signifies hydrogen or lower alkyl and $R_4$ signifies hydrogen.

5. A process in accordance with claim 4, characterized in that there are manufactured compounds of general formula I defined in claim 1 wherein $R_5$ signifies chlorine or fluorine and $R_6$ signifies chlorine.

6. A process in accordance with claim 1, characterized in that 8-chloro-6-(2-chlorophenyl)-2 H, 4 H-pyrrolo[3,4-d] [2]benzazepine is manufactured.

7. A process in accordance with claim 1, characterized in that 8-chloro-6-(2-fluorophenyl)-2 H,4 H-pyrrolo[3,4-d] [2]benzazepine is manufactured.

8. A process in accordance with claim 1, characterized in that 8-chloro-6-(2-chlorophenyl)-1-methyl-2 H,4 H-pyrrolo[3,4-d] [2]benzazepine is manufactured.

9. A process in accordance with claim 1, characterized in that 8-chloro-6-(2-chlorophenyl)-2-methyl-2 H,4 H-pyrrolo[3,4-d] [2]benzazepine is manufactured.

10. A process in accordance with claim 1, characterized in that 8-chloro-6-(2-fluorophenyl)-2 H,4 H-pyrrolo[3,4-d] [2]benzazepine-2-ethanol is manufactured.

11. A process in accordance with claim 1, characterized in that 8-chloro-6-(2-chlorophenyl)-2 H,4 H-pyrrolo[3,4-d] [2]benzazepine-2-carboxylic acid methyl ester is manufactured.

**Revendications pour les Etats contractants: BE, CH, DE, FR, GB, IT, LI, LU, NL, SE**

1. Pyrrolo[3,4-d] [2] benzazépines de formule générale:

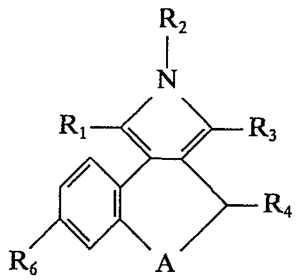

(I)

dans laquelle A représente l'un des groupes:

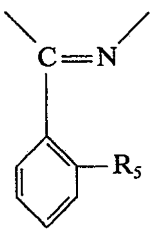 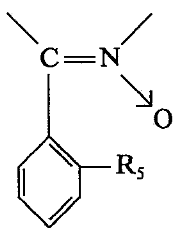 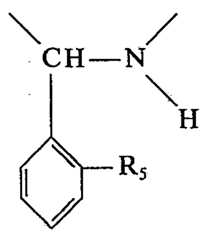 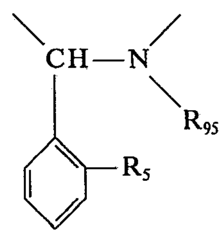 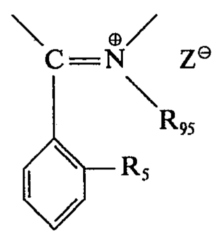

(a) (b) (c) (d) et (e)

$R_1$ représente l'hydrogène, un groupe alkyle inférieur, alcényle en $C_3-C_7$, alcynyle en $C_3-C_7$, hydroxyméthyle ou le groupe $-COR_{11}$, $R_{11}$ représente l'hydrogène, un groupe hydroxy, alcoxy inférieur ou amino ou mono- ou di-(alkyle inférieur)-amino, $R_3$ représente l'hydrogène, un groupe alkyle inférieur, alcényle en $C_3-C_7$, alcynyle en $C_3-C_7$, hydroxyméthyle ou le groupe $-COR_{111}$, $R_{111}$ représente l'hydrogène, un groupe hydroxy, trihalogènométhyle, alcoxy inférieur ou amino ou mono- ou di-(alkyle inférieur)-amino, $R_2$ représente l'hydrogène, un groupe alkyle inférieur, alcényle en $C_3-C_7$, alcynyle en $C_3-C_7$, le groupe $-COO-$alkyle inférieur, un groupe hydroxyalkyle en $C_2-C_7$, amino- ou mono- ou di-(alkyle inférieur)-amino-alkyle en $C_2-C_7$ ou le groupe (alkyle en $C_1-C_6$)$-COR_{21}$, $R_{21}$ représente un groupe hydroxy, alcoxy inférieur, amino- ou mono- ou di-(alkyle inférieur)-amino, $R_4$ représente l'hydrogène, un groupe alcoxy inférrieur à chaîne droite, hydroxy ou $-OCOR$, R représente l'hydrogène, un groupe alkyle en $C_1-C_6$, halogénalkyle inférieur ou phényle, $R_5$ représente un halogène de numéro atomique de dépassant pas 35 ou l'hydrogène, $R_6$ représente un halogène de numéro atomique ne dépassant pas 35, $R_{95}$ représente un groupe alkyle inférieur et $Z^{\ominus}$ est l'anion d'un acide, étant spécifié que les groupes appelés »inférieurs« contiennent au maximum 7 atomes de carbone, et en outre que:

(A) lorsque l'un des symboles $R_1$ et $R_3$ représente un groupe hydroxyméthyle ou $-COR_{11}$ ou $-COR_{111}$, $R_{11}$ et $R_{111}$ ayant les significations indiqués ci-dessus, l'autre symbole représente l'hydrogène, un groupe alkyle inférieur, alcényle en $C_3-C_7$ ou alcynyle en $C_3-C_7$ et $R_2$ représente l'hydrogène;
(B) lorsque $R_4$ représente un groupe $-OCOR$, $R_1$ et $R_3$ représente l'hydrogène, des groupes alkyle inférieurs, alcényle en $C_3-C_7$ ou alcynyle en $C_3-C_7$ et $R_2$ représente un groupe $-CO-$alcoxy inférieur;
(C) lorsque $R_4$ représente une groupe hydroxy, $R_1$ et $R_3$ représentent l'hydrogene, des groupes alkyle inférieurs, alcényle en $C_3-C_7$ ou alcynyle en $C_3-C_7$ et $R_2$ représente l'hydrogène;
(D) lorsque $R_4$ représente un groupe alcoxy inférieur à chaîne droite, $R_1$ et $R_3$ représentent l'hydrogène, des groupes alkyle inférieurs, alcényle en $C_3-C_7$ ou alcynyle en $C_3-C_7$;
(E) lorsque A représente le groupe (b), (c), (d) ou (e) ci-dessus, $R_4$ représente l'hydrogène; et
(F) lorsque A représente le groupe (b) ou (e) ci-dessus, $R_2$ ne peut représenter un groupe amino- ou mono-ou di-(alkyle inférieur)-amino-alkyle en $C_2-C_7$;

et les sels des composés de formule I dans laquelle A représente le groupe (a), formés par addition avec des acides acceptables pour l'usage pharmaceutique.

2. Composés selon la revendication 1, caractérisés en ce que $R_2$ ne représente pas un groupe carboxy(alkyle en $C_1$—$C_6$), étant spécifié en outre que:

(i) lorsque A représente le groupe (a) et que $R_4$ représente un groupe alcoxy inférieur à chaîne droite, $R_2$ représente l'hydrogène ou un groupe alkyle inférieur; et

(ii) lorsque A représente le groupe (b), $R_2$ représente l'hydrogène ou un groupe —COO—alkyle inférieur.

3. Composés selon la revendication 1 ou 2, caractérisés en ce que A représente le groupe (a).

4. Composés selon la revendication 3, caractérisés en ce que $R_3$ représente l'hydrogène, $R_5$ et $R_6$ représente chacun un halogène, $R_1$ représente l'hydrogène ou un groupe alkyle inférieur et $R_4$ l'hydrogène.

5. Composés selon la revendication 4, caractérisés en ce que $R_5$ représente le chlore ou le fluor et $R_6$ le chlore.

6. La 8-chloro-6-(2-chlorophényl)-2 H,4 H-pyrrolo[3,4-d] [2]benzazépine.

7. La 8-chloro-6-(2-fluorophényl)-2 H,4 H-pyrrolo[3,4-d] [2]benzazépine.

8. La 8-chloro-6-(2-chlorophényl)-1-méthyl-2 H,4 H-pyrrolo[3,4-d] [2]benzazépine.

9. La 8-chloro-6-(2-chlorophényl)-2-méthyl-2 H,4 H-pyrrolo[3,4-d] [2]benzazépine.

10. Le 8-chloro-6-(2-fluorophényl)-2 H,4 H-pyrrolo[3,4-d] [2]benzazépine-2-éthanol.

11. Le 8-chloro-6-(2-chlorophényl)-2 H,3 H-pyrrolo[3,4-d] [2]benzazépine-2-carboxylate de méthyle.

12. Composés de formule générale:

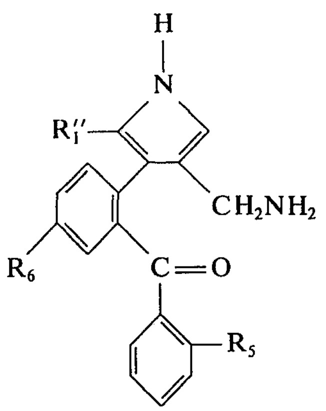

dans laquelle $R_5$ et $R_6$ ont les significations indiquées dans la revendication 1 et $R_1''$ représent l'hydrogène, un groupe anlyle inférieur, alcényle en $C_3—C_7$ ou alcynyle en $C_3—C_7$.

13. Composés de formule générale:

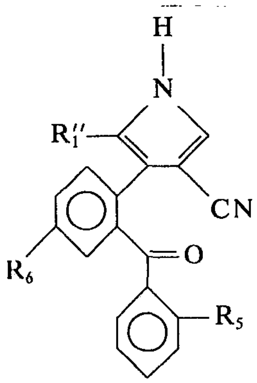

(XI)

dans laquelle $R_5$ et $R_6$ ont les significations indiquées dans la revendication 1 et $R_1''$ représente l'hydrogène, un groupe alkyle inférieur, alcényle en $C_3—C_7$ ou alcynyle en $C_3—C_7$.

14. Composés de formule générale:

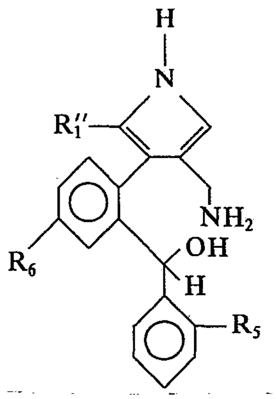

(XII)

dans laquelle $R_5$ et $R_6$ ont les significations indiquées dans la revendication 1 et $R_1''$ représente l'hydrogène, un groupe alkyle inférieur, alcényle en $C_3-C_7$ ou alcynyle en $C_3-C_7$.

15. Composés selon l'une des revendications 1 à 11, en tant que substances actives pharmaceutiques.

16. Composés selon l'une des revendications 1 à 11, en tant que substances actives sédatives et anxiolytiques.

17. Procédé de préparation des composés selon l'une des revendications 1 à 11, caractérisé en ce que:

(a) on cyclise un composé de formule générale:

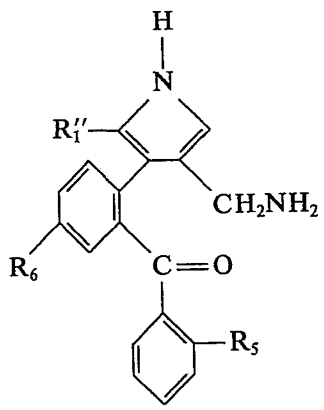

(B)

dans laquelle $R_5$ et $R_6$ ont les significations indiquées ci-dessus et $R_1''$ représente l'hydrogène, un groupe alkyle inférieur, alcényle en $C_3-C_7$ ou alcynyle en $C_3-C_7$ ou bien

(b) on introduit un groupe alkyle inférieur, alcényle en $C_3-C_7$ ou alcynyle en $C_3-C_7$ en position 1 et/ou 3 d'un composé de formule I de la revendication 1 dans laquelle A représente le groupe (a) de la revendication 1, $R_2$ et $R_4$ représentent chacun l'hydrogène, et l'un des symboles $R_1$ et $R_3$ représente l'hydrogène et l'autre l'hydrogene, un groupe alkyle inférieur, alcényle en $C_3-C_7$ ou alcynyle en $C_3-C_7$, ou bien

(c) on introduit un substituant alkyle inférieur, alcényle en $C_3-C_7$, alcynyle en $C_3-C_7$ ou hydroxy-alkyle en $C_2-C_7$ ou $-COO-$alkyle inférieur, $-(CH_2)_n-COO-$alkyle inférieur, $-(CH_2)_n-CO-NR_{91}R_{92}$ ou $-(CH_2)_{n+1}-NR_{91}R_{92}$, n étant un nombre de là 6 et $R_{91}$ et $R_{92}$ représentant chacun l'hydrogène ou un groupe alkyle inférieur en position 2 d'un composé de formule I de la revendication 1 dans laquelle A représente le groupe (a) de la revendication 1, $R_1$ et $R_3$ représentent chacun l'hydrogène, un groupe alkyle inférieur, alcényle en $C_3-C_7$ ou alcynyle en $C_3-C_7$ et $R_2$ représente l'hydrogène, ou bien

(d) on réduit un composé de formule I de la revendication 1 dans laquelle A représente le groupe (a) de la revendication 1, $R_1$ et $R_2$ représentent chacun l'hydrogène, un groupe alkyle inférieur, alcényle en $C_3-C_7$ ou alcynyle en $C_3-C_7$ et $R_2$ représente un groupe $-(CH_2)_nCOO-$alkyle inférieur ou $-(CH_2)_n-CO-NR_{91}R_{92}$, n, $R_{91}$ et $R_{92}$ ayant les significations indiquées ci-dessus, ou bien

(e) on convertit un composé de formule I de la revendication 1 dans laquelle A représente le groupe (a) de la revendication 1, $R_1$ et $R_3$ représentent chacun l'hydrogène, un groupe alkyle inférieur, alcényle en $C_3-C_7$ ou alcynyle en $C_3-C_7$ et $R_2$ représente un groupe $-(CH_2)_n-COO-$alkyle inférieur, n ayant la signification ci-dessus, en l'acide carboxylique correspondant ou en un amide, alkylamide inférieur ou di-(alkyle inférieur)-amide correspondant, ou bien

(f) on soumet un composé de formule I définie dans la revendication 1 dans laquelle A représente le groupe (a) de la revendication 1, $R_2$ et $R_4$ représentent chacun l'hydrogène, et l'un des symboles $R_1$ et $R_3$ représente l'hydrogène et l'autre l'hydrogène, un groupe alkyle inférieur, alcényle en $C_3-C_7$ ou alcynyle en $C_3-C_7$, à formylation ou hydroxyméthylation en position 1 ou 3, ou bien

(g) on réduit un composé de formule I de la revendication 1 dans laquelle A représentele groupe (a) de la revendication 1, $R_2$ et $R_4$ représentent chacun l'hydrogène, et l'un des symboles $R_1$ et $R_3$ représente un groupe formyle et l'autre l'hydrogène, un groupe alkyle inférieur, alcényle en $C_3-C_7$ ou alcynyle en $C_3-C_7$, en le composé hydroxy méthylé correspondant, ou bien

(h) on traite un composé de formule I de la revendication 1 dans laquelle A représente le groupe (a) de la revendication 1, $R_2$, $R_3$ et $R_4$ représentent chacun l'hydrogène et $R_1$ représente l'hydrogène, un groupe alkyle inférieur, alcényle en $C_3-C_7$ ou alcynyle en $C_3-C_7$, par un chlorure de trihalogénoacétyle, ou bien

(i) on traite un composé de formule I de la revendication 1 dans laquelle A représente le groupe (a) de la revendication 1, $R_2$ et $R_4$ représentent chacun l'hydrogène, $R_1$ représente l'hydrogène, un groupe alkyle inférieur, alcényle en $C_3-C_7$ ou alcynyle en $C_3-C_7$ et $R_3$ représente un groupe trihalogénoacétyle, par un alcoolate inférieur de métal alcalin ou par un composé de formule $HNR_{35}R_{36}$ dans laquelle $R_{35}$ et $R_{36}$ représentent chacun l'hydrogène ou un groupe alkyle inférieur, ou bien

(j) on oxyde un composé de formule I de la revendication 1, dans laquelle A représente le groupe (a) de la revendication 1, $R_2$ et $R_4$ représentent chacun l'hydrogène et l'un des symboles $R_1$ et $R_3$ représente un groupe formyle et l'autre l'hydrogène, un groupe alkyle inférieur, alcényle en $C_3-C_7$ ou alcynyle en $C_3-C_7$, en l'acide carboxylique correspondant, ou bien

(k) on etsérifie un composé de formule I dela revendication 1 dans laquelle A représente le groupe (a) de la revendication 1, $R_2$ et $R_4$ représentent chacun l'hydrogène et l'un des symboles $R_1$ et $R_3$ représente le groupe $-COOH$ et l'autre l'hydrogène, un groupe alkyle inférieur, alcényle en $C_3-C_7$ ou alcynyle en $C_3-C_7$, ou bien

(l) on traite un composé de formule I de la revendication 1, dans laquelle A représente le groupe (a) de la revendication 1, $R_2$ et $R_4$ représentent chacun l'hydrogène et l'un des symboles $R_1$ et $R_3$ représente un groupe alcoxycarbonyle inférieur et l'autre l'hydrogène, un groupe alkyle inférieur, alcényle en $C_3-C_7$ ou alcynyle en $C_3-C_7$, par un composé de formule $HNR_{35}R_{36}$ dans laquelle $R_{35}$ et $R_{36}$ ont les significations indiquées ci-dessus, ou bien

(m) on oxyde sur l'azote $N_5$ un composé de formule I de la revendication 1, dans laquelle A représente le groupe (a) de la revendication 1, $R_4$ représente l'hydrogène, $R_1$ et $R_3$ représentent chacun l'hydrogène, un groupe alkyle inférieur, alcényle en $C_3-C_7$ ou alcynyle en $C_3-C_7$ et $R_2$ représente l'hydrogène, un groupe alkyle inférieur, alcényle en $C_3-C_7$, alcynyle en $C_3-C_7$, le groupe $-COO-$alkyle inférieur, un groupe hydroxyalkyle en $C_2-C_7$, le groupe $-(CH_2)_n-COO-$alkyle inférieur ou $(CH_2)_n-CO-NR-R_{91}R_{92}$, n, $R_{91}$ et $R_{92}$ ayant les significations indiques ci-dessus, ou bien

(n) on traite un composé de formule I de la revendication 1, dans laquelle A représente le groupe (b) de la revendication 1, $R_4$ représente l'hydrogène, $R_2$ un groupe $-COO-$alkyle inférieur et $R_1$ et $R_3$ représentent chacun l'hydrogène, un groupe alkyle inférieur, alcényle en $C_3-C_7$ ou alcynyle en $C_3-C_7$, par un agent acylant fournissant le reste $-COR$, R ayant la signification indiquée dans la revendication 1, ou bien

(o) on soumet un composé de formule I de la revendication 1, dans laquelle A représente le groupe (a) de la revendication 1, $R_4$ représente un groupe $-OCOR$, $R_2$ un groupe $-COO-$alkyle inférieur, $R_1$ et $R_3$ représentent chacun l'hydrogène, un groupe alkyle inférieur, alcényle en $C_3-C_7$ ou alcynyle en $C_3-C_7$ et R a la signification indiquée dans la revendication 1, ou bien un composé de formule I de la revendication 1 dans laquelle A représente le groupe (b) de la revendication 1, $R_2$ représente un groupe $-COO-$alkyle inférieur, $R_4$ l'hydrogène et $R_1$ et $R_3$ représentent chacun l'hydrogène, un groupe alkyle inférieur, alcényle en $C_3-C_7$ ou alcynyle en $C_3-C_7$, à une hydrolyse alcaline, ou bien

(p) on traite un composé de formule I de la revendication 1, dans laquelle A représente le groupe (a) de la revendication 1, $R_4$ un groupe $-OCOR$, $R_2$ un groupe $-COO-$alkyle inférieur, $R_1$ et $R_3$ représentent chacun l'hydrogène, un groupe alkyle inférieur, alcényle en $C_3-C_7$ ou alcynyle en $C_3-C_7$, et R a la signification indiquée dans la revendication 1, en présence d'une alcool à chaîne droite en $C_1-C_7$, par un hydroxyde ou un alcoolate de métal alcalin, ou bien

(q) on réduit un composé de formule I de la revendication 1 dans laquelle A représente le groupe (a) de la revendication 1, $R_4$ représente l'hydrogène et $R_1$ et $R_3$ représentent chacun l'hydrogène, un groupe alkyle inférieur, alcényle en $C_3-C_7$ ou alcynyle en $C_3-C_7$, ou bien

(r) dans un composé de formule I de la revendication 1, dans laquelle A représente le groupe (a) de la revendication 1, $R_4$ représente l'hydrogène, $R_1$ et $R_3$ représentant chacun l'hydrogène, un groupe alkyle inférrieur, alcényle en $C_3-C_7$ ou alcynyle en $C_3-C_7$ et $R_2$ représente l'hydrogène, un groupe alkyle inférieur, alcényle en $C_3-C_7$, alcynyle en $C_3-C_7$, un groupe $-COO-$alkyle inférieur, hydroxy$-$alkyle en $C_2-C_7$ ou $-(CH_2)_n-COO-$alkyle inférieur ou $-(CH_2)_n-$ $CO-NR_{91}R_{92}$, n, $R_{91}$ et $R_{92}$ ayant les significations indiquées ci-dessus, on quaternise le groupe imine;

(s) on réduit un composé de formule I de la revendication 1, dans laquelle A représente le groupe (e) de la revendication 1, $R_4$ représente l'hydrogène, $R_1$ et $R_3$ représentent chacun l'hydrogène, un groupe alkyle inférieur, alcényle en $C_3-C_7$ ou alcynyle en $C_3-C_7$ et $R_2$ représente l'hydrogène, un groupe alkyle inférieur, alcényle en $C_3-C_7$, alcynyle en $C_3-C_7$, $-COO-$alkyle inférieur, hydroxyalkyle en $C_2-C_7$ ou $-(CH_2)_n-COO-$alkyle inférieur ou $-(CH_2)_n-CO-NR_{91}R_{92}$, n, $R_{91}$ et $R_{92}$ ayant les significations indiquées ci-dessus, ou bien

(t) on convertit un composé de formule I de la revendication 1, dans laquelle A représente le groupe (a) de la revendication 1, en un sel formé par addition avec un acide acceptable pour l'usage pharmaceutique.

18. Médicaments contenant un composé selon l'une des revendications 1 à 11.
19. Agents sédatifs et anxiolytiques contenant un composé selon l'une des revendications 1 à 11.

**Revendications pour l'Etat contractant: AT**

1. Procédé de préparation de pyrrolo[3,4-d] [2]benzazépines de formule générale

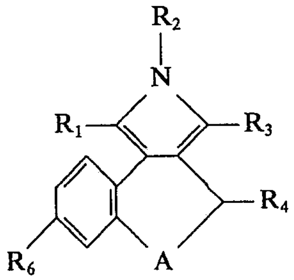

(I)

dans laquelle A représente l'un des groupes

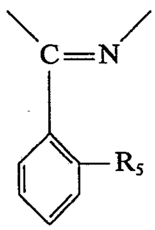

(a)

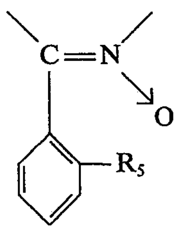

(b)

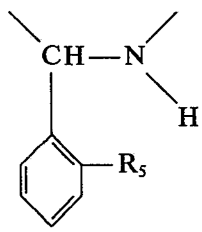

(c)

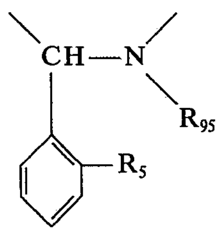

(d) et

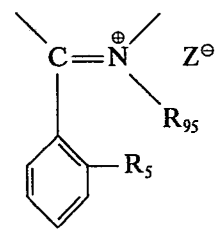

(e)

$R_1$ représente l'hydrogène, un groupe alkyle inférieur, alcényle en $C_3-C_7$, alcynyle en $C_3-C_7$, hydroxyméthyle ou $-COR_{11}$, $R_{11}$ représente l'hydrogène, un groupe hydroxy, alcoxy inférieur ou amino ou mono- ou di-(alkyle inférieur)-amino, $R_3$ représente l'hydrogène, un groupe alkyle inférieur, alcényle en $C_3-C_7$, alcynyle en $C_3-C_7$, hydroxyméthyle ou $-COR_{111}$, $R_{111}$ représente l'hydrogène, un groupe hydroxy, trihalogémométhyle, alcoxy inférieur ou amino ou mono- ou di-(alkyle inférieur)-amino, $R_2$ représente l'hydrogène, un groupe alkyle inférieur, alcényle en $C_3-C_7$, alcynyle en $C_3-C_7$, $-COO-$alkyle inférieur, hydroxyalkyle en $C_2-C_7$, amino- ou mono- ou di-(alkyle inférieur)-amino-alkyle en $C_2-C_7$, ou un groupe (alkyle en $C_1-C_6$)$-COR_{21}$, $R_{21}$ représente un groupe hydroxy, alcoxy inférieur, amino ou mono- ou di-(alkyle inférieur)-amino, $R_4$ représente l'hydrogène, un groupe alcoxy inférieur à chaîne droite, hydroxy ou $-OCOR$, R représente l'hydrogène, un groupe alkyle en $C_1-C_6$, halogénoalkyle inférieur ou phényle, $R_5$ représente un halogène de numéro atomique ne dépassant pas 35 ou l'hydrogène, $R_6$ représente de numéro atomique ne dépassant pas 35, $R_{95}$ représente un groupe alkyle inférieur et $Z^{\ominus}$ représente l'anion d'un acide, étant spécifié que les groupes appelés »inférieurs« contiennent au maximum 7 atomes de carbone, et étant en outre spécifié que:

(A) lorsque l'un des symboles $R_1$ et $R_3$ représente un groupe hydroxymthyle ou $-COR_{11}$ ou $COR_{111}$, $R_{11}$ et $R_{111}$ ayant les significations indiquées ci-dessus, l'autre représente l'hydrogène, un groupe alkyle inférieur, alcényle en $C_3-C_7$ ou alcynyle en $C_3-C_7$ et $R_2$ représente l'hydrogène;

(B) lorsque $R_4$ représente un groupe $-OCOR$, $R_1$ et $R_3$ représentent l'hydrogène, des groupes alkyle inférieurs, alcényle en $C_3-C_7$ ou alcynyle en $C_3-C_7$ et $R_2$ représente un groupe $-CO-$alcoxy inférieur;

(C) lorsque $R_4$ représente un groupe hydroxy, $R_1$ et $R_3$ représentent l'hydrogène, des groupes alkyle inférieurs, alcényle en $C_3-C_7$ ou alcynyle en $C_3-C_7$ et $R_2$ représente l'hydrogène;

(D) lorsque $R_4$ représente un groupe alcoxy inférieur à chaîne droite, $R_1$ et $R_3$ représentent l'hydrogène, des groupes alkyle inférieurs, alcényle en $C_3-C_7$ ou alcynyle en $C_3-C_7$;
(E) lorsque A représente le groupe (b), (c), (d) ou (e) ci-dessus, $R_4$ représente l'hydrogène; et
(F) lorsque A représente le groupe (b) ou (e), $R_2$ ne peut représenter un groupe amino- ou mono- ou di-(alkyle inférieur)-amino-alkyle en $C_2-C_7$;

et des sels des composés de formule I dans laquelle A représente le groupe (a), formés par addition avec des acides acceptables pour l'usage pharmaceutique, caractérisé en ce que:

a) on cyclise un composé de formule générale

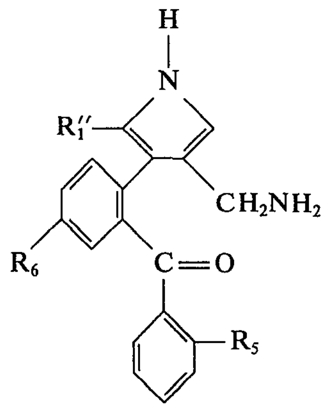

(B)

dans laquelle $R_5$ et $R_6$ ont les significations indiquées ci-dessus, et $R_1''$ représente l'hydrogène, une groupe alkyle inférieur, alcényle en $C_3-C_7$ ou alcynyle en $C_3-C_7$, ou bien

b) on introduit un groupe alkyle inférieur, alcényle en $C_3-C_7$ ou alcynyle en $C_3-C_7$ en position 1 et/ou 3 d'un composé de formule I dans laquelle A représente le groupe (a) ci-dessus, $R_2$ et $R_4$ représentent chacun l'hydrogène, et l'un des symboles $R_1$ et $R_3$ représente l'hydrogène et l'autre l'hydrogène, un groupe alkyle inférieur, alcényle en $C_3-C_7$ ou alcynyle en $C_3-C_7$, ou bien

c) dans un composé de formule I dans laquelle A représente le groupe (a) ci-dessus, $R_1$ et $R_3$ représentent chacun l'hydrogène, un groupe alkyle inférieur, alcényle en $C_3-C_7$ ou alcynyle en $C_3-C_7$ et $R_2$ représente l'hydrogène, on introduit en position 2 un substituant alkyle inférieur, alcényle en $C_3-C_7$, alcynyle en $C_3-C_7$ ou hydroxyalkyle en $C_2-C_7$ ou $-COO-$alkyle inférieur, $-(CH_2)_n-COO-$alkyle inférieur, $-(CH_2)_n-CO-NR_{91}R_{92}$ ou $-(CH_2)_{n+1}-NR_{91}R_{92}$, n étant un nombre de 1 à 6 et $R_{91}$ et $R_{92}$ représentant chacun l'hydrogène ou un groupe alkyle inférieur, ou bien

d) on réduit un composé de formule I dans laquelle A représente le groupe (a) ci-dessus, $R_1$ et $R_3$ représentent chacun l'hydrogène, un groupe alkyle inférieur, alcényle en $C_3-C_7$ ou alcynyle en $C_3-C_7$ et $R_2$ représente un groupe $-(CH_2)_n-COO-$alkyle inférieur ou $-(CH_2)_n-CO-NR_{91}R_{92}$,n, $R_{91}$ et $R_{92}$ ayant les significations indiquées ci-dessus, ou bien

e) on convertit un composé de formule I dans laquelle A représente le groupe (a) ci-dessus, $R_1$ et $R_3$ représentent chacun l'hydrogène, un groupe alkyle inférieur, alcényle en $C_3-C_7$ ou alcynyle en $C_3-C_7$ et $R_2$ représente un groupe $-(CH_2)_n-COO-$alkyle inférieur, n ayant la signification indiquée ci-dessus, en l'acide carboxylique correspondant ou en un amide, alkylamide inférieur ou di-(alkyle inférieur)-amide correspondant, ou bien

f) on soumet un composé de formule I dans laquelle A représente le groupe (a) ci-dessus, $R_2$ et $R_4$ représentent chacun l'hydrogène et l'un des symboles $R_1$ et $R_3$ représentent l'hydrogène et l'autre l'hydrogène, un groupe alkyle inférieur, alcényle en $C_3-C_7$ ou alcynyle en $C_3-C_7$, à formylation ou hydroxyméthylation en position 1 ou 3, ou bien

g) on réduit un composé de formule I dans laquelle A représente le groupe (a) ci-dessus, $R_2$ et $R_4$ représentent chacun l'hydrogène et l'un des symboles $R_1$ et $R_3$ représente un groupe formyle et l'autre l'hydrogène, un groupe alkyle inférieur, alcényle en $C_3-C_7$ ou alcynyle en $C_3-C_7$, en le composé hydroxyméthylé correspondant, ou bien

h) on traite un composé de formule I dans laquelle A représente le groupe (a) ci-dessus, $R_2$, $R_3$ et $R_4$ représentent chacun l'hydrogène et $R_1$ représente l'hydrogène, un groupe alkyle inférieur, alcényle en $C_3-C_7$ ou alcynyle en $C_3-C_7$, par un chlorure de trihalogénoacétyle, ou bien

i) on traite un composé de formule I dans laquelle A représente le groupe (a) ci-dessus, $R_2$ et $R_4$ représentent chacun l'hydrogène, $R_1$ représente l'hydrogène, un groupe alkyle inférieur, alcényle en $C_3-C_7$ ou alcynyle en $C_3-C_7$ et $R_3$ un groupe trihalogénoacétyle, par un alcoolate inférieur de métal alcalin ou par un composé de formule $HNR_{35}R_{36}$ dans laquelle $R_{35}$ et $R_{36}$ représentent chacun l'hydrogène ou un groupe alkyle inférieur, ou bien

j) on oxyde un composé de formule I dans laquelle A représente le groupe (a) ci-dessus, $R_2$ et $R_4$

représentent chacun l'hydrogène et l'un des symboles $R_1$ et $R_3$ représente un groupe formyle et l'autre l'hydrogène, un groupe alkyle inférieur, alcényle en $C_3-C_7$ ou alcynyle en $C_3-C_7$, en l'acide carboxylique correspondant, ou bien

k) on estérifie un composé de formule I dans laquelle A représente le groupe (a) ci-dessus, $R_2$ et $R_4$ représentent chacun l'hydrogène et l'un des symboles $R_1$ et $R_3$ représente le groupe $-COOH$ et l'autre l'hydrogène, un groupe alkyle inférieur, alcényle en $C_3-C_7$ ou alcynyle en $C_3-C_7$, ou bien

l) on traite un composé de formule I dans laquelle A représente le groupe (a) ci-dessus, $R_2$ et $R_4$ représentent chacun l'hydrogène et l'un des symboles $R_1$ et $R_3$ représente un groupe alcoxycarbonyle inférieur et l'autre l'hydrogène, un groupe alkyle in férieur, alcényle en $C_3-C_7$ ou alcynyle en $C_3-C_7$, par un composé de formule $HNR_{35}R_{36}$ dans laquelle $R_{35}$ et $R_{36}$ ont les significations indiquées ci-dessus, ou bien

m) on oxyde sur l'azote $N_5$ un composé de formule I dans laquelle A représente le groupe (a) ci-dessus, $R_4$ représente l'hydrogène, $R_1$ et $R_3$ représentent chacun l'hydrogène, un groupe alkyle inférieur, alcényle en $C_3-C_7$ ou alcynyle en $C_3-C_7$ et $R_2$ représente l'hydrogène, un groupe alkyle inférieur, alcényle en $C_3-C_7$, alcynyle en $C_3-C_7$, $-COO-$alkyle inférieur, hydroxyalkyle en $C_2-C_7$ ou $-(CH_2)_n-COO-$alkyle inférieur ou $-(CH_2)_n-CO-NR_{91}R_{92}$, n, $R_{91}$ et $R_{92}$ ayant les significations indiquées ci-dessus, ou bien

n) on traite un composé de formule I dans laquelle A représente le groupe (b) ci-dessus, $R_4$ représente l'hydrogène, $R_2$ un groupe $-COO-$alkyle inférieur, et $R_1$ et $R_3$ représentent chacun l'hydrogène, un groupe alkyle inférieur, alcényle en $C_3-C_7$ ou alcynyle en $C_3-C_7$, par un agent acylant fournissant le reste $-COR$ dans lequel R a la signification indiquée ci-dessus, ou bien

o) on soumet un composé de formule I dans laquelle A représente le groupe (a) ci-dessus, $R_4$ un groupe $-OCOR$, $R_2$ un groupe $-COO-$alkyle inférieur, $R_1$ et $R_3$ représentent chacun l'hydrogène, un groupe alkyle inférieur, alcényle en $C_3-C_7$ ou alcynyle en $C_3-C_7$ et R a la signification indiquée ci-dessus, ou bien un composé de formule I dans laquelle A représente le groupe (b) ci-dessus, $R_2$ un groupe $-COO-$alkyle inférieur, $R_4$ l'hydrogène et $R_1$ et $R_3$ représentent chacun l'hydrogène, un groupe alkyle inférieur, alcényle en $C_3-C_7$ ou alcynyle en $C_3-C_7$, à une hydrolyse alcaline, ou bien

p) on traite un composé de formule I dans laquelle A représente le groupe (a) ci-dessus, $R_4$ un groupe $-OCOR$, $R_2$ un groupe $-COO$-alkyle inférieur, $R_1$ et $R_3$ représentent chacun l'hydrogène, un groupe alkyle inférieur, alcényle en $C_3-C_7$ ou alcynyle en $C_3-C_7$ et R a la signification indiquée ci-dessus, en présence d'un alcool à chîne droite en $C_3-C_7$, par un hydroxyde ou un alcoolate de métal alcalin ou bien

q) on réduit un composé de formule I dans laquelle A représente le groupe (a) ci-dessus, $R_4$ représente l'hydrogène et $R_1$ et $R_3$ représentent chacun l'hydrogène, un groupe alkyle inférieur, alcényle en $C_3-C_7$ ou alcynyle en $C_3-C_7$, ou bien

r) dans un composé de formule I dans laquelle A représente le groupe (a) ci-dessus, $R_4$ représente l'hydrogène, $R_1$ et $R_3$ représente chacun l'hydrogène, un groupe alkyle inférieur, alcényle en $C_3-C_7$ ou alcynyle en $C_3-C_7$ et $R_2$ représente l'hydrogène, un groupe alkyle inférieur, alcényle en $C_3-C_7$, alcynyle en $C_3-C_7$, $-COO-$alkyle inférieur, hydroxyalkyle en $C_2-C_7$ ou $-(CH_2)_n-COO-$alkyle inférieur ou $-(CH_2)_n-CO-NR_{91}R_{92}$, n $R_{91}$ et $R_{92}$ ayant les significations indiquées ci-dessus, on quaternise le groupe imine, ou bien

s) on réduit un composé de formule I dans laquelle A représente le groupe (e) ci-dessus, $R_4$ représente l'hydrogène, $R_1$ et $R_3$ représentent chacun l'hydrogène, un groupe alkyle inférieur, alcényle en $C_3-C_7$ ou alcynyle en $C_3-C_7$ et $R_2$ représente l'hydrogène, un groupe alkyle inférieur, alcényle en $C_3-C_7$, alcynyle en $C_3-C_7$, $-COO-$alkyle inférieur, hydroxyalkyle en $C_2-C_7$ ou $-(CH_2)_n-COO-$alkyle inférieur ou $-(CH_2)_n-CO-NR_{91}R_{92}$, n, $R_{91}$ et $R_{92}$ ayant les significations indiquées ci-dessus, ou bien

t) on convertit un composé de formule I dans laquelle A représente le groupe (a) ci-dessus en un sel formé par addition avec un acide acceptable pour l'usage pharmaceutique.

2. Procédé selon la revendication 1, pour la préparation de composés de formule I de la revendication 1, dans laquelle $R_2$ a une signification autre qu'un groupe carboxy—alkyle en $C_1-C_6$, étant spécifié que:

(i) lorsque A représente le groupe (a) et $R_4$ un groupe alcoxy inférieur à chaîne droite, $R_2$ représente l'hydrogène ou un groupe alkyle inférieur; et

(ii) lorsque A représente le groupe (b), $R_2$ représente l'hydrogène ou un groupe $-COO-$alkyle inférieur,

ou des sels des composés de formule I dans laquelle A représente le groupe (a) formés par addition avec des acides acceptables pour l'usage pharmaceutique, caractérisé en ce que l'on prépare ces composés ou sels selon les variantes opératoires (a), (b), (f), (g), (h), (i), (j), (k), (l), (n), (o), (p), (q), (r), ou (s); ou selon la variante opératoire (c), où $R_4$ représente dans le produit de départ l'hydrogène et où l'on introduit en position 2 de ce produit de départ un substituant autre qu'un groupe hydroxy—al-

kyle en $C_2-C_7$ ou $-(CH_2)_n-COO-NR_{91}R_{92}$, ou bien où $R_4$ représente dans le produit de départ un groupe alcoxy inférieur à chaîne droite et où l'on introduit dans ce produit de départ, en position 2, un substituant alkyle inférieur; ou bien selon la variante opératoire (d) où le symbole $R_4$ représente l'hydrogène dans le produit de départ et où l'on convertit celui-ci en un amide, alkylamide inférieur ou di-(alkyle inférieur)-amide correspondant; ou bien selon la variante opératoire (m)où $R_2$ représente dans le produit de départ l'hydrogène ou un groupe $-COO-$alkyle inférieur.

3. Procédé selon la revendication 1 ou 2, caractérisé en ce que l'on prépare des composés de formule générale I de la revendication 1 dans laquelle A représente le groupe (a).

4. Procédé selon la revendication 3, caractérisé en ce que l'on prépare des composés de formule générale I de la revendication 1 dans laquelle $R_3$ représente l'hydrogène, $R_5$ et $R_6$ représentent chacun un halogène, $R_1$ représente l'hydrogène ou un groupe alkyle inférieur et $R_4$ l'hydrogène.

5. Procédé selon la revendication 4, caractérisé en ce que l'on prépare des composés de formule générale I de la revendication 1 dans laquelle $R_5$ représente le chlore ou le fluor et $R_6$ le chlore.

6. Procédé selon la revendication 1, caractérisé en ce que l'on prépare la 8-chloro-6-(2-chlorophényl)-2 H,4 H-pyrrolo[3,4-d] [2]benzazépine.

7. Procédé selon la revendication 1, caractérisé en ce que l'on prépare la 8-chloro-6-(2-fluorophényl)-2 H,4 H-pyrrolo[3,4-d] [2]benzazépine.

8. Procédé selon la revendication 1, caractérisé en ce que l'on prépare la 8-chloro-6-(2-chlorophényl)-1-méthyl-2 H,4 H-pyrrolo[3,4-d] [2]benzazépine.

9. Procédé selon la revendication 1, caractérisé en ce que l'on prépare la 8-chloro-6-(2-chlorophényl)-2-méthyl-2 H,4 H-pyrrolo[3,4-d] [2]benzazépine.

10. Procédé selon la revendication 1, caractérisé en ce que l'on prépare le 8-chloro-6-(2-fluorophényl)-2 H,4 H-pyrrolo[3,4-d] [2]benzazépine-2-éthanol.

11. Procédé selon la revendication 1, caractérisé en ce l'on prépare le 8-chloro-6-(2-chlorophényl)-2 H,4 H-pyrrolo[3,4-d] [2]benzazépine-2-carboxylate de méthyle.